# EUROPEAN PATENT APPLICATION

(11) **EP 4 560 021 A1**
(43) Date of publication of application: **28.05.2025**
(21) Application number: 23842316.4
(22) Date of filing: 18.07.2023
(51) Int. Cl.: C12N 15/50, A61K 48/00, A61K 39/215, A61P 31/14

(54) **MRNA FOR SARS-COV-2 S PROTEIN AND USE THEREOF**

(30) Priority: 19.07.2022 CN 202210845690; 02.06.2023 WO PCT/CN2023/098013; 15.06.2023 WO PCT/CN2023/100319
(71) Applicant: Shenzhen Shenxin Biotechnology Co., Ltd., Shenzhen, Guangdong 518000 (CN)
(72) Inventor: HUANG, Hui, Shenzhen, Guangdong 518000 (CN); PANG, Silin, Shenzhen, Guangdong 518000 (CN); LI, Linxian, Shenzhen, Guangdong 518000 (CN); LI, Yongbing, Shenzhen, Guangdong 518000 (CN)
(74) Representative: Epping - Hermann - Fischer
(86) International application number: PCT/CN2023/107936
(87) International publication number: WO 2024/017253

(57) **Abstract**

The present invention relates to an RNA encoding the S protein of SARS-COV-2, a vaccine comprising the RNA, and uses thereof. The present invention also relates to a universal polynucleotide molecule comprising a 5'-UTR and/or a 3'- UTR, and a nucleic acid sequence encoding a protein and/or polypeptide of interest, and optionally comprising a polyA.

## Description

### Technical Field

The present invention belongs to the pharmaceutical field. Specifically, the present invention relates to an RNA encoding the S protein of SARS-COV-2, a vaccine comprising the RNA, and uses thereof. The present invention also relates to a universal polynucleotide molecule for preparing a novel mRNA vaccine, comprising a 5'-UTR and a nucleic acid sequence encoding a polypeptide of interest, and optionally comprising a 3'-UTR and/or a polyA.

### Background Art

SARS-COV-2 can cause serious infectious diseases and is highly contagious. The International Committee on Taxonomy of Viruses (ICTV) named this virus SARS coronavirus 2 (severe acute respiratory syndrome coronavirus 2, SARS-CoV-2). At present, there are some inactivated vaccines, attenuated vaccines, and recombinant protein subunit vaccines etc. against this virus, which have achieved some results. However, SARS-COV-2 has been constantly mutating and evolving, and many new strains with stronger infectivity and pathogenicity have emerged. These new mutant viruses have immune evasion against the antibodies produced by the existing vaccines. Therefore, the development of an effective vaccine that can prevent mutant COVID-19 is still the best means and urgent need to control or completely eliminate the epidemic.

SARS-COV-2 belongs to the Coronavirus family. The coronavirus particles are spherical or elliptical. The virus has an envelope with spikes on it. The internal genome is a single-stranded positive-strand RNA (+ssRNA). The entire virus particle has a crown or a corona-like appearance under an electron microscope. The viral genome can be divided into two categories according to its function: the open reading frames (ORF) ORF1a and ORF1b that are mainly responsible for encoding non-structural proteins such as enzymes related to viral replication and transcription, and the gene sequences encoding structural proteins, e.g, the main structural proteins such as spike protein (S), envelope protein (E), membrane protein (M), and nucleocapsid protein (N). Among them, the S protein can recognize and bind to the host cell surface receptor angiotensin converting enzyme 2 (ACE2), mediating the fusion of the virus and host cell membranes. The S protein is also the main antigen that induces the host to produce humoral and cellular immune responses, especially humoral immune responses. Therefore, the S protein is suitable as a target for developing a vaccine against SARS-COV-2.

mRNA (messenger RNA) is a single-stranded RNA molecule. It is manufactured using the genomic DNA sequence in the cell nucleus as a template and then exported to the cytoplasm, and mRNA is translated in the ribosome to produce specific proteins, thereby exerting biological effects. Mature mRNA mainly contains the following structures: 5'-cap structure, 5'-UTR, coding region, 3'-UTR and polyA tail. Among them, the 5'-cap structure is crucial for the recognition of the ribosome and the protection of mRNA molecules from RNase. The untranslated regions 5'-UTR and 3'-UTR control the stability and translation efficiency of mRNA molecules, and 3'-UTR even determines the cytoplasmic localization of mRNA. The coding region is mainly composed of codons, which are encoded by the ribosome and translated into proteins. The start codon is generally AUG, and the stop codon is generally UGA in eukaryotic cells. The polyA tail is a long adenine nucleotide sequence, which facilitates the export from the cell nucleus and translation and protects mRNA from degradation.

Compared with recombinant protein subunit vaccines, inactivated vaccines or DNA vaccines, mRNA vaccines have several obvious advantages. First, since mRNA does not infect the body or integrate into genomic DNA, its safety is greatly improved. Secondly, the use of modified bases can reduce the inherent immunogenicity of mRNA molecules and reduce their degradation by the body, further improving the safety of mRNA vaccines and the stability of mRNA. In addition, the yield of mRNA synthesis *in vitro* is very high, so mRNA vaccines have the potential to be efficient, rapidly developed, low-cost manufactured and easy to manage safely. For the control of new outbreaks of viruses, mRNA vaccines against new viruses can be developed and produced on a large scale more rapidly. Therefore, compared with traditional vaccine products, research and development of mRNA vaccine has great potential.

Since its emergence, SARS-COV-2 has evolved into many different variants, including Alpha (such as B.1.1.7 variant and its descendant lineage), Beta (such as B.1.351 variant and its descendant lineage), Gamma (such as P.1 variant and its descendant lineage), Delta (such as B.1.617.2 and AY lineage) and Omicron, etc. With the widespread spread and prevalence of mutant lineages such as Delta and Omicron around the world, the efficiency of neutralizing antibodies by existing vaccines has been significantly reduced, resulting in the weakening of the protection induced by existing vaccines, increased transmissibility and disease severity, and the mutant lineages such as Delta and Omicron have become a variant of concern (VOC) worldwide. Therefore, the rapid development of a new mRNA vaccine against the SARS-COV-2 variant is extremely challenging and of great significance.

The untranslated region (UTR) of mRNA controls the translation, degradation and localization of genes, including the stem-loop structure, upstream start codon, upstream open reading frame, internal ribosome entry site and various cis-acting elements that bind to RNA-binding proteins.

UTRs play a crucial role in post-transcriptional regulation of gene expression, including regulation of mRNA nuclear export and translation efficiency, subcellular localization, and stability. UTRs may also play additional roles, such as the specific incorporation of the modified amino acid selenocysteine at the UGA codon of selenoprotein-encoding mRNA, a process mediated by a conserved stem-loop structure in the 3'-UTR.

Those skilled in the art know that the translation efficiency of mRNA molecules directly affects the dosage and dosing interval of mRNA drugs (especially mRNA vaccines), which ultimately affects the bioavailability of mRNA drugs and determines the clinical value of mRNA drugs. Although there are technical solutions for increasing the translation efficiency and stability of mRNA molecules, such as increasing the translation efficiency of mRNA molecules by adding untranslated regions (UTRs), there is still a need for further improvement in terms of enhancing the translation efficiency of mRNA molecules.

Therefore, there is a need to identify or design UTRs that can achieve higher mRNA translation efficiency and/or stability.

### Summary of the invention

The inventors of the present application unexpectedly discovered that by analyzing the mutation sites of SARS-COV-2, a novel RNA coding sequence of SARS-COV-2 S protein was designed and a novel mRNA vaccine was prepared therefrom. The mRNA vaccine shows cross-protective reactions against different strains, including SARS-COV-2 wild-type, Delta and Omicron variants, and has superior immune effects against Delta and Omicron variants, and can be developed and mass-produced more rapidly.

The inventors of the present application also unexpectedly constructed a universal polynucleotide molecule for preparing a novel mRNA vaccine, which comprises a 5'-UTR and a nucleic acid sequence encoding a protein and/or polypeptide of interest, and optionally comprises a 3'-UTR and/or a polyA. These innovative designs have increased the level of protein expressed by the mRNA. The polynucleotide molecule can not only be used to prepare an mRNA vaccine with superior immune effects against SARS-COV-2 wild-type, Delta and Omicron variants, but can also be used to transfect cells or bacteria to produce corresponding antibodies or antigenic proteins, and is therefore a universal core element combination.

In addition, the inventors of the present application have identified a variety of 5'-UTRs and/or 3'-UTRs that can improve the translation efficiency and/or stability of mRNA molecules, as well as RNA (such as mRNA) containing the UTRs. The above-mentioned UTRs are universal core elements, which confer RNA (such as mRNA) containing the UTRs enhanced translation efficiency, significantly improve the expression of target genes and/or mRNA stability, and have broad utility in the industrialization of RNA drugs.

### BRIEF DESCRIPTION OF THE DRAWINGS

Figure 1A is a flow chart of construction of plasmid H;
Figure 1B is a flow chart of construction of engineered plasmid;
Figure 2 is a map of luciferase-pcDNA3 plasmid;
Figure 3 is a map of plasmid B of the present invention;
Figure 4 is a map of plasmid C of the present invention;
Figure 5 is a map of plasmid D of the present invention;
Figure 6 is a map of plasmid F of the present invention;
Figure 7 is a map of plasmid G of the present invention;
Figure 8 is a map of plasmid H of the present invention;
Figure 9 is a map of engineered plasmid of the present invention;
Figure 10A is a schematic diagram of the construction and modification of plasmid H;
Figure 10B is a schematic diagram of the construction and modification of engineered plasmid;
Figure 11 shows the results of liver imaging *in vivo* for mice injected with different 5'-UTR mRNAs;
Figure 12 is a summary of the average photon number of liver imaging *in vivo* for mice injected with different 5'-UTR mRNAs;
Figure 13 is the results of liver imaging of mouse *in vivo* produced by combinations of a fixed 5'-UTR and different 3'-UTRs;
Figure 14 is a summary of the average photon number of liver imaging of mouse *in vivo* produced by combinations of a fixed 5'-UTR and different 3'-UTRs;
Figure 15 shows the results of liver imaging of mouse *in vivo* using a selection of polyA;
Figure 16 is a summary of the average photon number of liver imaging of mouse *in vivo* using a selection of polyA;
Figure 17 is the western blot results of the antigen protein expressed by cells transfected with the engineered plasmid;
Figure 18 is the FACS flow results of the antigen protein expressed by cells transfected with engineered plasmids and SARS-COV-2 mRNA;
Figure 19 shows the ELISA results of the expression level of SARS-COV-2 mRNA transfected cells;
Figure 20 shows the test results of IgG antibodies against the Delta variant S protein in the serum of mouse immunized with the SARS-COV-2 mRNA vaccine formulation;
Figure 21 shows the results of the SARS-COV-2 pseudovirus neutralization test in the SARS-COV-2 mRNA *in vivo* immunization assay;
Figure 22 shows the results of the Delta pseudovirus neutralization test in the SARS-COV-2 mRNA *in vivo* immunization assay;
Figure 23 shows the test results of IgG antibodies against the S protein of the Omicron variant induced by immunization of mice with the SARS-COV-2 mRNA vaccine *in vivo;*
Figure 24 shows the test results of neutralizing antibodies against Omicron (BA.2) pseudovirus induced by immunization of mice with the SARS-COV-2 mRNA vaccine *in vivo;*
Figure 25 shows the Elispot (IFN-γ) test results of spleen lymphocytes after immunization of mice with the SARS-COV-2 mRNA vaccine *in vivo;*
Figure 26 shows the results of liver imaging *in vivo* for mRNAs containing individual 3UTR-NO3, 3UTR-NO30, and 3UTR-NO50;
Figure 27 shows the summary results of the average photon number of liver imaging *in vivo* for mRNAs containing individual 3UTR-NO3, 3UTR-NO30, and 3UTR-NO50.

### DETAILED DESCRIPTION

To make the objectives, technical solutions and advantages of the present invention clearer, the technical solutions of the present invention will be described in detail below. Apparently, the described embodiments are only part of the embodiments of the present invention, rather than all the embodiments. Based on the embodiments of the present invention, all other implementations obtained by the person of ordinary skill in the art without exercising inventive labor fall into the scope of the present invention.

### Definition

All patents, patent applications, scientific publications, manufacturer's specifications and instructions, etc., cited herein, whether supra or infra, are hereby incorporated by reference in their entirety. Nothing herein should be construed as an admission that the present disclosure is not entitled to antedate such publication.

Unless otherwise indicated, the scientific and technical terms used herein have the meanings commonly understood by those skilled in the art. In addition, the terms relating to protein and nucleic acid chemistry, molecular biology, cell and tissue culture, and microbiology used herein are all widely used in the relevant fields (see, for example, Molecular Cloning: A Laboratory Manual, 2nd Edition, J. Sambrook et al. eds., Cold Spring Harbor Laboratory Press, Cold Spring Harbor 1989). Meanwhile, in order to better understand the present invention, the definitions and explanations of the related terms are provided below.

As used herein, the expressions "comprise", "include", "contain" and "having" are open ended, meaning the inclusion of the listed elements, steps, or components but not the exclusion of other unlisted elements, steps, or components. The expression "consisting of" excludes any element, step, or component not specified. The expression "consisting essentially of" means that the scope is limited to the specified elements, steps, or components, plus optional elements, steps, or components that do not materially affect the basic and novel properties of the claimed subject matter. It should be understood that the expressions "consisting essentially of" and "consisting of" are encompassed within the meaning of the expression "comprising".

As used herein, unless the context indicates otherwise, the singular expressions "a", "an", "the", and similar references used in the context of describing the present invention (particularly in the context of the claims) should be interpreted as covering both the singular and the plural. The terms "one or more" or "at least one" encompass 1, 2, 3, 4, 5, 6, 7, 8, 9 or more. The term "at least one" encompasses 1, 2, 3, 4, 5, 6, 7, 8, 9 or more. The term "at least two" encompasses 2, 3, 4, 5, 6, 7, 8, 9 or more. The term "at least two" encompasses 2, 3, 4, 5, 6, 7, 8, 9 or more.

The numerical ranges described herein should be understood to include any and all subranges contained therein. For example, the range "1 to 10" should be understood to include not only the explicitly stated values of 1 and 10, but also any single value (e.g., 2, 3, 4, 5, 6, 7, 8, and 9) and subranges (e.g., 1 to 2, 1.5 to 2.5, 1 to 3, 1.5 to 3.5, 2.5 to 4, 3 to 4.5, etc.) within the range of 1 to 10. This principle also applies to ranges with only one value as the minimum or maximum value.

Unless otherwise stated, all methods described herein can be performed in any suitable order.

As used herein, the term "wild type" means that the sequence is naturally occurring and has not been artificially modified, including naturally occurring mutants. The term "fragment" or "fragment of a nucleic acid" refers to a portion of a nucleic acid, for example, a nucleic acid shortened at the 5' and/or 3' ends. The fragment of a nucleic acid contains at least 50%, 60%, 70% or 80% of nucleotide residues from the nucleic acid. In some embodiments, the fragment of a nucleic acid contains at least 70% or 80% of nucleotide residues from the nucleic acid, preferably at least 90%, 95%, 96%, 97%, 98% or 99%. Generally, it can be a shorter portion of the full length of the nucleic acid.

The term "variant" relating to nucleic acid refers to a nucleic acid variant, at least one nucleotide of which is different from a reference nucleic acid (or "parent"). Compared with a reference nucleic acid, a variant nucleic acid comprises single or multiple nucleotide deletions, additions, mutations and/or insertions, wherein deletions include removing one or more nucleotides from a reference nucleic acid; additions include fusing one or more nucleotides (e.g., 1, 2, 3, 5, 10, 20, 30, 50 or more nucleotides) to the 5' and/or 3' ends of a reference nucleic acid; mutations may include, but are not limited to, substitutions (e.g., at least one nucleotide is removed and another nucleotide is inserted in its place (e.g., transversion and conversion)); insertions include adding at least one nucleotide. In some embodiments, the nucleic acid variant is a variant of a 5'-UTR, a variant of a 3'-UTR, or a variant of ploy A. In some embodiments, the mutations introduced into the nucleic acid variant can prevent the nucleic acid variant from being recognized by a nuclease and cleaved, from binding to micro RNA, and from generating complex secondary structures such as hairpin structures or G-quadruplexes. For example, the nucleic acid variant is a variant derived from the 3'-UTR of the HDGFL1 gene (GeneBank Accession No. NM_138574.4), in which two "C"s are mutated to two "A"s based on the 3'-UTR of the HDGFL1 gene to avoid recognition and cleavage by nucleases.

The term "nucleic acid variant" used herein includes naturally occurring variants and engineered variants. Therefore, "nucleic acid variant" defined herein can be derived from a reference nucleic acid, separated from, related to, based on or homologous to a reference nucleic acid sequence." Nucleic acid variant" optionally has at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99% identity to a counterpart naturally occurring (wild type) nucleic acid or its homologue, fragment or derivative, and in some embodiments, at least 70%, at least 80%, at least 85%, at least 90%, at least 95% or at least 97% sequence identity. It is understood that for a nucleic acid molecule, the term "variant" includes a degenerate nucleic acid sequence, wherein the degenerate nucleic acid sequence according to the present invention is a nucleic acid different from the reference nucleic acid in the codon sequence due to the degeneracy of the genetic code.

As used herein, the term "% identity" or "% homology" refers to the percentage of identical nucleotides or amino acids in the optimal alignment between the sequences to be compared, and the differences between the two sequences can be distributed over a local region (segment) or over the entire length of the sequences to be compared. Usually, the identity between the two sequences is determined after the optimal alignment of the segment or "comparison window". The optimal alignment can be performed manually or with the aid of algorithms known in the art. Algorithms known in the art include, but are not limited to, the local homology algorithm described by Smith and Waterman, 1981, Ads App. Math. 2, 482 and Neddleman and Wunsch, 1970, J. Mol. Biol. 48, 443, the similarity search method described by Pearson and Lipman, 1988, Proc. Natl Acad. Sci. USA 88, 2444, or the similarity search method described by computer programs, such as GAP, BESTFIT, FASTA, BLAST P, BLAST N, and TFASTA in the Wisconsin Genetics Software Package, Genetics Computer Group, 575 Science Drive, Madison, Wis. For example, the percent identity of two sequences can be determined using the publicly available BLASTN or BLASTP algorithms on the website of the National Center for Biotechnology Information (NCBI).

"% identity" or "% homology" can be obtained by determining the number of identical positions corresponding to the sequences to be compared, dividing this number by the number of positions compared (e.g., the number of positions in the reference sequence), and multiplying this result by 100 to obtain %identity. In some embodiments, at least about 50%, at least about 55%, at least about 60%, at least about 65%, at least about 70%, at least about 75%, at least about 80%, at least about 85%, at least about 90%, at least about 95% or about 100% of the region gives the degree of identity. In some embodiments, the degree of identity is given to the entire length of the reference sequence. The alignment for determining sequence identity can be performed with tools known in the art, preferably using optimal sequence alignments, for example, using Align, using standard settings, preferably EMBOSS: needle, Matrix: Blosum62, Gap Open 10.0, Gap Extend 0.5.

In some embodiments, a fragment or variant of a specific nucleic acid or a nucleic acid having a specific degree of identity to a specific nucleic acid has at least one functional property of the specific nucleic acid and is preferably functionally equivalent to the specific nucleic acid, such as a nucleic acid that exhibits properties that are the same as or similar to those of the specific nucleic acid.

"Nucleotide" herein includes deoxyribonucleotide, ribonucleotide, deoxyribonucleotide derivative, and ribonucleotide derivative. As used herein, "ribonucleotide" is a constituent substance of ribonucleic acid (RNA), composed of one molecule of base, one molecule of pentose and one molecule of phosphoric acid, which refers to a nucleotide having a hydroxyl group at the 2' position of the β-D-ribofuranosyl group, while "deoxyribonucleotide" is a constituent substance of deoxyribonucleic acid (DNA), also composed of one molecule of base, one molecule of pentose and one molecule of phosphoric acid, which refers to a nucleotide in which the hydroxyl group at the 2' position of the β-D-ribofuranosyl group is replaced by hydrogen, and is the major chemical components of the chromosome.

"Nucleotide" is usually referred to by a single letter representing the base: "A" or "A nucleotide" refers to adenine deoxyribonucleotide or adenine ribonucleotide containing adenine, "C" or "C nucleotide" refers to cytosine deoxyribonucleotide or cytosine ribonucleotide containing cytosine, "G" or "G nucleotide" refers to guanine deoxyribonucleotide or guanine ribonucleotide containing guanine, and "U" or "U nucleotide" refers to guanine deoxyribonucleotide or guanine ribonucleotide containing guanine. "U" or "U nucleotide" refers to an uracil ribonucleotide containing uracil, and "T" or "T nucleotide" refers to a thymine deoxyribonucleotide containing thymine.

As used herein, the term "nucleic acid" generally refers to a polymer containing deoxyribonucleotides (deoxyribonucleic acid, referred to as DNA) or a polymer containing ribonucleotides (ribonucleic acid, referred to as RNA) or any compound of a combination thereof. In addition, nucleic acids herein also include derivatives of nucleic acids. The term "derivatives of nucleic acids" includes chemical derivatization of nucleic acids on the bases, sugars or phosphates of nucleotides, as well as nucleic acids containing non-natural nucleotides and nucleotide analogs. In addition, the nucleic acid herein can be in the form of single-stranded or double-stranded linear or covalently closed circular molecule.

"Polynucleotide sequence", "nucleic acid sequence" and "nucleotide sequence" can be used interchangeably to indicate the order of nucleotides in a polynucleotide. It should be understood by those skilled in the art that a DNA coding strand (sense strand) and its encoded RNA can be regarded as having the same nucleotide sequence, and deoxythymidylic acid in the DNA coding strand sequence corresponds to uridine acid in the RNA sequence encoded by it. An RNA encoded by a DNA refers to an RNA corresponding to the DNA, i.e., a polynucleotide in which all T in the DNA is replaced by U, for example, an RNA encoded by a polynucleotide as set forth in at least one of the sequences SEQ ID NOs: 47 to 67 refers to an RNA corresponding to a polynucleotide (DNA) as set forth in at least one of the sequences SEQ ID NOs: 47 to 67, i.e., a polynucleotide in which all T in the polynucleotide as set forth in at least one of the sequences SEQ ID NOs: 47 to 67 is replaced by U. An RNA sequence corresponding to a DNA sequence refers to a nucleic acid sequence in which all T in the DNA sequence is replaced by U. For example, the RNA sequence corresponding to the nucleic acid sequence as set forth in SEQ ID NOs: 9, 16, 17, 23, 24, 28, 29, 32, 37, 39 or 42 refers to a nucleic acid sequence in which all T in the nucleic acid sequence as set forth in SEQ ID NOs: 9, 16, 17, 23, 24, 28, 29, 32, 37, 39 or 42 is replaced by U.

The polynucleotide may comprise one segment or multiple segments (nucleic acid fragments) (e.g., 1, 2, 3, 4, 5, 6, 7, 8 segments). For example, the polynucleotide may comprise a segment encoding a polypeptide of interest (e.g., a polypeptide and polypeptide antigen described herein). In a specific embodiment, the polynucleotide may comprise a segment encoding a polypeptide of interest and a regulatory segment (including but not limited to a segment for transcriptional regulation and translational regulation). In one embodiment, the regulatory segment comprises a polynucleotide corresponding to one or more of the following regulatory elements: a promoter, a 5' untranslated region (5'-UTR), a 3' untranslated region (3'-UTR), and a poly (A) tail.

The term "promoter" refers to a polynucleotide located upstream of the 5' end of the coding region of a gene, which contains a conserved sequence required for specific binding and transcription initiation by RNA polymerase, can activate RNA polymerase, and enable RNA polymerase to accurately bind to template DNA and have the specificity of transcription initiation. Promoters can be derived from viruses, bacteria, fungi, plants, insects and animals. Representative examples of promoters include bacteriophage T7 promoter, bacteriophage T3 promoter, SP6 promoter, lac operon-promoter, tac promoter, SV40 late promoter, SV40 early promoter, RSV-LTR promoter, CMV IE promoter, SV40 early promoter or SV 40 late promoter and CMV IE promoter.

As used herein, the term "5' untranslated region" or "5'-UTR" can be an RNA sequence that is located upstream of a coding sequence and is not translated into a protein in an mRNA. The 5'-UTR in a gene usually starts at the transcription start site and ends at the nucleotide upstream of the translation start codon of the coding sequence. The 5'-UTR can contain elements that control gene expression, such as a ribosome binding site, a 5'-terminal oligopyrimidine tract, and a translation initiation signal such as a Kozak sequence. mRNA can be post-transcriptionally modified by adding a 5' cap. Therefore, the 5'-UTR in a mature mRNA can also refer to an RNA sequence between the 5' cap and the start codon.

As used herein, the term "3' untranslated region" or "3'-UTR" can be an RNA sequence that is located downstream of a coding sequence and is not translated into a protein in an mRNA. The 3'-UTR in mRNA is located between the stop codon and the poly(A) sequence of the coding sequence, for example, starting from the nucleotide downstream of the stop codon and ending at the nucleotide upstream of the poly(A) sequence.

As used herein, "5' or 3'-UTR derived from gene A" refers to 5' or 3'-UTR of mRNA from gene A. The 5' or 3'-UTR derived from gene A may be the entire 5' or 3'-UTR of mRNA of gene A, or may be a partial 5' or 3'-UTR of mRNA of gene A, and the partial 5' or 3'-UTR of mRNA of gene A includes a partial 5'-UTR formed by splicing multiple fragments of 5'-UTR of mRNA of gene A or a partial 3'-UTR formed by splicing multiple fragments of 3' -UTR of mRNA of gene A.

As used herein, the terms "polyadenine, "polyA", "poly(A) sequence" and "poly(A) tail" can be used interchangeably, and naturally occurring poly(A) sequences are generally composed of adenine ribonucleotides. According to the present invention, the term "modified poly(A) sequence" refers to a poly(A) sequence comprising nucleotide(s) or nucleotide segment(s) other than adenine ribonucleotides. The poly(A) sequence is generally located at the 3' end of the mRNA, for example, the 3' end (downstream) of the 3'-UTR.

As used herein, the term "5'-cap structure" is generally located at the 5' end of the mature mRNA. In some embodiments, the 5'-cap structure is connected to the 5'-end of the mRNA via a 5'-5'-triphosphate bond. The 5'-cap structure is generally formed by modified (e.g., methylated) ribonucleotides (especially by guanine nucleotide derivatives), for example, m7GpppN (cap 0 or known as "cap0", which is a cap structure formed by the reaction between 5' phosphate group of hnRNA with the 5'-phosphate group of m7GTP under the action of guanylyl transferase to form a 5',5'-phosphodiester bond), wherein N is the terminal 5' nucleotide of the nucleic acid carrying the 5'-cap structure. In some embodiments, the 5'-cap structure includes, but is not limited to, cap 0, cap 1 (a cap structure formed by further methylating the 2'-OH of the first nucleotide sugar group of hnRNA on the basis of cap 0, or known as "cap1"), cap 2 (a cap structure formed by further methylating the 2'-OH of the second nucleotide sugar group of hnRNA on the basis of cap 1, or "cap2"), cap 4, cap 0 analogs, cap 1 analogs, cap 2 analogs or cap 4 analogs.

As used herein, the term "expression" includes transcription and/or translation of a nucleotide sequence. Therefore, expression can involve the production of a transcript and/or a polypeptide. The term "transcription" refers to the process of transcribing the genetic code in a DNA sequence into RNA (transcript). The term "in vitro transcription" refers to the in vitro synthesis of RNA, particularly mRNA, in a cell-free system (e.g., in an appropriate cell extract) (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). The vector that can be used to produce a transcript is also called a "transcription vector", which contains the regulatory sequence required for transcription. The term "transcription" encompasses "in vitro transcription".

As used herein, the term "polypeptide" refers to a polymer comprising two or more amino acids covalently linked by peptide bond(s). A "protein" may comprise one or more polypeptides, wherein the polypeptides interact with each other by covalent or non-covalent means.

As used herein, the term "host cell" refers to a cell for receiving, maintaining, replicating, expressing a polynucleotide or a vector. The term "host cell" includes prokaryotic cells (e.g., *Escherichia coli)* or eukaryotic cells (e.g., yeast cells and insect cells), for example, cells from human, mouse, hamster, pig, goat, primate. The cell can be derived from a variety of tissue types and includes primary cells and cell lines. Some specific examples include keratinocytes, peripheral blood leukocytes, bone marrow stem cells, and embryonic stem cells. In other embodiments, the host cell is an antigen presenting cell, particularly a dendritic cell, a monocyte, or a macrophage. The nucleic acid can be present in a host cell in a single copy or in several copies. In some embodiments, the host cell can be a cell wherein a polypeptide of the present invention is expressed.

As used herein, the term "recombination" or "recombinant" means "produced by genetic engineering". In some embodiments, in the context of the present invention, "recombinant material", such as a recombinant RNA molecule, is non-naturally occurring. The term "naturally occurring" or "naturally present" as used herein refers to the fact that the material can be found in nature. For example, a peptide or nucleic acid that is present in an organism (including a virus) and can be isolated from a source in nature and has not been intentionally modified by man in an experiment is naturally occurring.

In the context of the present invention, the term "plasmid" generally refers to a circular DNA molecule, though the term can also encompass linear DNA molecules. In particular, the term "plasmid" also covers molecules which result from linearizing a circular plasmid by cutting it, e.g. with a restriction enzyme, thereby converting the circular plasmid molecule into a linear molecule. Plasmids can replicate, i.e. amplify in a cell independently from the genetic information stored as chromosomal DNA in the cell and can be used for cloning, i.e. for amplifying genetic information in a bacterial cell. In some embodiments, the DNA plasmid is a medium- or high-copy plasmid. In some embodiments, the DNA plasmid is a high-copy plasmid. Examples of such high copy plasmids include, for example, pUC and pTZ plasmids or any other plasmids which contain an origin of replication (e.g., pMB 1, pCoIE1) that support high copies of the plasmid.

As used herein, the term "vaccine" is typically understood as a prophylactic or therapeutic material that provides at least one antigen or has antigenic function, and can stimulate the adaptive immune system in the body to provide an adaptive immune response.

As used herein, the term "antigen" refers typically to a substance which may be recognized by the immune system, preferably by the adaptive immune system, and is capable of triggering an antigen-specific immune response, e.g. by formation of antibodies and/or antigen-specific T cells. Typically, an antigen may be or may comprise a peptide or protein which may be presented by the MHC to T-cells. In the sense of the present invention an antigen may be the product of translation of a provided nucleic acid molecule, e.g., RNA, RNA molecules, DNA herein. In addition, fragments, variants, and derivatives of peptides or proteins derived from peptides or proteins comprising at least one epitope or antigen (e.g., tumor antigens, viral antigens, bacterial antigens, protozoan antigens) can be understood as antigens.

As used herein, "treatment" and "treating" and the like generally mean obtaining a desired pharmacological and physiological effect. Thus, the treatment of the present invention may relate to the treatment of a certain disease state, but may also relate to prophylactic treatment in terms of completely or partially preventing a disease or its symptoms. **In** some embodiments, the term "treatment/treating" is understood to be therapeutic in terms of a partial or complete cure of a disease and/or adverse effect attributed to the disease and/or symptoms. Treatment may also be prophylactic or preventive treatment, i.e., measures taken to prevent a disease, for example, to prevent infection and/or the onset of a disease.

As used herein, the terms "subject" and "patient" can be used interchangeably. **In** certain embodiments, the subject is a mammal, such as a human, a non-human primate (e.g., apes, chimpanzees, monkeys, and orangutans), a domestic animal (including dogs and cats and livestock (e.g., horses, cattle, pigs, sheep, and goats)), or other mammals. Additional mammals include, but are not limited to, mice, rats, guinea pigs, rabbits, hamsters, etc. **In** a specific embodiment, the subject is a human. **In** one embodiment, the subject is a mammal (e.g., a human) suffering from an infectious disease or a neoplastic disease. **In** another embodiment, the subject is a mammal (e.g., a human) at risk of developing an infectious disease or a neoplastic disease.

As used herein, the term "administer" refers to providing or administering an agent to a subject by any effective route. Exemplary routes of administration include, but are not limited to, one or more of the following: injection (e.g., subcutaneous, intramuscular, intradermal, intraperitoneal, intrathecal, intracerebroventricular, or intravenous), oral, intraluminal bile duct, sublingual, rectal, transdermal, intranasal, vaginal, and inhalation. When used to treat a disease, disorder, condition, or symptom thereof, administration of the agent is typically performed after the onset of the disease, disorder, condition, or symptom thereof. When used to prevent a disease, disorder, condition, or symptom, administration of the agent is typically performed before the onset of the disease, disorder, condition, or symptom.

Herein, some elements of the present application will be described. These elements are listed with particular embodiments, but it should be understood that they may be combined in any manner and in any number to create additional embodiments. The examples and preferred embodiments described differently should not be construed as limiting the invention to only the explicitly described embodiments. The description should be understood to support and cover embodiments that combine the explicitly described embodiments with any number of disclosed and/or preferred elements. Furthermore, unless the context indicates otherwise, any permutation and combination of all described elements in the present invention should be considered to be disclosed by the specification of the present invention. For example, in one embodiment, the 5'-UTR of the RNA molecule comprises a polynucleotide having a sequence as set forth in SEQ ID NO: 9, and in another embodiment, the 3'-UTR of the RNA molecule comprises a polynucleotide having a sequence as set forth in SEQ ID NO: 10, then the following scheme is also an embodiment of the present invention: the 5'-UTR of the RNA molecule comprises a polynucleotide having a sequence as set forth in SEQ ID NO: 9, and the 3'-UTR of the RNA molecule comprises a polynucleotide having a sequence as set forth in SEQ ID NO: 10.

**In** the first aspect, the present invention relates to an RNA encoding the S protein of SARS-COV-2, a vaccine comprising the RNA, and uses thereof.

In some embodiments, the present invention provides an RNA encoding the S protein of SARS-COV-2, wherein the S protein comprises an amino acid sequence as set forth in SEQ ID NO: 13. In some embodiments, the amino acid sequence of the S protein is set forth in SEQ ID NO: 13; in some embodiments, the RNA is mRNA.

In some embodiments, the present invention provides an RNA encoding the S protein of SARS-COV-2, wherein the S protein comprises at least 95%, 96%, 97%, 98%, 99%, 99.5%, 99.6%, 99.7%, 99.8% or 99.9% identity to the amino acid sequence as set forth in SEQ ID NO: 13.

In some embodiments, the RNA comprises the nucleic acid sequence as set forth in SEQ ID NO: 14. In some embodiments, the nucleic acid sequence of the RNA is set forth in SEQ ID NO: 14.

In some embodiments, part or all of the cytosine and/or uracil in the RNA is chemically modified to improve the stability of the RNA *in vivo.*

In some embodiments, the chemical modification comprises the following:
one or more uridine nucleosides, such as 1, 2, 3, 4, 5, 6, 7, 8, 9, 10 uridine nucleosides or at least 50%, at least 60%, at least 70%, at least 80%, at least 90% or 100% of the uridine nucleosides in the RNA are replaced by at least one nucleoside selected from the group consisting of pseudouridine, N1-methyl-pseudouridine, N1-ethyl pseudouridine, 2-thiouridine, 4'-thiouridine, 5-methylcytosine, 5-methyluridine, 2-thio-1-methyl-1-deaza-pseudouridine, 2-thio T-methyl-pseudouridine, 2-thio-5-aza-uridine, 2-thio-dihydro-pseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine or 5-methoxyuridine and 2'-O-methyluridine. In an optional specific embodiment, the following nucleoside substitutions are selected: pseudouridine, N1-methyl pseudouridine or N1-ethyl pseudouridine; and/or
one or more cytosine nucleosides in the RNA, such as 1, 2, 3, 4, 5, 6, 7, 8, 9, 10 cytosine nucleosides or at least 50%, at least 60%, at least 70%, at least 80%, at least 90% or 100% of the cytosine nucleosides are replaced by 5-methylcytosine nucleoside.

In some embodiments, all or part of the uridine nucleosides in the RNA are replaced by pseudouridine. In some embodiments, all or part of the uridine nucleosides in the RNA are replaced by N1-methyl pseudouridine. In some embodiments, all or part of the uridine nucleosides in the nucleic acid sequence as set forth in SEQ ID NO: 14 are replaced by pseudouridine, preferably N1-methyl pseudouridine.

In some embodiments, all uridine nucleosides in the nucleic acid sequence as set forth in SEQ ID NO: 14 are replaced by N1-methyl pseudouridine.

In some embodiments, the RNA further comprises at least one of a 5'-cap structure, a 5'-UTR, a 3'-UTR, and a polyA.

In some embodiments, the 5'-cap structure is selected from at least one of m⁷GpppG, m₂^{7,3'-O}GpppG, m⁷Gppp(5')N1 or m⁷Gppp(m^{2'-O})N1, preferably m⁷Gppp(5')N1 or m⁷Gppp(m^{2'-O})N1, wherein "m7G" represents a 7-methylguanosine cap nucleoside, "ppp" represents a triphosphate bond between the 5' carbon of the cap nucleoside and the first nucleotide of the primary RNA transcript, N1 is the 5'-most nucleotide, "G" represents a guanosine nucleoside, "7" represents a methyl -group at the 7-position of guanine, and "m^{2'-O"} represents a methyl group at the 2' O-position of the nucleotide;
and/or the 5'-UTR is selected from the following (1)-(5):
(1) a 5'-UTR comprising an RNA sequence corresponding to the nucleic acid sequence as set forth in SEQ ID NOs: 9, 16, 17, 23, 24, 28, 29, 32, 37, 39 or 42, a homologue, a fragment or a variant thereof, wherein the homologue, the fragment or the variant has the same or better function of improving translation efficiency as the 5'-UTR shown in the RNA sequence corresponding to SEQ ID NOs: 9, 16, 17, 23, 24, 28, 29, 32, 37, 39 or 42; in some embodiments, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 9, 16, 17, 23, 24, 28, 29, 32, 37, 39 or 42;
(2) a 5'-UTR consisting of an RNA sequence corresponding to the nucleic acid sequence as set forth in SEQ ID NOs: 9, 16, 17, 23, 24, 28, 29, 32, 37, 39 or 42;
(3) a 5'-UTR consisting of an RNA sequence corresponding to the nucleic acid sequence as set forth in SEQ ID NO: 9;
(4) a 5'-UTR obtained by concatenating two or more identical 5'-UTRs in (1) to(3) above; or
(5) a 5'-UTR obtained by concatenating two or more different 5'-UTRs in (1) to (3) above;
   and/or the 3'-UTR is selected from the following (1)-(5):
      (1) a 3'-UTR comprising an RNA sequence corresponding to the nucleic acid sequence as set forth in SEQ ID NOs: 47-67 or 10, a homologue, a fragment or a variant thereof; the homologue, the fragment or the variant has the same or better function of improving translation efficiency and/or stability as the 3'-UTR shown in the RNA sequence corresponding to SEQ ID NOs: 47-67 or 10; in some embodiments, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 47-67 or 10;
      (2) a 3'-UTR consisting of an RNA sequence corresponding to the nucleic acid sequence as set forth in SEQ ID NOs: 47-67 or 10;
      (3) a 3'-UTR consisting of an RNA sequence corresponding to the nucleic acid sequence as set forth in SEQ ID NO: 10;
      (4) a 3'-UTR obtained by concatenating two or more identical 3'-UTRs in (1) to (3) above; or
      (5) a 3'-UTR obtained by concatenating two or more different 3'-UTRs in (1) to (3) above;
   and/or the polyA is a truncated polyA, i.e., a 10 bp non-A linker sequence is added after a plurality of consecutive nucleotides A and then a plurality of consecutive nucleotides A is added; in some embodiments, the polyA is 30 consecutive nucleotides A, then a 10 bp non-A linker sequence and then 70 consecutive nucleotides A;
   in some embodiments, the polyA is selected from one of the following (1)-(3):
      (1) a polyA comprising an RNA sequence corresponding to the nucleic acid sequence as set forth in SEQ ID NOs: 68-70, 72, 75 or 11, a homologue, a fragment or a variant thereof, wherein the homologue, the fragment or the variant has the same or better function of improving translation efficiency and/or stability as the polyA shown in the RNA sequence corresponding to SEQ ID NOs: 68-70, 72, 75 or 11; in some embodiments, the homologue comprises a nucleic acid sequence having at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 68-70, 72, 75 or 11;
      (2) a polyA consisting of an RNA sequence corresponding to the nucleic acid sequence as set forth in SEQ ID NOs: 68-70, 72, 75 or 11;
      (3) a polyA consisting of an RNA sequence corresponding to the nucleic acid sequence as set forth in SEQ ID NO: 11.

In some embodiments, the RNA comprises the nucleic acid sequence as set forth in SEQ ID NO: 15. In some embodiments, the nucleic acid sequence of the RNA is set forth in SEQ ID NO: 15. The sequence of SEQ ID NO: 15 itself already comprises a cap structure: cap G¹G² = m⁷G⁺-5'-ppp-5'-Gm^{2'}-3'-p- [m⁷ = 7-CH₃; m^{2'} = 2'-O-CH₃; - ppp- = -PO₂H-O-PO₂H-O-PO₂H)-; -p- = -PO₂H-]. It should be noted that, according to WIPO standard ST.26 for nucleotide or amino acid sequence listings, the t (thymine) in the RNA sequence (e.g., SEQ ID NO: 14, SEQ ID NO: 15) in the sequence listing is actually u (uracil).

In embodiments wherein the RNA comprises the nucleic acid sequence as set forth in SEQ ID NO: 15, all or part of the uridine nucleosides in the RNA are replaced by pseudouridine, preferably N1-methyl pseudouridine; in some embodiments, all or part of the uridine nucleosides in the nucleic acid sequence as set forth in SEQ ID NO: 15 are replaced by pseudouridine, preferably N1-methyl pseudouridine; in some embodiments, all uridine nucleosides in the nucleic acid sequence as set forth in SEQ ID NO: 15 are replaced by N1-methyl pseudouridine.

In some embodiments, the present invention provides a protein expressed by any of the aforementioned RNAs; in some embodiments, the protein comprises an amino acid sequence as set forth in SEQ ID NO: 13, and in some embodiments, its amino acid sequence is set forth in SEQ ID NO: 13.

In some embodiments, the present invention provides a DNA encoding the RNA of any of the above embodiments; in some embodiments, the DNA comprises the nucleic acid sequence as set forth in SEQ ID NO: 12; in some embodiments, the DNA comprises the nucleic acid sequence as set forth in SEQ ID NO: 8.

In some embodiments, the present invention provides a vector comprising the DNA of any of the above embodiments.

In some embodiments, the present invention provides a host cell comprising the vector of any of the above embodiments.

In some embodiments, the present invention provides a lipid nanoparticle comprising the RNA of any one of the above embodiments.

In some embodiments, the lipid nanoparticles further contain one or more of an ionizable cationic lipid, a helper lipid, a structural lipid, and a PEG-lipid.

In some embodiments, the ionizable cationic lipid is selected from one or more of Dlin-MC3-DMA, Dlin-KC2-DMA, DODMA, c12-200 or DlinDMA; and/or the helper lipid is selected from one or more of DSPC, DOPE, DOPC or DOPS; and/or the structural lipid is selected from at least one of cholesterol, cholesterol ester, steroid hormone, steroid vitamin and bile acid; and/or the PEG-lipid is selected from PEG-DMG or PEG-DSPE, preferably PEG-DMG. PEG-DMG is a polyethylene glycol (PEG) derivative of 1,2-dimyristyl glyceride. In some embodiments, the average molecular weight of PEG is about 2000 Daltons to 5000 Daltons. In an alternative specific example, the average molecular weight of PEG is about 2000 or 5000 Daltons.

In some embodiments, the present invention provides a pharmaceutical composition comprising the RNA, the protein, the DNA, the vector, the host cell or the lipid nanoparticle, and a pharmaceutically acceptable carrier and /or excipient.

In some embodiments, the present invention provides the use of the RNA, the protein, the DNA, the vector, the host cell or the lipid nanoparticle in the preparation of a vaccine for preventing SARS-COV-2 infection. In some embodiments, the SARS-COV-2 includes Alpha (such as B.1.1.7 variants and their progeny lineages), Beta (such as B.1.351 variants and their progeny lineages), Gamma (such as P.1 variants and their progeny lineages), Delta (such as B.1.617.2 and AY lineages) and Omicron, etc.

In some embodiments, the present invention provides a method for controlling, preventing or treating an infectious disease caused by SARS-COV-2 in a subject, comprising administering a therapeutically effective amount of the RNA, the protein, the DNA, the vector, the host cell, the lipid nanoparticle or the pharmaceutical composition to the subject.

In some embodiments, the subject is a human or non-human mammal. In some embodiments, the subject is an adult, an elderly person, a child, or a toddler. In some embodiments, the subject is at risk of or susceptible to SARS-COV-2 infection. In some embodiments, the subject has been diagnosed with positive SARS-COV-2 infection or is asymptomatic.

**In a second aspect,** the present invention also relates to a universal polynucleotide molecule that can be used to prepare a novel RNA vaccine (eg, an mRNA vaccine), which comprises a 5'-UTR and/or a 3'-UTR.

In some embodiments, the present invention provides an artificial RNA molecule comprising a 5'-UTR and a nucleic acid sequence encoding a polypeptide and/or protein of interest, wherein the 5'-UTR is selected from the following (1)-(5):
(1) a 5'-UTR comprising an RNA sequence corresponding to the nucleic acid sequence as set forth in SEQ ID NOs: 9, 16, 17, 23, 24, 28, 29, 32, 37, 39 or 42, a homologue, a fragment or a variant thereof, wherein the homologue, the fragment or the variant has the same or better function of improving translation efficiency as the 5'-UTR shown in the RNA sequence corresponding to SEQ ID NOs: 9, 16, 17, 23, 24, 28, 29, 32, 37, 39 or 42; in some embodiments, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 9, 16, 17, 23, 24, 28, 29, 32, 37, 39 or 42;
(2) a 5'-UTR consisting of an RNA sequence corresponding to the nucleic acid sequence as set forth in SEQ ID NOs: 9, 16, 17, 23, 24, 28, 29, 32, 37, 39 or 42;
(3) a 5'-UTR consisting of an RNA sequence corresponding to the nucleic acid sequence as set forth in SEQ ID NO: 9;
(4) a 5'-UTR obtained by concatenating two or more identical 5'-UTRs in (1) to (3) above; or
(5) a 5'-UTR obtained by concatenating two or more different 5'-UTRs in (1) to (3) above.

Herein, "A, a homologue, a fragment or a variant thereof" refers to A, a homologue of A, a fragment of A or a variant of A, for example, "a 5'-UTR comprising an RNA sequence corresponding to the nucleic acid sequence as set forth in SEQ ID NOs: 9, 16, 17, 23, 24, 28, 29, 32, 37, 39 or 42, a homologue, a fragment or a variant thereof" refers to a 5'-UTR comprising an RNA sequence corresponding to the nucleic acid sequence as set forth in SEQ ID NOs: 9, 16, 17, 23, 24, 28, 29, 32, 37, 39 or 42, a homologue of the 5'-UTR, a fragment of the 5'-UTR or a variant of the 5'-UTR.

In some embodiments, the RNA molecule further comprises a 3'-UTR and a polyA. In some embodiments, the 3'-UTR is selected from the following (1)-(5):
(1) a 3'-UTR comprising an RNA sequence corresponding to the nucleic acid sequence as set forth in SEQ ID NOs: 47-67 or 10, a homologue, a fragment or a variant thereof; the homologue, the fragment or the variant has the same or better function of improving translation efficiency and/or stability as the 3'-UTR shown in the RNA sequence corresponding to SEQ ID NOs: 47-67 or 10; in some embodiments, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 47-67 or 10;
(2) a 3'-UTR consisting of an RNA sequence corresponding to the nucleic acid sequence as set forth in SEQ ID NOs: 47-67 or 10;
(3) a 3'-UTR consisting of an RNA sequence corresponding to the nucleic acid sequence as set forth in SEQ ID NO: 10;
(4) a 3'-UTR obtained by concatenating two or more identical 3'-UTRs in (1) to (3) above; or
(5) a 3'-UTR obtained by concatenating two or more different 3'-UTRs in (1) to (3) above.

In some embodiments, the polyA is a truncated polyA, i.e., a 10 bp non-A linker sequence is added after a plurality of consecutive nucleotides A and then a plurality of consecutive nucleotides A is added; in some embodiments, the polyA is 30 consecutive nucleotides A, then a 10 bp non-A linker sequence and then 70 consecutive nucleotides A;
In some embodiments, the polyA is selected from the following (1)-(3):
(1) a polyA comprising an RNA sequence corresponding to the nucleic acid sequence as set forth in SEQ ID NOs: 68-70, 72, 75 or 11, a homologue, a fragment or a variant thereof, wherein the homologue, the fragment or the variant has the same or better function of improving translation efficiency and/or stability as the polyA shown in the RNA sequence corresponding to SEQ ID NOs: 68-70, 72, 75 or 11; in some embodiments, the homologue comprises a nucleic acid sequence having at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 68-70, 72, 75 or 11;
(2) a polyA consisting of an RNA sequence corresponding to the nucleic acid sequence as set forth in SEQ ID NOs: 68-70, 72, 75 or 11; or
(3) a polyA consisting of an RNA sequence corresponding to the nucleic acid sequence as set forth in SEQ ID NO: 11.

In some embodiments, the present invention also provides another RNA molecule comprising:
(1) a first nucleotide sequence encoding a polypeptide and/or protein of interest; and
(2) a second nucleotide sequence containing a 3'-untranslated region (3'-UTR);

The 3'-UTR comprises at least one of the following polynucleotides:
(a): a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, and GHRL; in some embodiments, a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, and GHRL;
(b): a fragment of the 3'-UTR described in (a);
(c): a variant of the 3'-UTR described in (a); and
(d): a variant of the fragment described in (b);
the first nucleotide sequence and the second nucleotide sequence do not naturally occur in the same RNA molecule.

In some embodiments, the gene is a gene of a eukaryotic organism. In some embodiments, the gene is a gene of an animal. In some embodiments, the gene is a gene of a chordate, such as a gene of a human or a mouse.

In some embodiments, the gene is a human gene.

In some embodiments, the GenBank accession number of the APOA1 gene is NM_000039.3; the Gene ID of the UL122 gene is 3077563 (corresponding to GenBank accession number 170689..174090 of NC_006273.2), the GenBank accession number of the COP1 gene is AY885669; the GenBank accession number of the MYSM1 gene is NM_001085487.3; the GenBank accession number of the ASAH2B gene is NM_001079516.4; the GenBank accession number of the NDUFAF2 gene is NG_008978.1; the GenBank accession number of the APOD gene is NM_001647; the GenBank accession number of the HBB gene is MK476504.1; the GenBank accession number of the TF gene is NM_001063.4; the GenBank accession number of the TMSB4X gene is BC001631.1; the GenBank accession number of the CPAMD8 gene is NM_015692.5; the GenBank accession number of the VIM gene is NM_003380.5; The GenBank accession number of HDGFL1 gene is NM_138574.4; the GenBank accession number of TTR gene is NM_000371; the GenBank accession number of SRM gene is NM_003132.3; the GenBank accession number of HBA1 gene is NM_000558.5; the GenBank accession number of PRPF8 gene is NM_006445.4; the GenBank accession number of LMBRD1 gene is NM_001367272.1; the GenBank accession number of IFNA1 gene is NM_024013.3; the GenBank accession number of CCDC146 gene is NM_020879.3; the GenBank accession number of GHRL gene is NM_001134941.3; and the GenBank accession number of GH1 gene is NM_000515.5.

In some embodiments, the second nucleotide sequence comprises at least one of the following polynucleotides:
(e): 3'-UTR derived from at least one of the genes COP1 and HDGFL1;
(f): a fragment of the 3'-UTR described in (e);
(g): a variant of the 3'-UTR described in (e); and
(h): a variant of the fragment described in (f).

In some embodiments, the second nucleotide sequence comprises at least one of the following polynucleotides: an RNA encoded by a polynucleotide having a sequence as set forth in at least one of SEQ ID NOs: 47 to 67, a fragment of an RNA encoded by a polynucleotide having a sequence as set forth in at least one of SEQ ID NOs: 47 to 67, a variant of an RNA encoded by a polynucleotide having a sequence as set forth in at least one of SEQ ID NOs: 47 to 67, and a variant of a fragment of an RNA encoded by a polynucleotide having a sequence as set forth in at least one of SEQ ID NOs: 47 to 67.

In some embodiments, the variant of an RNA encoded by a polynucleotide having a sequence as set forth in at least one of SEQ ID NOs: 47 to 67, the fragment of an RNA encoded by a polynucleotide having a sequence as set forth in at least one of SEQ ID NOs: 47 to 67, and the variant of a fragment of an RNA encoded by a polynucleotide having a sequence as set forth in at least one of SEQ ID NOs: 47 to 67 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 .

In some embodiments, the fragment, the variant, or the variant of a fragment of an RNA encoded by a polynucleotide having a sequence as set forth in at least one of SEQ ID NOs: 47 to 67 has at least 1, 2, 3, 4, 5, 6, 7, 8, 9, 10 or more nucleotides inserted, added, deleted or substituted compared to the RNA encoded by a polynucleotide having a sequence as set forth in one of SEQ ID NOs: 47 to 67.

It should be noted that, "a fragment, a variant or a variant of a fragment of A" herein is an abbreviation of a fragment of A, a variant of A or a variant of a fragment of A. For example, "a fragment, a variant or a variant of a fragment of RNA encoded by a polynucleotide having a sequence as set forth in at least one of SEQ ID NOs: 47 to 67" refers to a fragment of an RNA encoded by a polynucleotide having a sequence as set forth in at least one of SEQ ID NOs: 47 to 67, a variant of an RNA encoded by a polynucleotide having a sequence as set forth in at least one of SEQ ID NOs: 47 to 67, or a variant of a fragment of an RNA encoded by a polynucleotide having a sequence as set forth in at least one of SEQ ID NOs: 47 to 67. In some embodiments, a fragment of A, a variant of A, or a variant of a fragment of A has the same or better performance as A, such as improving the translation efficiency and/or stability of a nucleic acid (e.g., mRNA).

In some embodiments, the second nucleotide sequence comprises at least one of: 3'-UTR of at least two genes among the genes described in (a); a fragment of 3'-UTR of at least two genes among the genes described in (a); a variant of 3'-UTR of at least two genes among the genes described in (a); and a variant of a fragment of 3'-UTR of at least two genes among the genes described in (a). In some embodiments, the at least two genes are two genes, three genes, four genes, five genes, six genes, seven genes, eight genes, nine genes, or ten genes.

In some embodiments, the second nucleotide sequence comprises at least one of: at least two copies of the 3'-UTR in (a), at least two copies of a fragment of the 3'-UTR in (a), at least two copies of a variant of the 3'-UTR in (a), and at least two copies of a variant of a fragment of the 3'-UTR in (a). In some embodiments, the at least two copies are two copies, three copies, four copies, five copies, six copies, seven copies, eight copies, nine copies, or ten copies.

In some embodiments, the RNA molecule further comprises at least one of a promoter, a 5'- cap structure, a 5'-UTR and a polyA.

In some embodiments, the RNA molecule further comprises at least one of a 5'-cap structure, a 5'-UTR and a polyA.

In some embodiments, the 5'-cap structure comprises at least one of m⁷GpppG, m₂^{7,3'-O}GpppG, m⁷Gppp(5')N1 and m⁷Gppp(m^{2'-O})N1.

In some embodiments, the 5' -UTR comprises one of i) to iv):
i) at least one of: a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D, a fragment, a variant and a variant of a fragment thereof;
ii) at least one of the following polynucleotides: an RNA encoded by a polynucleotide having a sequence as set forth in SEQ ID NO: 9, a fragment of an RNA encoded by a polynucleotide having a sequence as set forth in SEQ ID NO: 9, a variant of an RNA encoded by a polynucleotide having a sequence as set forth in SEQ ID NO: 9, and a variant of a fragment of an RNA encoded by a polynucleotide having a sequence as set forth in SEQ ID NO: 9.
iii) at least two copies of one of the polynucleotides in i) or ii); or
iv) at least two polynucleotides in i).

In some embodiments, the species or specific GenBank accession numbers of the source genes of the 5' -UTR can refer to the relevant description regarding the third nucleotide sequence below, which will not be repeated here.

In some embodiments, the 5' -UTR in i) comprises or is one or more of the following: an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46.

In some embodiments, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and the variant of a fragment of an RNA encoded by a polynucleotide having a sequence as set forth in at least one of SEQ ID NOs: 16 to 46 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46.

In some embodiments, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 in ii) have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In some embodiments, the 3'-UTR is derived from one or more of the 3'-UTR of the COP1 gene, a fragment, a variant, and a variant of a fragment thereof, and the 5'-UTR comprises one or more of the following polynucleotides: an RNA encoded by a polynucleotide having a sequence as set forth in SEQ ID NO: 9, a fragment, a variant, and a variant of a fragment thereof. In an alternative specific example, the 3'-UTR is one or more of an RNA encoded by a polynucleotide having a sequence as set forth in SEQ ID NO: 49, a fragment, a variant, and a variant of a fragment thereof. It should be noted that "a fragment, a variant or a variant of a fragment of A" herein refers to a fragment of A, a variant of A or a variant of a fragment of A. For example, "a fragment, variant or variant of a fragment of an RNA encoded by a polynucleotide having a sequence as set forth in SEQ ID NO: 9" refers to a fragment of an RNA encoded by a polynucleotide having a sequence as set forth in SEQ ID NO: 9, a variant of an RNA encoded by a polynucleotide having a sequence as set forth in SEQ ID NO: 9, or a variant of a fragment of an RNA encoded by a polynucleotide having a sequence as set forth in SEQ ID NO: 9.

In some embodiments, the 3'-UTR is derived from one or more of the 3'-UTR of the HDGFL1 gene, a fragment, a variant, and a variant of a fragment thereof, and the 5'-UTR comprises one or more of the following polynucleotides: an RNA encoded by a polynucleotide having a sequence as set forth in SEQ ID NO: 9, a fragment, a variant, and a variant of a fragment thereof. In an alternative specific example, the 3'-UTR is one or more of an RNA encoded by a polynucleotide having a sequence as set forth in SEQ ID NO: 59, a fragment, a variant, and a variant of a fragment thereof.

In some embodiments, the fragment, the variant and the variant of a fragment of an RNA encoded by a polynucleotide having a sequence as set forth in SEQ ID NO: 9, 49, or 59 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9, 49, or 59.

In some embodiments, the present invention also provides another RNA molecule comprising:
(1) a first nucleotide sequence encoding a polypeptide and/or protein of interest; and
(2) a third nucleotide sequence containing a 5'-untranslated region (5'-UTR);

the 5'-UTR comprises at least one of the following polynucleotides:
   (a): a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D;
   (b): a fragment of the 5'-UTR described in (a);
   (c): a variant of the 5'-UTR described in (a); and
   (d): a variant of the fragment described in (b);
the first nucleotide sequence and the third nucleotide sequence do not naturally occur in the same RNA molecule.

In some embodiments, the gene is a gene of a eukaryotic organism. In some embodiments, the gene is a gene of an animal. In some embodiments, the gene is a gene of a chordate, such as a gene of a human or mouse. In some embodiments, the gene is a human gene.

In some embodiments, the GenBank accession number of the APOA1 gene is NM_000039.3; the GenBank accession number of the CARD16 gene is NM_052889.4; the GenBank accession number of the ALB gene is AH002596.2; the GenBank accession number of the APOC1 gene is NM_001645.5; the GenBank accession number of the EEF1A1 gene is NM_001402.6; the GenBank accession number of the RBP4 gene is BC020633.1; the GenBank accession number of the GHRL gene is NM_016362.5; the GenBank accession number of the MPND gene is NM_032868; the GenBank accession number of the ASAH2B gene is NM_001079516.4; the GenBank accession number of the FBX016 gene is NM_172366.4; the GenBank accession number of FBH1 gene is NG_047206.1; the GenBank accession number of SRM gene is M64231.1; the GenBank accession number of NAAA gene is NM_001363719.2; the GenBank accession number of ACTB gene is NM_001101.5; the GenBank accession number of MKNK2 gene is NM_017572.4; the GenBank accession number of ORM1 gene is NG_012108.1; the GenBank accession number of GADPH gene is NG_007073; the GenBank accession number of NDUFAF2 gene is NG_008978.1; the GenBank accession number of FGFR2 gene is NM_000141.5; the GenBank accession number of MYCBPAP gene is AK303041.1; the GenBank accession number of CHMP2A gene is NM_198426.3; the GenBank accession number of TSG101 gene is NM_006292.4; the GenBank accession number of PRPF8 gene is NM_006445; the GenBank accession number of NFKB2 gene is NM_001322935; the GenBank accession number of NAE1 gene is NM_001018159; the GenBank accession number of HDGFL1 gene is HM005397; the GenBank accession number of GSDMD gene is BC008904.2; the GenBank accession number of FBXW12 gene is NM_207102.2; the GenBank accession number of FBXW10 gene is XM_054314720.1; the GenBank accession number of FBXL8 gene is NM_018378; the GenBank accession number of the ATG4D gene is NM_032885.6.

In some embodiments, the third nucleotide sequence comprises at least one of the following polynucleotides: an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46.

In some embodiments, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46.

In some embodiments, the fragment, the variant, or the variant of a fragment of an RNA encoded by a polynucleotide having a sequence as set forth in at least one of SEQ ID NOs: 16 to 46 has at least 1, 2, 3, 4, 5, 6, 7, 8, 9, 10 or more nucleotides inserted, added, deleted or substituted compared to the RNA encoded by a polynucleotide having a sequence as set forth in one of SEQ ID NOs: 16 to 46.

In some embodiments, the third nucleotide sequence comprises at least one of: 5'-UTR of at least two genes among the genes in (a), a fragment of 5'-UTR of at least two genes among the genes in (a), a variant of 5'-UTR of at least two genes among the genes in (a), and a variant of the fragment of 5'-UTR of at least two genes among the genes in (a). In some embodiments, the at least two genes are two genes, three genes, four genes, five genes, six genes, seven genes, eight genes, nine genes, or ten genes.

In some embodiments, the third nucleotide sequence comprises: at least two copies of the 5'-UTR in (a), at least two copies of a fragment of the 5'-UTR in (a), at least two copies of a variant of the 5'-UTR in (a), and at least two copies of a variant of a fragment of the 5'-UTR in (a). In some embodiments, the at least two copies are two copies, three copies, four copies, five copies, six copies, seven copies, eight copies, nine copies, or ten copies.

In some embodiments, the RNA molecule further comprises at least one of a promoter, a 5'- cap structure, a 3'-UTR and a polyA.

In some embodiments, the RNA molecule further comprises at least one of a 5'-cap structure, a 3'-UTR and a polyA.

In some embodiments, the 5'- cap structure includes at least one of m⁷ GpppG, m 2 ^{7,3'-O} GpppG, m ⁷ Gppp(5')N1 and m ⁷ Gppp(m ^{2'-O})N1;
In some embodiments, the 3'-UTR comprises one of i) to iii):
i): at least one of a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof; or at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof;
ii) at least two copies of one of the polynucleotides described in i).
iii) at least two polynucleotides in i).

In some embodiments, the species or specific GenBank accession numbers of the source genes of the 3'-UTR can refer to the relevant description regarding the second nucleotide sequence above, which will not be repeated here.

In some embodiments, the 3'-UTR in i) comprises or is one or more of the following: an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10.

In some embodiments, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In some embodiments, the present invention also provides another RNA molecule comprising:
(1) a first nucleotide sequence encoding a polypeptide and/or protein of interest; and
(2) a 5'-untranslated region (5'-UTR); wherein the 5'-UTR comprises at least one of the following polynucleotides: an RNA encoded by a polynucleotide having a sequence as set forth in SEQ ID NO: 9, a fragment of an RNA encoded by a polynucleotide having a sequence as set forth in SEQ ID NO: 9, a variant of an RNA encoded by a polynucleotide having a sequence as set forth in SEQ ID NO: 9, and a variant of a fragment of an RNA encoded by a polynucleotide having a sequence as set forth in SEQ ID NO: 9.

In some embodiments, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9.

In some embodiments, the fragment, the variant, or the variant of a fragment of the RNA encoded by a polynucleotide having a sequence as set forth in SEQ ID NO: 9 has at least 1, 2, 3, 4, 5, 6, 7, 8, 9, 10 or more nucleotides inserted, added, deleted or substituted compared to the RNA encoded by a polynucleotide having a sequence as set forth in SEQ ID NO: 9.

In some embodiments, the RNA molecule further comprises at least one of a promoter, a 5'- cap structure, a 3'-UTR and a polyA.

In some embodiments, the RNA molecule further comprises at least one of a 5'-cap structure, a 3'-UTR and a polyA.

In some embodiments, the 5'- cap structure includes at least one of m ⁷ GpppG, m ₂ ^{7,3'-O} GpppG, m ⁷ Gppp(5')N1 and m ⁷ Gppp(m ^{2'-O})N1;

In some embodiments, the 3'-UTR comprises one of i) to iii):
i): at least one of a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof; or at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof.
ii) at least two copies of one of the polynucleotides described in i).
iii) at least two polynucleotides in i).

In some embodiments, the species or specific GenBank accession numbers of the source genes of the 3'-UTR can refer to the relevant description regarding the second nucleotide sequence above, which will not be repeated here.

In some embodiments, the 3'-UTR in i) comprises or is one or more of the following: an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10.

In some embodiments, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In some embodiments, the RNA molecule of any of the above embodiments is a recombinant RNA molecule or an artificial RNA molecule.

In some embodiments, all or part of the uridines in the RNA molecule are replaced by at least one nucleoside selected from the group consisting of pseudouridine, N1-methyl-pseudouridine, N1-ethyl pseudouridine, 2-thiouridine, 4'-thiouridine, 5-methylcytosine, 5-methyluridine, 2-thio-1-methyl-1-deaza-pseudouridine, 2-thio T-methyl-pseudouridine, 2-thio-5-aza-uridine, 2-thio-dihydro-pseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine or 5-methoxyuridine and 2'-O-methyluridine; in some embodiments, all or part of the uridine nucleosides in the RNA molecule are replaced by pseudouridine, N1-methylpseudouridine or N1-ethylpseudouridine

In some embodiments, one or more cytosine nucleosides (e.g., 1, 2, 3, 4, 5, 6, 7, 8, 9, or 10 cytosine nucleosides) or at least 50%, at least 60%, at least 70%, at least 80%, at least 90%, or 100% of the cytosine nucleosides in the RNA molecule are replaced by 5-methylcytosine nucleoside.

In some embodiments, the RNA of any of the above embodiments, the RNA molecule of any of the above embodiments, or the artificial RNA molecule of any of the above embodiments comprises one or more modified nucleotides. In some embodiments, the modified nucleotides have base modifications and/or sugar modifications. For example, the modified nucleotides have base modifications.

In some embodiments, the RNA of any of the above embodiments, the RNA molecule of any of the above embodiments, or the artificial RNA molecule of any of the above embodiments comprises one or more modified nucleosides.

In some embodiments, the RNA of any of the above embodiments, the RNA molecule of any of the above embodiments, or the artificial RNA molecule of any of the above embodiments comprises at least one of modified uridine, modified cytidine, modified adenosine, and modified guanosine.

In some embodiments, the modified nucleoside is a modified uridine. In some embodiments, 0.1% to 100% of the uridine in the RNA of any of the above embodiments, in the RNA molecule of any of the above embodiments, or in the artificial RNA molecule of any of the above embodiments is modified. In some embodiments, 80% to 100% of the uridine is modified. In some embodiments, 100% of the uridine is modified. Exemplary modified uridines include, but are not limited to, one or more of the following: pseudouridine (ψ), N1-methyl pseudouridine, pyridin-4-one ribonucleoside, 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho⁵U), 5-aminoallyl-uridine, 5-halo-uridine (e.g., 5-iodo-uridineor 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propynyl-uridine, 1-propynyl-pseudouridine, 5-taurinomethyl-uridine (τcm⁵U), 1-taurinomethyl-pseudouridine, 5-taurinomethyl-2-thio-uridine (τm⁵s²U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s²U), 1-methyl-4-thio-pseudouridine (m¹s⁴ψ)-4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ ψ), 5-(isopentenylaminomethyl)uridine (inm⁵U), 5-(isopentenylaminomethyl)-2-thio-uridine (inm⁵s²U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s²Um), 5-methoxycarbonylmethyl-2'-O-methyl-uridine (mem⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), and 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl)uridine, and 5-[3-(1-E-propenylamino)uridine.

In some embodiments, the modified nucleoside is a modified cytidine. In some embodiments, 0.1% to 100% of the cytidine in the RNA of any of the above embodiments, in the RNA molecule of any of the above embodiments, or in the artificial RNA molecule of any of the above embodiments is modified. In some embodiments, 80% to 100% of the cytidine is modified. In some embodiments, 100% of the cytidine is modified. Exemplary modified cytidines include, but are not limited to, one or more of the following: 5-aza-cytidine, 6-aza-cytidine, pseudoisocytidine, 3-methyl-cytidine (m³C), N4-acetyl-cytidine (ac⁴C), 5-formyl-cytidine (f⁶C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s²C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k₂C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴ ₂Cm), 1-thio-cytidine, 2'-F-ara-cytidine, 2'-F-cytidine, and 2'-OH-ara-cytidine.

In some embodiments, the modified nucleoside is a modified adenosine. In some embodiments, 0.1% to 100% of the adenosine in the RNA of any of the above embodiments, in the RNA molecule of any of the above embodiments, or in the artificial RNA molecule of any of the above embodiments is modified. In some embodiments, 80% to 100% of the adenosine is modified. In some embodiments, 100% of the adenosine is modified. Exemplary modified adenosines include, but are not limited to, one or more of the following: 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m'A), 2-methyl-adenine (m²A), N6-methyl-adenosine (m⁶A), 2-methylthio-N6-methyl-adenosine (ms²m⁶ A), N6-isopentenyl-adenosine (i⁶A), 2-methylthio-N6-isopentenyl-adenosine (ms²io⁶A), N6-(cis-hydroxyisopentenyl)adenosine (io⁶A), 2-methylthio-N6-(cis-hydroxyisopentenyl)adenosine (ms²io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms²hn⁶A), N6-acetyl-adenosine (ac⁶ A), 7-methyl-adenine, 2-methylthio-adenine, 2-methoxy-adenine, α-thio-adenosine, 2'-O-methyl-adenosine (Am), N6,2'-O-dimethyl-adenosine (m⁶Am), N6,N6,2'-O-trimethyl-adenosine (m⁶ ₂Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

In some embodiments, the modified nucleoside is a modified guanosine. In some embodiments, 0.1% to 100% of the guanosine in the RNA of any of the above embodiments, in the RNA molecule of any of the above embodiments, or in the artificial RNA molecule of any of the above embodiments is modified. In some embodiments, 80% to 100% of the guanosine is modified. In some embodiments, 100% of the guanosine is modified. Exemplary modified guanosines include, but are not limited to, one or more of the following: inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m¹G), N2-methyl-guanosine (m²G), N2,N2-dimethyl-guanosine (m²₂G), N2,7-dimethyl-guanosine (m^{2,7}G), N2,N2,7-dimethyl-guanosine (m^{2,2,7}G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m² ₂Gm), 1-methyl-2'-O-methyl-guanosine (m¹Gm), N2,7-dimethyl-2'-O-methyl-guanosine (m^{2,7}Gm), 2'-O-methyl-inosine (Im), 1,2'-O-dimethyl-inosine (m¹Im), 2'-O-ribosylguanosine (phosphate) (Gr(p)), 1-thio-guanosine, O6-methyl-guanosine, 2'-F-ara-guanosine, and 2'-F-guanosine.

In some embodiments, the modified nucleotide comprises isotope-containing nucleotides.

In some embodiments, the RNA of any of the above embodiments, the RNA molecule of any of the above embodiments, or the artificial RNA molecule of any of the above embodiments comprises nucleotides containing isotopes of hydrogen. The isotopes of hydrogen are not limited to deuterium and tritium. In addition, in some embodiments, the RNA further comprises or is a nucleotide containing isotopes of other elements except hydrogen, wherein the other elements include but are not limited to carbon, oxygen, nitrogen and phosphorus.

In some embodiments, the nucleotide sequence encoding the polypeptide of interest of any of the above embodiments or the first nucleotide sequence of any of the above embodiments encodes at least one polypeptide of interest, for example, one, two, three, four, five, six, seven, eight, nine or ten polypeptides of interest. In some embodiments, the nucleotide sequence encoding the polypeptide of interest of any of the above embodiments or the first nucleotide sequence of any of the above embodiments encodes at least one protein of interest, for example, one, two, three, four, five, six, seven, eight, nine or ten proteins of interest. In some embodiments, the nucleotide sequence encoding the polypeptide and protein of any of the above embodiments or the first nucleotide sequence of any of the above embodiments encodes at least one polypeptide of interest and at least one protein of interest, for example, one, two, three, four, five, six, seven, eight, nine or ten polypeptides of interest and one, two, three, four, five, six, seven, eight, nine or ten proteins of interest.

In some embodiments, the protein of interest is the S protein of SARS-COV-2. In some embodiments, the amino acid sequence of the S protein of SARS-COV-2 is as described above. In some embodiments, the sequence of the RNA encoding the S protein of SARS-COV-2 is as described above. It is understood that the protein of interest is not limited to the S protein of SARS-COV-2, but may also be other proteins.

In some embodiments, the RNA of any of the above embodiments, the RNA molecule of any of the above embodiments, or the artificial RNA molecule of any of the above embodiments does not exceed 50000 nt. In some embodiments, the RNA of any of the above embodiments, the RNA molecule of any of the above embodiments, or the artificial RNA molecule of any of the above embodiments does not exceed 40000 nt, 30000 nt, 20000 nt, 10000 nt, 9000 nt, 8000 nt, or 6000 nt. In some embodiments, the RNA of any of the above embodiments, the RNA molecule of any of the above embodiments, or the artificial RNA molecule of any of the above embodiments is 500 nt to 50000 nt. In some embodiments, the RNA of any of the above embodiments, the RNA molecule of any of the above embodiments, or the artificial RNA molecule of any of the above embodiments is 1000 nt to 40000 nt, 1000 nt to 30000 nt, 1500 nt to 10000 nt, or 1500 nt to 8000 nt.

In some embodiments, the present invention further provides a DNA encoding the RNA of any of the above embodiments, a DNA encoding the artificial RNA molecule of any of the above embodiments, or a DNA encoding the RNA molecule of any of the above embodiments.

In some embodiments, the DNA of any of the above embodiments does not exceed 50,000 bp. In some embodiments, the DNA of any of the above embodiments does not exceed 40,000 bp, 30,000 bp, 20,000 bp, 10,000 bp, 9,000 bp, 8,000 bp, or 6,000 bp. In some embodiments, the DNA of any of the above embodiments is 500 bp to 50,000 bp. In some embodiments, the DNA of any of the above embodiments is 1000 bp to 40000bp, 1000 bp ~ 30000bp, 1500 bp ~ 10000bp or 1500 bp ~ 8000bp.

In some embodiments, the present invention also provides a vector comprising the DNA of any one of the above embodiments.

In some embodiments, the present invention also provides another vector comprising a fourth nucleotide sequence encoding a 3'-UTR, wherein the fourth nucleotide sequence comprises at least one of the following polynucleotides:
(a): a polynucleotide encoding a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, and GHRL; in some embodiments, a polynucleotide encoding a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, and GHRL;
(b): a polynucleotide encoding a fragment of the 3'-UTR described in (a);
(c): a polynucleotide encoding a variant of the 3'-UTR described in (a); and
(d): a polynucleotide encoding a variant of the fragment described in (b).

In some embodiments, the gene is a human gene.

In some embodiments, the vector does not contain a polynucleotide encoding a protein and/or polypeptide of interest.

In some embodiments, the fourth nucleotide sequence comprises at least one of the following polynucleotides :
(e): a polynucleotide encoding a 3'-UTR derived from at least one of the genes COP 1 and HDGFL1;
(f): a polynucleotide encoding a fragment of the 3'-UTR described in (e);
(g): a polynucleotide encoding a variant of the 3'-UTR described in (e); and
(h): a polynucleotide encoding a variant of the fragment described in (f).

In some embodiments, the species or specific GenBank accession numbers of the source genes of the 3'-UTR can refer to the relevant description regarding the second nucleotide sequence above, which will not be repeated here.

In some embodiments, the fourth nucleotide sequence comprises at least one of the following polynucleotides (1) to (3):
(1) at least one of a polynucleotide having a sequence as set forth in at least one of SEQ ID NOs: 47 to 67, a fragment of a polynucleotide having a sequence as set forth in at least one of SEQ ID NOs: 47 to 67, a variant of a polynucleotide having a sequence as set forth in at least one of SEQ ID NOs: 47 to 67, and a variant of a fragment of a polynucleotide having a sequence as set forth in at least one of SEQ ID NOs: 47 to 67;
(2) a polynucleotide encoding a 3'-UTR of at least two genes among the genes described in (a), a polynucleotide encoding a fragment of a 3'-UTR of at least two genes among the genes described in (a), a polynucleotide encoding a variant of a 3'-UTR of at least two genes among the genes described in (a), and a polynucleotide encoding a variant of a fragment of a 3'-UTR of at least two genes among the genes described in (a). In some embodiments, the at least two genes are two genes, three genes, four genes, five genes, six genes, seven genes, eight genes, nine genes, or ten genes;
(3) at least one of: at least two copies of the 3'-UTR in (a), at least two copies of a fragment of the 3'-UTR in (a), at least two copies of a variant of the 3'-UTR in (a), and at least two copies of a variant of a fragment of the 3'-UTR in (a). In some embodiments, the at least two copies are two copies, three copies, four copies, five copies, six copies, seven copies, eight copies, nine copies, or ten copies.

In some embodiments, the variant of a polynucleotide having a sequence as set forth in at least one of SEQ ID NOs: 47 to 67, the fragment of a polynucleotide having a sequence as set forth in at least one of SEQ ID NOs: 47 to 67, and the variant of a fragment of a polynucleotide having a sequence as set forth in at least one of SEQ ID NOs: 47 to 67 have at least 70%, 80%, 85%, 90%, 91%, 92%, 93%, 94%, 95%, 96%, 97%, 98% or 99% identity to the polynucleotide having a sequence as set forth in at least one of SEQ ID NOs: 47 to 67.

In some embodiments, the fragment, the variant, or the variant of a fragment of a polynucleotide having a sequence as set forth in at least one of SEQ ID NOs: 47 to 67 has at least 1, 2, 3, 4, 5, 6, 7, 8, 9, 10 or more nucleotides inserted, added, deleted or substituted compared to the polynucleotide having a sequence as set forth in at least one of SEQ ID NOs: 47 to 67.

In some embodiments, the vector further comprises at least one of a promoter, a polynucleotide encoding a 5'-UTR, and a polynucleotide encoding a polyA.

In some embodiments, the 5' -UTR comprises one of i) to iv):
i) at least one of a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D, a fragment, a variant and a variant of a fragment thereof;
ii) at least one of the following polynucleotides: an RNA encoded by a polynucleotide as set forth in SEQ ID NO: 9, a fragment of an RNA encoded by a polynucleotide as set forth in SEQ ID NO: 9, a variant of an RNA encoded by a polynucleotide as set forth in SEQ ID NO: 9, and a variant of a fragment of an RNA encoded by a polynucleotide as set forth in SEQ ID NO: 9;
iii) at least two copies of one of the polynucleotides in i) or ii);
iv) at least two polynucleotides in i).

In some embodiments, the species or specific GenBank accession numbers of the source genes of the 5'-UTR can refer to the relevant description regarding the third nucleotide sequence above, which will not be repeated here.

In some embodiments, the 5'-UTR in i) comprises or is one or more of the following: an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46.

In some embodiments, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and the variant of a fragment of an RNA encoded by a polynucleotide having a sequence as set forth in at least one of SEQ ID NOs: 16 to 46 have at least 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46.

In some embodiments, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In some embodiments, the present invention also provides another vector comprising a fifth nucleotide sequence encoding a 5'- UTR, wherein the fifth nucleotide sequence comprises at least one of the following polynucleotides:
(a): a polynucleotide encoding a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D;
(b): a polynucleotide encoding a fragment of the 5'-UTR described in (a);
(c): a polynucleotide encoding a variant of the 5'-UTR described in (a); and
(d): a polynucleotide encoding a variant of the fragment described in (b) .

In some embodiments, the species or specific GenBank accession numbers of the source genes of the 5'-UTR can refer to the relevant description regarding the third nucleotide sequence above, which will not be repeated here.

In some embodiments, the vector does not contain a polynucleotide encoding a protein and/or polypeptide of interest.

In some embodiments, the fifth nucleotide sequence comprises at least one of the following polynucleotides: a polynucleotide having a sequence as set forth in at least one of SEQ ID NOs: 16 to 46, a fragment of a polynucleotide having a sequence as set forth in at least one of SEQ ID NOs: 16 to 46, a variant of a polynucleotide having a sequence as set forth in at least one of SEQ ID NOs: 16 to 46, and a variant of a fragment of a polynucleotide having a sequence as set forth in at least one of SEQ ID NOs: 16 to 46.

In some embodiments, the variant of a polynucleotide having a sequence as set forth in at least one of SEQ ID NOs: 16 to 46, the fragment of a polynucleotide having a sequence as set forth in at least one of SEQ ID NOs: 16 to 46, and the variant of a fragment of a polynucleotide having a sequence as set forth in at least one of SEQ ID NOs: 16 to 46 have at least 40%, 50%, 60%, 70%, 80%, 85%, 90%, 91%, 92%, 93%, 94%, 95%, 96%, 97%, 98% or 99% identity to the polynucleotide having a sequence as set forth in at least one of SEQ ID NOs: 16 to 46. In some embodiments, the fragment, the variant, or the variant of a fragment of a polynucleotide having a sequence as set forth in at least one of SEQ ID NOs: 16 to 46 has at least 1, 2, 3, 4, 5, 6, 7, 8, 9, 10 or more nucleotides inserted, added, deleted, or substituted compared to a polynucleotide having a sequence as set forth in one of SEQ ID NOs: 16 to 46.

In some embodiments, the fifth nucleotide sequence comprises at least one of: a 5'-UTR of at least two genes among the genes in (a), a fragment of the 5'-UTR of at least two genes among the genes in (a), a variant of the 5'-UTR of at least two genes among the genes in (a), and a variant of a fragment of the 5'-UTR of at least two genes among the genes in (a). In some embodiments, the at least two genes are two genes, three genes, four genes, five genes, six genes, seven genes, eight genes, nine genes, or ten genes.

In some embodiments, the fifth nucleotide sequence comprises at least one of: at least two copies of the 5'-UTR in (a), at least two copies of a fragment of the 5'-UTR in (a), at least two copies of a variant of the 5'-UTR in (a), and at least two copies of a variant of a fragment of the 5'-UTR in (a). In some embodiments, the at least two copies are two copies, three copies, four copies, five copies, six copies, seven copies, eight copies, nine copies, or ten copies.

In some embodiments, the vector further comprises at least one of a promoter, a polynucleotide encoding 3'-UTR, and a polynucleotide encoding a polyA.

In some embodiments, the 3'-UTR comprises one of i) to iii):
i): at least one of a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof; or at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof.
ii) at least two copies of one of the polynucleotides described in i).
iii) at least two polynucleotides in i).

In some embodiments, the species or specific GenBank accession numbers of the source genes of the 3'-UTR can refer to the relevant description regarding the second nucleotide sequence above, which will not be repeated here.

In some embodiments, the 3'-UTR in i) comprises or is one or more of the following: an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10.

In some embodiments, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In some embodiments, the present invention also provides another vector, wherein the vector comprises a polynucleotide sequence encoding a 5'-UTR; wherein the polynucleotide sequence encoding the 5'-UTR comprises at least one of the following polynucleotides: a polynucleotide having a sequence as set forth in SEQ ID NO: 9, a fragment of a polynucleotide having a sequence as set forth in SEQ ID NO: 9, a variant of a polynucleotide having a sequence as set forth in SEQ ID NO: 9, and a variant of a fragment of a polynucleotide having a sequence as set forth in SEQ ID NO: 9.

In some embodiments, the variant of a polynucleotide having a sequence as set forth in SEQ ID NO: 9, the fragment of a polynucleotide having a sequence as set forth in SEQ ID NO: 9, and the variant of a fragment of a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the polynucleotide having a sequence as set forth in SEQ ID NO: 9. In some embodiments, the fragment, the variant, or the variant of a fragment of a polynucleotide having a sequence as set forth in SEQ ID NO: 9 has at least 1, 2, 3, 4, 5, 6, 7, 8, 9, 10 or more nucleotides inserted, added, deleted or substituted compared to the polynucleotide having a sequence as set forth in SEQ ID NO: 9.

In some embodiments, the vector further comprises at least one of a promoter, a polynucleotide encoding 3'-UTR, and a polynucleotide encoding a polyA.

In some embodiments, the 3'-UTR comprises one of i) to iii):
i): at least one of a 3'-UTR derived from at least one of the genes APOA1, UL122, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof; or at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, a fragment, a variant and a variant of a fragment thereof;
ii) at least two copies of one of the polynucleotides described in i);
iii) at least two polynucleotides in i).

In some embodiments, the species or specific GenBank accession numbers of the source genes of the 3'-UTR can refer to the relevant description regarding the second nucleotide sequence above, which will not be repeated here.

In some embodiments, the 3'-UTR in i) comprises or is one or more of the following: an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10.

In some embodiments, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10.

In some embodiments, the nucleotides constituting the polyA include at least 20, at least 40, at least 80, at least 100 or at least 120 A nucleotides; in some embodiments, the nucleotides constituting the polyA include consecutively at least 20, at least 40, at least 80, at least 100 or at least 120 A nucleotides.

In some embodiments, the nucleotides constituting the polyA include one or more nucleotides other than the A nucleotide.

In some embodiments, the polyA is a truncated polyA, in some embodiments, a 10 bp non-A linker sequence is added after a plurality of consecutive nucleotides A and then a plurality of consecutive nucleotides A is added. In some embodiments, the polyA is 30 consecutive nucleotides A, then a 10 bp non-A linker sequence and then 70 consecutive nucleotides A.

In some embodiments, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11. In some embodiments, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11.

In some embodiments, the polyA is an RNA encoded by a polynucleotide having a sequence as set forth in one of SEQ ID NOs: 68 to 70, 72, 75 and 11. In some embodiments, the polyA is an RNA encoded by a polynucleotide having a sequence as set forth in SEQ ID NO: 11.

In any of the above embodiments, the polypeptide or protein of interest refers to a therapeutically or pharmaceutically active polypeptide or protein having a therapeutic or preventive effect, the function of which in or near a cell is necessary or beneficial, for example, such a protein, the deficiency or defective form of which causes a disease, and providing such a protein can regulate or prevent the disease, or such a protein, which is beneficial to the body in or near a cell. The polypeptide or protein may comprise a complete protein or a functional variant thereof.

In any of the above embodiments, the peptide and/or protein expressed by the nucleotide sequence or the first nucleotide sequence encoding the polypeptide and/or protein of interest comprises or is one or more of the following: (a) an antigen; (b) a therapeutic protein or polypeptide, a fragment, a variant and a variant of a fragment thereof; and (c) other polypeptides or proteins.

In some embodiments, the peptide and/or protein expressed by the nucleotide sequence encoding the polypeptide and/or protein of interest comprises or is an antigen.

In some embodiments, the antigen expressed by the nucleotide sequence encoding the polypeptide and/or protein of interest is derived from one or more of the following: (1) pathogenic antigens, fragments, variants, or variants of fragments thereof, (2) tumor antigens, fragments, variants, or variants of fragments thereof, (3) allergic antigens, fragments, variants, or variants of fragments thereof, and (4) autoimmune self-antigens, fragments, variants, or variants of fragments thereof.

In some embodiments, pathogenic antigens are derived from pathogenic organisms which are capable of eliciting an immune response in a subject, e.g., a mammalian subject, e.g., a human. In some embodiments, pathogenic organisms include or are one or more of the following: bacteria, viruses, fungi, and protozoa, e.g., unicellular organisms, multicellular organisms.

In some embodiments, the pathogenic antigen comprises or is a surface antigen, a fragment, a variant, or a variant of a fragment thereof, such as a protein located on the surface of a virus, bacteria, or protozoa, a fragment (e.g., an exterior portion of a surface antigen), a variant, or a variant of a fragment therof.

In some embodiments, the pathogenic antigen comprises or is derived from a polypeptide or protein from a pathogen associated with an infectious disease.

In some embodiments, the pathogenic antigen is selected from, but not limited to, the antigens derived from pathogens recorded on pages 21 to 35 of WO2018/078053A1, the antigens derived from pathogens recorded on page 57, paragraph 3 to page 63, paragraph 2 of WO2019/077001A1, the antigens derived from pathogens recorded on page 32, line 26 to page 34, line 27 of WO2013/120628A1, and the antigens recorded on page 34, line 29 to page 59, line 5 of WO2013/120628A1.

In some embodiments, the pathogen of the pathogenic antigen is selected from but not limited to one or more of the following: scabies, Babesia, Leishmania, Gnatostoma, Ancylostoma braziliensis, Ancylostoma duodenale, Strongyloides stercoralis, Trichuris trichuris, Toxocara canis, Toxocara cati, Toxoplasma, Trypanosoma brucei, Trypanosoma cruzi, Brugia malayi, Onchocerca volvulus, Wuchereria bancrofti, Taenia (Tapeworm), Taenia solium, Echinococcus, Ascaris lumbricoides, Dinucleate Amoeba fragilis, Naegleria fowleri, Necator americanus, Paragonimus (e.g., Paragonimus westermani), Clonorchis sinensis, Plasmodium (e.g., P. falciparum, P. vivax, P. malariae, or P. ovale), Pneumocystis jirovecii, Metagonimus yokogawa, Trichinella spiralis, Trichomonas vaginalis, Giardia intestinalis, Schistosoma japonicum, Geotrichum candidum, Saccharomyces hominis, Bartonella henselae, Hortaea Wernicke, Aspergillus, Malassezia, Vibrio cholerae, Acinetobacter baumannii, Paracoccidioides brasiliensis, Neisseria gonorrhoeae, Neisseria meningitidis, Pasteurella, Nocardia asteroides, Nocardia, Sporothrix schenckii, Staphylococcus, Streptococcus agalactiae, Streptococcus pneumoniae, Streptococcus pyogenes, Trichophyton, Yersinia enterocolitica, Yersinia pestis, Yersinia pseudotuberculosis, Ureaplasma urealyticum, Salmonella, Francisella tularensis, Fusobacterium, Mycobacterium leprae, Mycobacterium leprae disseminated, Mycobacterium tuberculosis, Mycobacterium ulcerans, Shigella, Cryptococcus hemolyticus Bacillus, Bacillus anthracis, Bacillus cereus, Histoplasma capsulatum, Blastomyces dermatitidis, Bordetella pertussis, Borrelia burgdorferi, Borrelia, Brucella, Burkholderia (e.g., Burkholderia cepacia, Burkholderia glanders, Burkholderia pseudomallei), Campylobacter, Candida (e.g., Candida albicans), Chlamydia pneumoniae, Corynebacterium diphtheriae, Coxiella burnetii, Clostridium (e.g., Clostridium botulinum, Clostridium difficile, Clostridium perfringens, Clostridium perfringens), Clostridium tetani, Coccidioides, Ehrlichia chaffeensis, Ehrlichia ewingii, Ehrlichia, extra-intestinal pathogenic Escherichia coli, Kingella, Klebsiella granulomatosa, Anaplasmas (e.g., Anaplasma phagocytophilum), Leptospira, Borrelia burgdorferi, Treponema pallidum, Rickettsiae (e.g., Rickettsia prowazekii, Rickettsia rickettsii, Rickettsia typhi), Chlamydia psittaci, Chlamydia trachomatis, kuru prions, Lassa virus (LASV), Legionella pneumophila, Listeria monocytogenes, Enterococci, dermatophytes, Escherichia coli O157:H7 and O104:H4, Fasciola hepatica and Fasciola gigas, enteroviruses (e.g., Coxsackie virus A, enterovirus 71 (EV71)), FFI prion, CJD prion, Epstein-Barr virus (EBV), feline immunodeficiency virus (FIV), Flavivirus, GSS prion, Guanarito virus, Haemophilus ducreyi, Haemophilus influenzae, Helicobacter pylori, Bunyaviridae, Caliciviridae, Astroviridae, Coronavirus, Congo hemorrhagic fever virus, Cryptococcus neoformans, Cryptosporidium spp., Cytomegalovirus (CMV), BK virus, dengue virus, Ebola virus (EBOV), herpes simplex virus (HSV), human immunodeficiency virus (HIV), Human papillomavirus (HPV), influenza virus, rabies virus, norovirus, Nipah virus, Henipavirus (Hendra virus-Nipah virus), hepatitis A virus, hepatitis B virus (HBV), hepatitis C virus (HCV), hepatitis D virus, hepatitis E virus, human bocavirus (HBoV), human metapneumovirus (hMPV), human parainfluenza virus (HPIV), Japanese encephalitis virus, JC virus, Junin virus, yellow fever virus, MERS coronavirus, lymphocytic choriomeningitis virus (LCMV), Machupo virus, Marburg virus, measles virus, human molluscum contagiosum virus (MCV), mumps virus, parvovirus B19, Mycoplasma pneumoniae orthomyxovirus, poliovirus, rhinovirus, Rift Valley fever virus, rotavirus, rubella virus, Sabia virus, SARS coronavirus (e.g. SARS-CoV-2), nCoV-2019 coronavirus, Sin Nombre virus, hantavirus, vaccinia virus, respiratory syncytial virus (RSV), tick-borne encephalitis virus (TBEV), varicella-zoster virus (VZV), Venezuelan equine encephalitis virus, West Nile virus, western equine encephalitis virus, and Zika virus.

In some embodiments, the pathogenic antigen includes or is one or more of the following:
(1) one or more of the following proteins of SARS coronavirus 2 (SARS-CoV-2), nCoV-2019 coronavirus or SARS coronavirus (SARS-CoV)): spike protein (S), envelope protein (E), membrane protein (M) or nucleocapsid protein (N);
(2) one or more of the following proteins of MERS coronavirus: spike protein (S), spike S1 fragment (S1), envelope protein (E), membrane protein (M) or nucleocapsid protein (N);
(3) one or more of the following proteins of human papillomavirus (such as HPV16): replication protein E1, regulatory protein E2, protein E3, protein E4, protein E5, protein E6, protein E7, protein E8, major capsid protein L1 and minor capsid protein L2;
(4) one or more of the following proteins of human parainfluenza virus (HPIV/PIV) (such as hPIV-1, hPIV-2, hPIV-3 or hPIV-4 serotypes): fusion protein (F), hemagglutinin neuraminidase, glycoprotein (G), matrix protein (M), phosphoprotein (P), nucleocapsid protein, fusion glycoprotein F0, F1 or F2, recombinant PIV3/PIV1 fusion glycoprotein, C protein, D protein, viral replicase (L) and non-structural V protein;
(5) one or more of the following proteins of human metapneumovirus (hMPV): fusion (F) glycoprotein, glycoprotein (G), phosphoprotein (P), and nucleocapsid protein,
(6) one or more of the following proteins of influenza virus: hemagglutinin (HA), neuraminidase (NA), nucleoprotein (NP), M1 protein, M2 protein, NS1 protein, NS2 protein (NEP protein: nuclear export protein), PA protein, PB1 protein (polymerase basic 1 protein), PB1-F2 protein and PB2 protein;
(7) one or more of the following proteins of rabies virus: nucleoprotein (N), large structural protein (L), phosphoprotein (P), matrix protein (M) and glycoprotein (G);
(8) one or more of the following proteins of human immunodeficiency virus: HIV p24 antigen, HIV envelope protein (Gp120, Gp41, Gp160), polyprotein GAG, negative factor protein Nef, transcription transactivator Tat and Brec1;
(9) one or more of the following proteins of Chlamydia trachomatis: major outer membrane protein MOMP, probable outer membrane protein PMPC, outer membrane complex protein B OmcB, heat shock protein Hsp60 HSP10, protein IncA, type III secretion system protein, ribonucleotide reductase small chain protein NrdB, plasmid protein Pgp3, Chlamydial outer protein N CopN, antigen CT521, antigen CT425, antigen CT043, antigen TC0052, antigen TC0189, antigen TC0582, antigen TC0660, antigen TC0726, antigen TC0816, antigen TC0828;
(10) one or more of the following proteins of cytomegalovirus (CMV/HCMV): pp65 antigen, membrane protein pp15, capsid-proximal tegument protein pp150, protein M45, DNA polymerase UL54, helicase UL105, glycoprotein gM, glycoprotein gN, glycoprotein H, glycoprotein B gB, protein UL83, protein UL94, protein UL99, HCMV glycoprotein (selected from gH-gL, gB, gO, gN and gM), HCMV protein (selected from UL83, UL123, UL128, UL130 and UL131A), epidermal protein pp150 (pp150), tegument protein pp65/lower matrix phosphoprotein(pp65), envelope glycoprotein M (UL100), regulatory protein IE1 (UL123), envelope protein (UL128), envelope glycoprotein (130), envelope protein (UL131A), envelope glycoprotein B (UL55), structural glycoprotein N gpUL73 (UL73), structural glycoprotein O gpUL74 (UL74);
(11) one or more of the following proteins of dengue virus: capsid protein C, pre-membrane protein prM, membrane protein M, envelope protein E (domain I, domain II, domain II), protein NS1, protein NS2A, protein NS2B, protein NS3, protein NS4A, protein 2K, protein NS4B, protein NS5;
(12) one or more of the following proteins of EBOV virus: EBOV glycoprotein (GP), surface EBOV GP, wild-type EBOV pro GP, mature EBOV GP, secreted wild-type EBOV pro GP, secreted mature EBOV GP, EBOV nucleoprotein (NP), RNA polymerase L and EBOV matrix protein (selected from VP35, VP40, VP24 and VP30);
(13) one or more of the following proteins of hepatitis B virus (HBV): hepatitis B surface antigen HBsAg, hepatitis B core antigen HbcAg, polymerase, protein Hbx, pre-S2 middle surface protein, surface protein L, large S protein, viral protein VP1, viral protein VP2, viral protein VP3, and viral protein VP4;
(14) one or more of the following proteins of respiratory syncytial virus (RSV): fusion protein F, F protein, nucleoprotein N, matrix protein M, matrix protein M2-1, matrix protein M2-2, phosphoprotein P, small hydrophobic protein SH, major surface glycoprotein G, polymerase L, nonstructural protein 1NS1, nonstructural protein 2NS2, RSV attachment protein (G) (glycoprotein G), fusion (F) glycoprotein (glycoprotein F), nucleoprotein (N), phosphoprotein (P), large polymerase protein (L), matrix protein (M, M2), small hydrophobic protein (SH), nonstructural protein 1 (NS1), nonstructural protein 2 (NS2), membrane-bound RSV F protein, membrane-bound DS Cavl (stabilized prefusion RSV F protein);
(15) one or more of the following proteins of Mycobacterium tuberculosis: secretory antigen SssA (Staphylococcus, staphylococcal food poisoning); secretory antigen SssA (Staphylococcus, such as Staphylococcus aureus, staphylococcal infection); molecular chaperone DnaK, cell surface lipoprotein Mpt83, lipoprotein P23, phosphate transport system permeation protein pstA, 14kDa antigen, fibronectin binding protein C FbpC1, alanine dehydrogenase TB43, glutamine synthetase 1, ESX-1 protein, protein CFP10, TB10.4 protein, protein MPT83, protein MTB12, protein MTB8, Rpf-like protein, protein MTB32, protein MTB39, crystal protein, heat shock protein HSP65, and protein PST-S;
(16) one or more of the following proteins of yellow fever virus: genomic polyprotein, protein E, protein M, capsid protein C, protease NS3, protein NS1, protein NS2A, protein AS2B, protein NS4A, protein NS4B, protein NS5;
(17) cyclosporin; and
(18) one or more of the following proteins of Zika virus: Zika virus capsid protein (c), Zika virus pre-membrane protein (prM), Zika virus pr protein (pr), Zika virus membrane protein (M), Zika virus envelope protein (E), Zika virus non-structural protein, Zika virus prME antigen, Zika virus capsid protein, pre-membrane/membrane protein, ZIKV envelope protein, ZIKV non-structural protein 1, ZIKV non-structural protein 2A, ZIKV non-structural protein 2B, ZIKV non-structural protein 3, ZIKV non-structural protein 4A, ZIKV non-structural protein 4B, ZIKV non-structural protein 5, and Zika virus envelope protein (e).

In some embodiments, the tumor antigen is selected from but not limited to the group consisting of the tumor antigens described in WO2018/078053A1, pages 47-51.

In some embodiments, the antigens expressed by the nucleotide sequences encoding the polypeptides and/or proteins of interest include or are allergic antigens and autoimmune self-antigens. In some embodiments, allergic antigens and autoimmune self-antigens are derived from or selected from the group of antigens described in, but not limited to, WO2018/078053A1, pages 59 to 73.

In some embodiments, the antigens expressed by the nucleotide sequences encoding the polypeptides and/or proteins of interest are listed on pages 48 to 51 of WO2018/078053 A1.

In some embodiments, the polypeptide and/or protein expressed by the nucleotide sequence encoding the polypeptide and/or protein of interest comprises or is a therapeutic protein or polypeptide.

In some embodiments, the therapeutic protein or polypeptide includes or is one or more of the following:
(1) enzyme replacement therapy for the treatment of metabolic, endocrine or amino acid disorders, or therapeutic proteins or polypeptides for replacing missing, defective or mutant proteins; (2) therapeutic proteins or polypeptides for the treatment of blood disorders, diseases of the circulatory system, diseases of the respiratory system, infectious diseases or immunedeficiencies; (3) therapeutic proteins or polypeptides for the treatment of cancer or tumor diseases; (4) therapeutic proteins or polypeptides for hormone replacement therapy; (5) therapeutic proteins or polypeptides for reprogramming somatic cells into pluripotent stem cells or omnipotent stem cells; (6) therapeutic proteins or polypeptides for use as adjuvants or immunostimulants; (7) therapeutic proteins or polypeptides as therapeutic antibodies; (8) therapeutic proteins or polypeptides as gene editing agents; (9) therapeutic proteins or polypeptides for the treatment or prevention of liver diseases selected from the group consisting of liver fibrosis, cirrhosis and liver cancer; and (10) therapeutic proteins or polypeptides for the treatment or prevention of rare diseases.

In some embodiments, the enzyme replacement therapy for treating metabolic, endocrine or amino acid disorders or the therapeutic protein or polypeptide for replacing a missing, deleted or mutant protein comprises or is one or more of the following: Acid sphingomyelinase, Adipotide, Agalsidase-beta, Alglucosidase, alpha-galactosidase A, alpha- glucosidase, alpha-L-iduronidase, alpha-N-acetylglucosaminidase, Amphiregulin, angiopoietin (Ang1, Ang2, Ang3, Ang4, ANGPTL2, ANGPTL3, ANGPTL 4, ANGPTL5, ANGPTL6, ANGPTL7), ATPase, Cu(2+)-transporting β polypeptide (ATP7B), argininosuccinate synthetase (ASS1), Betacellulin, β-glucuronidase, bone morphogenetic protein BMP (BMP1, BMP2, BMP3, BMP4, BMP5, BMP6, BMP7, BMP8a, BMP8b, BMP10, BMP15), CLN6 protein, epidermal growth factor (EGF), Epigen, Epiregulin, fibroblast growth factors (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23), fumarylacetoacetate hydrolase (FAH), Galsulphase, growth hormone releasing peptide, glucocerebrosidase, GM-CSF, heparin-binding EGF-like growth factor (HB-EGF), hepatocyte growth factor HGF, hepatopoietin, human albumin, increased loss of albumin, Idursulphase (lduronate-2 -sulphatase) (iduronate-2-sulfatase), integrins αVβ3, αVβ5 and α5β1, uduronate sulfatase, laronidase, N-acetylgalactosamine-4-sulfatase (rhASB; galsulfase, arylsulfatase A (ARSA), arylsulfatase B (ARSB)), N-acetylglucosamine glucose-6-sulfatase, nerve growth factor (NGF, brain-derived neurotrophic factor (BDNF), neurotrophin-3 (NT-3) and neurotrophin 4/5 (NT-4/5), neuregulin (NRG1, NRG2, NRG3, NRG4), neuropilin (NRP-1, NRP-2), obestatin, phenylalanine hydroxylase (PAH), phenylalanine ammonia lyase (PAL), platelet-derived growth factor (PDGF (PDFF-A, PDGF-B, PDGF-C, PDGF- D)), TGFβ receptors (endothelin, TGFβ1 receptor, TGFβ2 receptor, TGFβ3 receptor), thrombopoietin (THPO) (megakaryocyte growth and development factor (MGDF)), transforming growth factor (TGF (TGF-a, TGF-β (TGFβ1, TGFβ2 and TGFβ3))), VEGF (VEGF-A, VEGF-B, VEGF-C, VEGF-D, VEGF-E, VEGF-F and PIGF), nesiritide, trypsin, adrenocorticotropic hormone (A CTH), atrial natriuretic peptide (ANP), cholecystokinin, gastrin, leptin, oxytocin, somatostatin, vasopressin (antidiuretic hormone), calcitonin, exenatide, growth hormone (GH), somatotropin, insulin, insulin-like growth factor 1 IGF-1, Mecasermin rinfabate, IGF-1 analog, Pegvisomant, Pramlintide, Teriparatide (human parathyroid hormone residues 1 -34), Becaplermin, Dibotermin-alpha (Bone morphogenetic protein 2), Histrelin acetate (gonadotropin releasing hormone; GnRH), Octreotide, hepatocyte nuclear factor 4 alpha (HNF4A), CCAAT/enhancer binding protein alpha (CEBPA), fibroblast growth factor 21 (FGF21), extracellular matrix protease or human collagenase MMP1, hepatocyte growth factor (HGF), TNF-related apoptosis-inducing ligand (TRAIL), opioid growth factor receptor-like 1 (OGFRL1), clostridial type II collagenase, relaxin 1 (RLN1), relaxin 2 (RLN2), relaxin 3 (RLN3), and palifermin (keratinocyte growth factor; KGF).

In some embodiments, the therapeutic protein or polypeptide for use in the treatment of metabolic or endocrine diseases is selected from the proteins or polypeptides described in Table A (in combination with Table C) of WO2017/191274.

In some embodiments, the therapeutic protein or polypeptide for use in the treatment of blood disorders, diseases of the circulatory system, diseases of the respiratory system, cancer or tumor diseases, infectious diseases or immunedeficiencies comprises or is one or more of the following: Alteplase (tissue plasminogen activator; tPA), Anistreplase, Antithrombin III (AT-III), Bivalirudin, Darbepoetin-alpha, Drotrecogin-alpha (activated protein C, Erythropoietin, Epoetin-alpha, erythropoetin, erthropoyetin, Factor IX, Factor Vila, Factor VIII, Lepirudin, Protein C concentrate, Reteplase (deletion mutein of tPA), Streptokinase, Tenecteplase, Urokinase, Angiostatin, Anti-CD22 immunotoxin, Denileukin diftitox, Immunocyanin, MPS (Metallopanstimulin), Aflibercept, Endostatin, Collagenase, Human deoxyribonuclease I, dornase, Hyaluronidase, Papain, L-Asparaginase, Peg-asparaginase, Rasburicase, Human chorionic gonadotropin (HCG), Human follicle-stimulating hormone (FSH), Lutropin-alpha, Prolactin, alpha-1 -Proteinase inhibitor, Lactase, Pancreatic enzymes (lipase, amylase, protease), Adenosine deaminase (pegademase bovine, PEC-ADA), Abatacept, Alefacept, Anakinra, Etanercept, Interleukin-1 (IL-1 ) receptor antagonist, Anakinra, Thymulin, TNF-alpha antagonist, Enfuvirtide, and Thymosin α1.

In some embodiments, the therapeutic protein or polypeptide for use in the treatment of cancer or tumor diseases comprises or is one or more of the following: cytokines, chemokines, suicide gene products, immunogenic proteins or peptides, apoptosis inducers, angiogenesis inhibitors, heat shock proteins, tumor antigens, β-catenin inhibitors, STING pathway activators, checkpoint regulators, innate immune activators, antibodies, dominant negative receptors and decoy receptors, myeloid-derived suppressor cells (MDSCs) inhibitors, IDO pathway inhibitors, and proteins or peptides that bind to apoptosis inhibitors;

In some embodiments, the hormone among the therapeutic protein or polypeptide for hormone replacement therapy includes one or more of the following: estrogen, progesterone, progestin, and testosterone.

In some embodiments, therapeutic proteins for reprogramming somatic cells into pluripotent or omnipotent stem cells include one or more of the following: Oct-3/4, Sox gene family (e.g., Sox1, Sox2, Sox3, and Sox15), Klf family (e.g., Klf1, Klf2, Klf4, and Klf5), Myc family (e.g., c-Myc, L-Myc, and N-Myc), Nanog, and LIN28.

In some embodiments, the therapeutic protein or polypeptide for use as an adjuvant or immunostimulatory protein includes or is one or more of the following: human adjuvant proteins, in particular pattern recognition receptors TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP6, NALP7, NALP7, NALP8, NALP9, NALP10, NALP11, NALP12, NALP13, NALP14J IPAF, NAIP, CIITA, RIG-I, MDA5 and LGP2, the signal transducers of TLR signaling including adaptor proteins including e.g. Trif and Cardif; components of the Small-GTPases signalling (RhoA, Ras, Rac1 , Cdc42, Rab etc.), components of the PIP signalling (PI3K, Src-Kinases, etc.), components of the MyD88-dependent signalling (MyD88, IRAKI, IRAK2, IRAK4, TIRAP, TRAF6 etc.), components of the MyD88-independent signalling (TIC AMI , TICAM2, TRAF6, TBK1 , IRF3, TAK1 , IRAKI etc.); the activated kinases (e.g. Akt, MEKK1 , MKK1 , MKK3, MKK4, MKK6, MKK7, ERK1 , ERK2, GSK3, PKC kinases, PKD kinases, GSK3 kinases, JNK, p38MAPK, TAK1 , IKK, and TAKl); the activated transcription factors (e.g. NF-κB, C-FOS, c-Jun, c-Myc, CREB, AP-1 , Elk-1 , ATF2, IRF-3, IRF-7), heat shock proteins, such as HSP10, HSP60, HSP65, HSP70, HSP75 and HSP90, gp96, Fibrinogen, Typlll repeat extra domain A of fibronectin; or components of the complement system (e.g. Clq, MBL, Cl r, Cl s, C2b, Bb, D, MASP-1 , MASP-2, C4b, C3b, C5a, C3a, C4a, C5b, C6, C7, C8, C9, CR1 , CR2, CR3, CR4, C1 qR, C1 INH, C4bp, MCP, DAF, H, I, P and CD59), or cell surface proteins that induce target genes (e.g. Beta-Defensin). In some embodiments, human adjuvant proteins include one or more of the following: trif, flt-3 ligand, Gp96 or fibronectin, cytokines which induce or enhance an innate immune response (e.g., IL-1α, IL-1R1, IL1β, IL-2, IL-6, IL-7, IL-8, IL-9, IL-12, IL-13, IL-15, IL-16, IL-17, IL-18, IL-21, IL-23, TNFα, IFNα, IFNβ, IFNγ, GM-CSF, G-CSF, M-CSF), chemokines (e.g., IL-8, IP-10, MCP-1, MIP-1α, RANTES, Eotaxin, CCL21), cytokines which are released from macrophages (e.g., IL-1, IL-6, IL-8, IL-12, TNF-α, etc.).

In some embodiments, therapeutic proteins or polypeptides for use as adjuvants or immunostimulators include one or more of the following: bacterial (adjuvant) proteins, protozoan (adjuvant) proteins, viral (adjuvant) proteins, fungal (adjuvant) proteins, and animal-derived proteins.

In some embodiments, the bacterial (adjuvant) protein includes one or more of the following: bacterial heat shock proteins or chaperons, including Hsp60, Hsp70, Hsp90, Hsp100; OmpA (Outer membrane protein) from gram-negative bacteria; bacterial porins, including OmpF; bacterial toxins such as pertussis toxin (PT) from Bordetella pertussis, pertussis adenylate cyclase toxin CyaA and CyaC from Bordetella pertussis, PT-9K/129G mutant from pertussis toxin, pertussis adenylate cyclase toxin CyaA and CyaC from Bordetella pertussis, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB) Escherichia coli heat-labile enterotoxin mutants with reduced toxicity such as LTK63, LTR72; phenol-soluble modulin; neutrophil-activating protein (HP-NAP) from Helicobacter pylori; Surfactant protein D; Outer surface protein A lipoprotein from Borrelia burgdorferi, Ag38 (38 kDa antigen) from Mycobacterium tuberculosis; proteins from bacterial fimbriae such as Pilin from pili from gram negative bacteria, and Surfactant protein A and bacterial flagellins.

In some embodiments, the protozoan (adjuvant) proteins include one or more of the following: Tc52 from Trypanosoma cruzi, PFTG from Trypanosoma gondii, Protozoan heat shock proteins, LeIF from Leishmania, and profiling-like protein from Toxoplasma gondii.

In some embodiments, the viral (adjuvant) proteins include one or more of the following: Respiratory Syncytial Virus fusion glycoprotein (F-protein), envelope protein from MMT virus, mouse leukemia virus protein, and Hemagglutinin protein of wild-type measles virus.

In some embodiments, the fungal (adjuvant) protein comprises a fungal immunomodulatory protein (FIP, such as LZ-8).

In some embodiments, the animal-derived protein comprises keyhole limpet hemocyanin (KLH).

In some embodiments, the polypeptide and/or protein expressed by the nucleotide sequence encoding the polypeptide and/or protein of interest comprises or is a therapeutic protein or polypeptide as a therapeutic antibody, such as one or more of the cytokines, chemokines, suicide enzymes and gene products, apoptosis inducers, endogenous angiogenesis inhibitors, heat shock proteins, tumor antigens, innate immune activators, and antibodies against proteins associated with tumor or cancer development described in Table 1, Table 2, Table 3, Table 4, Table 5, Table 6, Table 7, Table 8, Table 9, Table 10, Table 11 and Table 12 of WO2016/170176 A1.

In some embodiments, the present invention provides a host cell comprising the vector of any of the above embodiments, the RNA of any of the above embodiments, the artificial RNA molecule of any of the above embodiments, or the RNA molecule of any of the above embodiments.

In some embodiments, the present invention also provides a lipid nanoparticle comprising the RNA of any of the above embodiments, the artificial RNA molecule of any of the above embodiments, the RNA molecule of any of the above embodiments, the protein of any of the above embodiments, the DNA of any of the above embodiments, or the vector of any of the above embodiments.

In some embodiments, the lipid nanoparticle further comprises one or more of an ionizable cationic lipid, a helper lipid, a structural lipid, and a PEG-lipid.

In some embodiments, the lipid nanoparticle comprises ionizable cationic lipids.

In some embodiments, the lipid nanoparticle further comprises one or more of helper lipids, structural lipids and PEG-lipids (polyethylene glycol-lipids). In an alternative specific example, the lipid nanoparticle comprises ionizable cationic lipids, helper lipids, structural lipids and PEG-lipids.

In some embodiments, the ionizable cationic lipid is selected from one or more of Dlin-MC3-DMA, Dlin-KC2-DMA, DODMA, c12-200 and DlinDMA.

In some embodiments, the helper lipid is a phospholipid. The phospholipid is usually semi-synthetic, or it may be of natural origin or chemically modified. The phospholipid includes, but is not limited to, DSPC (distearylphosphatidylcholine), DOPE (dioleoylphosphatidylethanolamine), DOPC (dioleoylphosphatidylcholine), DOPS (dioleoylphosphatidylserine), DSPG (1,2-dioctadecanoyl-sn-glycerol-3-phospho-(1'-rac-glycerol)), DPPG (dipalmitoylphosphatidylglycerol), DPPC (dipalmitoylphosphatidylcholine), DGTS (1,2-dipalmitoyl-sn-glycerol-3-O-4'-(N,N,N-trimethyl)homoserine), lysophospholipid, etc. In some embodiments, the helper lipid is selected from one or more of DSPC, DOPE, DOPC, and DOPS. In some embodiments, the helper lipid is DSPC and/or DOPE.

In some embodiments, the structural lipid is a sterol substance, including but not limited to cholesterol, cholesterol ester, sterol hormone, sterol vitamin, bile acid, cholesterol, ergosterol, β-sitosterol and oxidized cholesterol derivatives. In some embodiments, the structural lipid is selected from at least one of cholesterol, cholesterol ester, sterol hormone, sterol vitamin and bile acid. In some embodiments, the structural lipid is cholesterol, preferably high-purity cholesterol, particularly injection-grade high-purity cholesterol. For example CHO-HP (produced by AVT).

As used herein, the term PEG-lipid (polyethylene glycol-lipid) is a conjugate of polyethylene glycol and a lipid structure. In some embodiments, the PEG-lipid is selected from PEG-DMG and PEG-distearoylphosphatidylethanolamine (PEG-DSPE). The PEG-DMG is a polyethylene glycol (PEG) derivative of 1,2-dimyristyl glyceride.

In some embodiments, the PEG-lipid is selected from PEG-DMG or PEG-DSPE.

In some embodiments, the average molecular weight of PEG is about 2000 Daltons to 5000 Daltons. More preferably, the average molecular weight of PEG is about 2000 Daltons or 5000 Daltons.

In some embodiments, the PEG-lipid is PEG 2000-DMG.

In some embodiments, the present invention also provides a pharmaceutical composition, comprising an artificial RNA molecule of any of the above embodiments, an RNA molecule of any of the above embodiments, a DNA of any of the above embodiments, a vector of any of the above embodiments, a host cell of any of the above embodiments, or a lipid nanoparticle of any of the above embodiments, and a pharmaceutically acceptable carrier.

In some embodiments, the present invention also provides use of an artificial RNA molecule of any of the above embodiments, an RNA molecule of any of the above embodiments, a DNA of any of the above embodiments, a vector of any of the above embodiments, a host cell of any of the above embodiments, or a lipid nanoparticle of any of the above embodiments, or a pharmaceutical composition of any of the above embodiments in the preparation of a medicament.

In some embodiments, the medicament is for use in gene therapy, genetic vaccination, protein replacement therapy, antisense therapy, or treatment by interfering RNA.

Gene vaccines are vaccines used for genetic vaccination and are generally understood as "third generation" vaccines. They usually consist of genetically engineered nucleic acid molecules that allow the expression of peptide or protein (antigen) fragments specific to pathogens or tumor antigens *in vivo.* Gene vaccines are expressed after being administered to the patient and taken up by the target cells. The expression of the administered nucleic acids leads to the production of the encoded proteins. When the immune system of the patient recognizes these proteins as foreign, an immune response is triggered.

In some embodiments, the present invention also provides use of an artificial RNA molecule of any of the above embodiments, an RNA molecule of any of the above embodiments, a DNA of any of the above embodiments, a vector of any of the above embodiments, a host cell of any of the above embodiments, or a lipid nanoparticle of any of the above embodiments, or a pharmaceutical composition of any of the above embodiments in the preparation of a medicament for nucleic acid transfer.

In some embodiments, the nucleic acid is RNA, messenger RNA (mRNA), antisense oligonucleotide, DNA, plasmid, ribosomal RNA (rRNA), micro RNA (miRNA), transfer RNA (tRNA), small interfering RNA (siRNA), and small nuclear RNA (snRNA).

In some embodiments, the medicament is for use in gene therapy, genetic vaccination, or protein replacement therapy.

In some embodiments, the medicament is for use in the treatment and/or prevention of a disease. In some embodiments, the medicament is a vaccine; in some embodiments, the medicament is a vaccine for preventing SARS-COV-2 infection.

In some embodiments, the disease is selected from the group consisting of rare diseases, infectious diseases, cancer, genetic diseases, autoimmune diseases, diabetes, neurodegenerative diseases, cardiovascular diseases, renal vascular diseases, and metabolic diseases.

In some embodiments, the cancer comprises one or more of lung cancer, stomach cancer, liver cancer, esophageal cancer, colon cancer, pancreatic cancer, brain cancer, lymphoma, blood cancer or prostate cancer. The genetic disease comprises one or more of hemophilia, thalassemia, and Gaucher's disease. The rare disease comprises one or more of the following: osteogenesis imperfecta, Wilson's disease, spinal muscular atrophy, Huntington's disease, Rett syndrome, amyotrophic lateral sclerosis, Duchenne muscular dystrophy, Friedrich's ataxia, methylmalonic acidemia, cystic fibrosis, glycogen storage disease 1a, glycogen storage disease III, Crigler-Najjar syndrome, ornithine carbamoyltransferase deficiency, propionic acidemia, phenylketonuria, hemophilia A, hemophilia B, β-thalassemia, Lafora disease, Dravet syndrome, Alexander disease, Leber congenital amaurosis, myelodysplastic syndrome, and CBS-deficient homocystinuria.

In some embodiments, the medicament is a nucleic acid drug, wherein the nucleic acid comprises at least one of RNA, messenger RNA (mRNA), DNA, plasmid, ribosomal RNA (rRNA), single guide RNA (sgRNA), and cas9 mRNA.

As used herein, the term "transfer" refers to the delivery of a nucleic acid, such as a therapeutic and/or prophylactic agent, to a target. For example, delivering a therapeutic and/or prophylactic agent to a subject may involve administering a lipid nanoparticle comprising the therapeutic and/or prophylactic agent to the subject (e.g., via intravenous, intramuscular, intradermal or subcutaneous routes, etc.).

In one embodiment, the polypeptide or protein of interest refers to a therapeutically or pharmaceutically active polypeptide or protein having a therapeutic or preventive effect, the function of which in or near a cell is necessary or beneficial, for example, such a protein, the deficiency or defective form of which causes a disease, and providing such a protein can regulate or prevent the disease, or such a protein, which is beneficial to the body in or near a cell. The polypeptide or protein may comprise a complete protein or a functional variant thereof. In some embodiments, the polypeptide or protein of interest is described above.

In some embodiments, the present invention also provides a method for treating and/or preventing a disease, comprising administering to a subject in need thereof an artificial RNA molecule of any of the above embodiments, an RNA molecule of any of the above embodiments, a DNA of any of the above embodiments, a vector of any of the above embodiments, a host cell of any of the above embodiments, or a lipid nanoparticle of any of the above embodiments, or a pharmaceutical composition of any of the above embodiments.

In some embodiments, the disease or disorder is described above in the context of the use of the artificial RNA molecule, RNA molecule, DNA, vector, host cell or lipid nanoparticle or pharmaceutical composition in the preparation of a medicament for nucleic acid transfer.

In some embodiments, the artificial RNA molecule, RNA molecule, DNA, vector, host cell or lipid nanoparticle or pharmaceutical composition is for use as a vaccine for preventing a disease. In some embodiments, the artificial RNA molecule, RNA molecule, DNA, vector, host cell or lipid nanoparticle or pharmaceutical composition is used to produce an antigen of pathogen or a portion thereof.

In some embodiments, the artificial RNA molecule, RNA molecule, DNA, vector, host cell or lipid nanoparticle or pharmaceutical composition is used to produce the genetic disease-associated protein.

In some embodiments, the artificial RNA molecule, RNA molecule, DNA, vector, host cell or lipid nanoparticle or pharmaceutical composition is used to produce antibodies, such as scFVs or nanobodies.

**In a third aspect,** the present invention provides a method for preparing the above-mentioned RNA, comprising the following steps:
(1) extraction of the engineered plasmid to ensure that the supercoil rate of the plasmid is above 90%;
(2) plasmid linearization and purification;
*(3) in vitro* transcription and purification; and
(4) Capping and purification.

**In a fourth aspect,** the present invention provides a method for preparing plasmid H, comprising:
(1) Construction of plasmid D: on the basis of the Luciferase-pcDNA3 plasmid as the backbone, new restriction sites HindIII and BamHI are added before the Kozack sequence, and new restriction sites KpnI and ApaI are added after the stop codon of luciferase to obtain plasmid B; 5'-UTR is inserted between the restriction sites HindIII and BamHI of plasmid B to obtain plasmid C; 3'-UTR is inserted between the restriction sites KpnI and ApaI of plasmid C to obtain plasmid D;
(2) Construction of plasmid G: on the basis of plasmid B as the backbone, the ampicillin resistance gene sequence is replaced with the kanamycin sulfate resistance gene sequence, and the neo/KanR sequence from nucleotide position 3746 to 4540 (corresponding to the position numbering of plasmid B) is removed to obtain plasmid F; a polyA sequence is inserted into plasmid F to obtain plasmid G;
(3) Construction of plasmid vector H: Plasmid G and plasmid D are double-digested with HindIII and ApaI, respectively. a fragment with a larger molecular weight is selected among the products resulting from double-digestion of plasmid G while a fragment with a smaller molecular weight is selected among the products resulting from double-digestion of plasmid D. The two fragments are ligated with T4 enzyme to obtain plasmid H. The plasmids shown in the nucleotide sequences of SEQ ID NO: 2 (plasmid B), SEQ ID NO: 3 (plasmid C), SEQ ID NO: 4 (plasmid D), SEQ ID NO: 5 (plasmid F) and SEQ ID NO: 6 (plasmid G) used in the preparation of plasmid H are also part of the present invention.

Plasmid H is double-digested with BamHI and KpnI to obtain a fragment with a larger molecular weight, and the fragment is homologously recombined with the S protein coding sequence of SARS-COV-2 of the present invention to obtain an engineered plasmid for an mRNA vaccine. Therefore, the present invention also provides an engineered plasmid for preparing an mRNA vaccine, which comprises a nucleotide sequence that can transcribe mRNA, specifically comprising a template nucleotide sequence of the mRNA vaccine of the present invention. The template nucleotide sequence refers to a DNA sequence in an engineered plasmid, which serves as a template for *in vitro* synthesis of mRNA to guide the synthesis of mRNA molecules; more specifically, the template nucleotide sequence of the mRNA vaccine of the present invention is the S protein coding sequence of SARS-COV-2 of the present invention. In one embodiment, the engineered plasmid is set forth in SEQ ID NO: 8.

Based on the above, the present invention has designed S protein of SARS-COV-2, coding DNA and counterpart mRNA. The serum obtained by immunizing the mouse with the vaccine prepared by the mRNA can simultaneously neutralize SARS-COV-2 pseudovirus, Delta pseudovirus and Omicron pseudovirus, indicating that the mRNA vaccine prepared by the mRNA can obtain cross-protection reactions against different strains. The sequence is expressed and utilized to prepare novel mRNA vaccines against the SARS-COV-2 Delta and Omicron variants, which show superior immune effects on the SARS-COV-2 Delta and Omicron variants and can be developed and mass-produced more rapidly.

Meanwhile, the present invention also has constructed a polynucleotide molecule for preparing a novel mRNA vaccine, which improves the protein expression level of mRNA through scientific design of individual regions of 5'-UTR, 3'-UTR and polyA. This polynucleotide molecule can not only be used to prepare mRNA vaccines that protect against wild-type SARS-COV-2, Delta and Omicron variants, but can also be used to transfect cells or bacteria to produce corresponding antibodies or antigen proteins. It is a universal core element combination, which is of great significance to promoting the development of the biopharmaceutical industry.

### Examples

### Example 1 Construction and preparation of the S protein coding sequence of SARS-COV-2

The mutation sites of the SARS-COV-2 were analyzed and the coding nucleic acid sequence of the S protein of SARS-COV-2 as set forth in SEQ ID NO: 12 was designed. The amino acid sequence encoded by the nucleic acid sequence is set forth in SEQ ID NO: 13, and the mRNA sequence is set forth in SEQ ID NO: 14, in which all uridine nucleosides are replaced by N1-methyl pseudouridine. The nucleic acid sequence (SEQ ID NO: 12) was synthesized by Nanjing GenScript Biotechnology Co., Ltd. and released by GenScript QC.

The characterization data were: GenScript QC release results showed that the sequence was correct.

### Example 2 Construction of Plasmid H

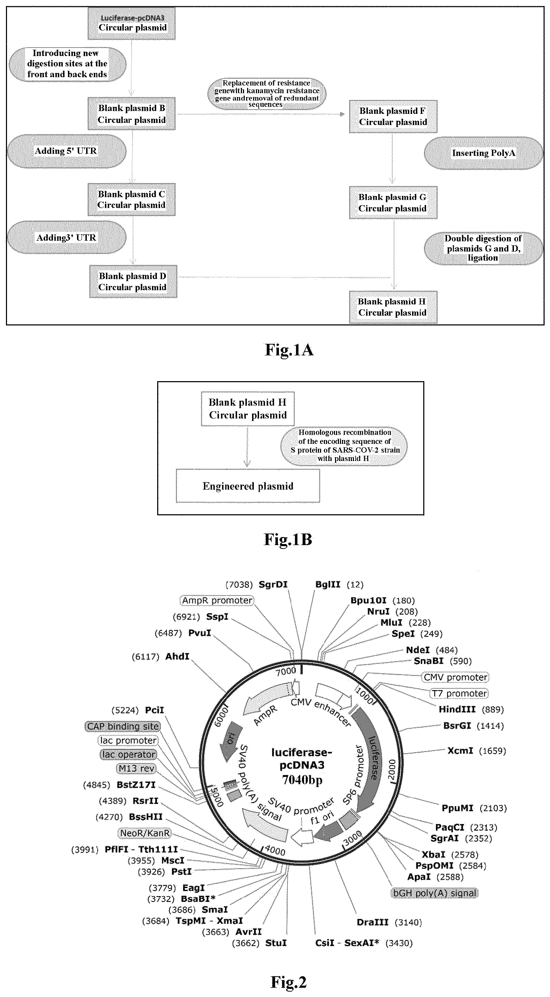

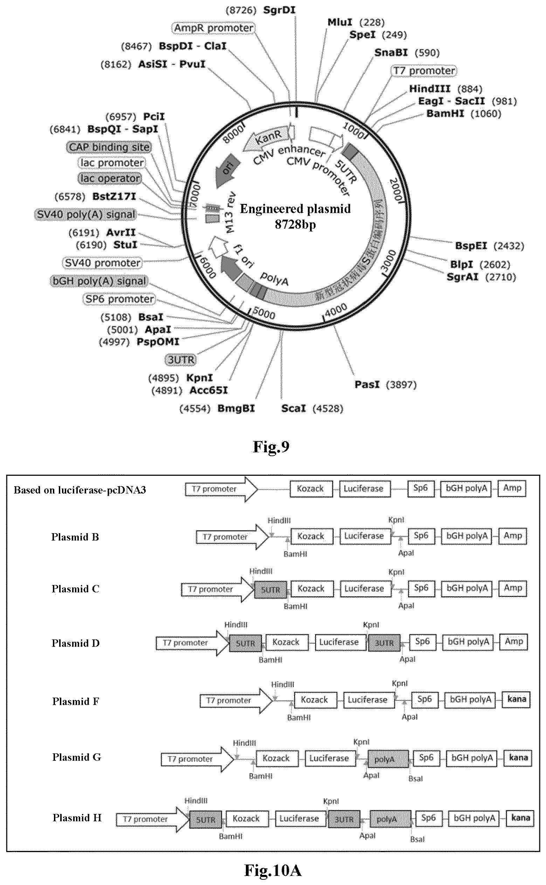

The flowchart for constructing plasmid H is shown in Figure 1A, and a schematic diagram of the construction and modification thereof is shown in Figure 10A.

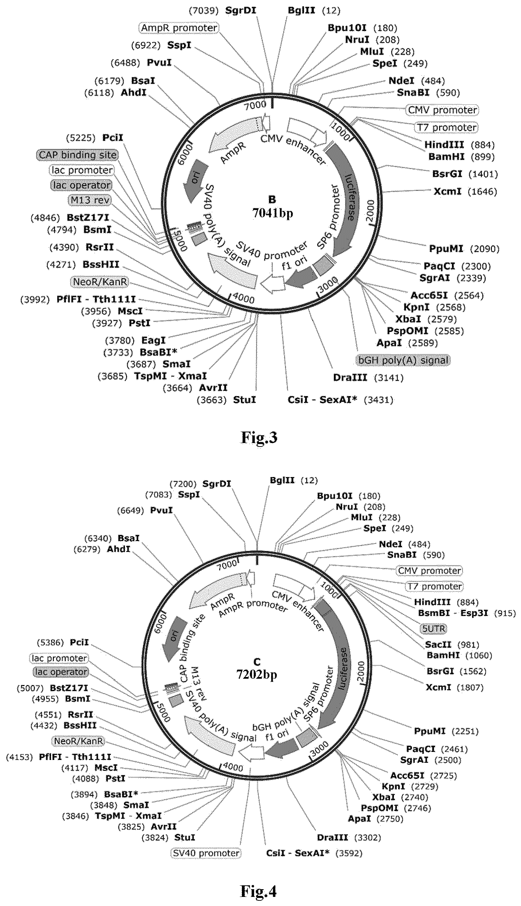

The specific construction method comprises:
Construction of plasmid B:
   On the basis of Luciferase-pcDNA3 plasmid (purchased from Addgene, its plasmid number #18964) as the backbone, new restriction sites HindIII and BamHI were added before the Kozack sequence, and new restriction sites KpnI and ApaI were added after the stop codon of luciferase to obtain plasmid B. The nucleotide sequence of the luciferase-pcDNA3 plasmid is set forth in SEQ ID NO: 1. The map of luciferase-pcDNA3 is shown in Figure 2. The map of plasmid B is shown in Figure 3. The nucleotide sequence of plasmid B is set forth in SEQ ID NO: 2.
Construction of plasmid C:
   The 5'-UTR sequence as set forth in SEQ ID NO: 9 was inserted between the restriction sites HindIII and BamHI of plasmid B to obtain plasmid C.

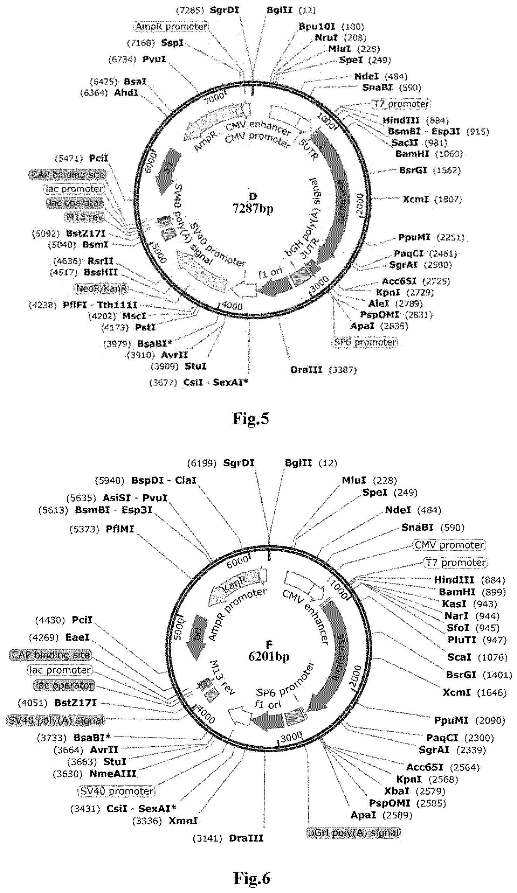

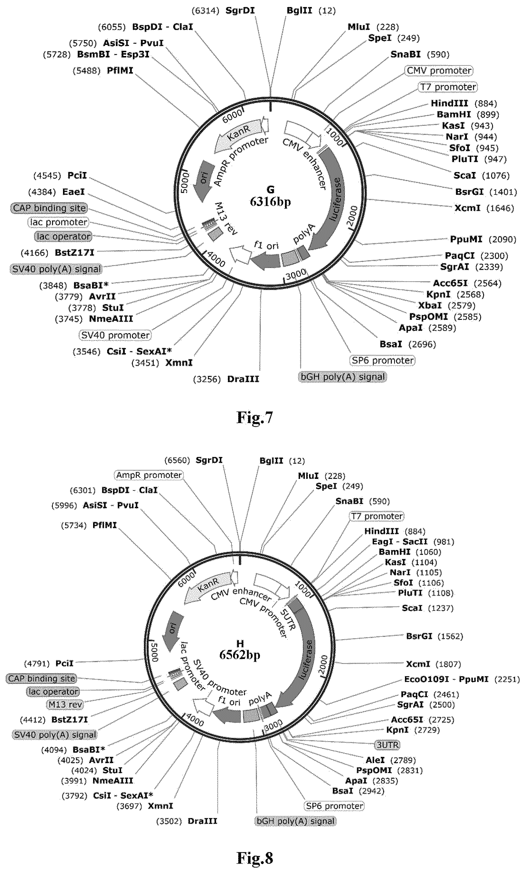

Specifically, plasmid B was double-digested with HindIII and BamHI, and the fragment with a molecular weight of about 7 kb as a vector fragment was obtained through gel excision, recovery and purification. The 5'-UTR sequence as set forth in SEQ ID NO: 9 was introduced into the homology arm sequence as an insert fragment by PCR. The vector fragment and the insert fragment were homologously recombined to generate plasmid C, and the map of plasmid C is shown in Figure 4. The nucleotide sequence of plasmid C is set forth in SEQ ID NO: 3.
Construction of plasmid D:
   The 3'-UTR sequence as set forth in SEQ ID NO: 10 was inserted between the restriction sites KpnI and ApaI of plasmid C to obtain plasmid D. The map of plasmid D is shown in Figure 5. The nucleotide sequence of plasmid D is set forth in SEQ ID NO: 4.
Construction of plasmid F:
   The Amp (ampicillin) resistance gene in plasmid B was replaced with the kana (kanamycin) resistance gene, and the neo/KanR sequence from 3746 to 4540 was removed to obtain plasmid F. The map of plasmid F is shown in Figure 6. The nucleotide sequence of plasmid F is set forth in SEQ ID NO: 5.
Construction of plasmid G:
   Plasmid F was inserted with a polyA sequence as set forth in SEQ ID NO: 11, single-digested with ApaI, and the resulting product was purified. The purified single-digested product and the polyA sequence as set forth in SEQ ID NO: 11 were constructed by homologous recombination. The product was transformed into DH5α, and clone screening was performed to select the plasmid with correct sequencing. The map of plasmid G is shown in Figure 7. The nucleotide sequence of plasmid G is set forth in SEQ ID NO: 6.
Construction of plasmid H:
   Plasmid D was double-digested with HindIII and ApaI, and the fragment with a molecular weight of about 1.6 kb was recovered from the gel (axygen) as fragment A. Plasmid G was double-digested with HindIII and ApaI, and the fragment with a molecular weight of about 4.5 kb was recovered from the gel (axygen) as fragment B. Fragment A and fragment B were ligated with quick ligase T4 (NEB) at 25°C for 5 minutes. The above reaction system was transformed into DH5α (takara) competent cells, which were plated with 50 µg/mL kanamycin. After culturing for 16 hours, 3 to 4 single clones were picked and cultured in a medium containing 50 µg/mL kanamycin for 8 hours, and the Mini Extraction plasmid was sent to Shanghai Sangon Biotech for sequencing. The map of plasmid H is shown in Figure 8. The nucleotide sequence of plasmid H is set forth in SEQ ID NO: 7.

### Example 3 Synthesis of 5'-UTR

The following 5'-UTR sequences were designed.

**Table 1: 5'-UTR sequence listing**

| 5'-UTR designation | Nucleotide sequence of 5'-UTR |
|---|---|
| 5UTR-NO1 Apolipoprotein A1 (APOA1) | AGAGACTGCGAGAAGGAGGTCCCCCACGGCCCTTCAGG (SEQ ID NO: 16) |
| 5UTR-NO3 caspase recruitment domain family member 16 (CARD16) | ACACAAGAAGGGTAGGAGAGAAAAGCC (SEQ ID NO: 17) |
| 5UTR-NO6 albumin (ALB) | AGTATATTAGTGCTAATTTCCCTCCGTTTGTCCTAGCTTTTCTCTTCTGTC AACCCCACACGCCTTTGGCACA (SEQ ID NO: 18) |
| 5UTR-NO8 apolipoprotein C1 (APOC1) | CTCCCCAGCCTGATAAAGGTCCTGCGGGCAGGACAGGACCTCCCAACC AAGCCCTCCAGCAAGGATTCAGAGTGCCCCTCCGGCCTCGCC (SEQ ID NO: 19) |
| 5UTR-NO9 Eukaryotic Translation Elongation Factor 1 Alpha 1 (EEF1A1) | CTTTTTCGCAACGGGTTTGCCGCCAGAACACAGGTGTCGTGAAAACTA CCCCTAAAAGCCAAA (SEQ ID NO: 20) |
| 5UTR-NO15 retinol binding protein 4 (RBP4) | CGCCTCCCTCGCTCCACGCGCGCCCGGACTCGGCGGCCAGGCTTGCGC GCGGTTCCCCTCCCGGTGGGCGGATTCCTGGGCAAG (SEQ ID NO: 21) |
| 5UTR-NO19 ghrelin and obestatin prepropeptide (GHRL) | AGTTCCCCAAAGATAACACAGCTTTGCACAGTGG (SEQ ID NO: 22) |
| 5UTR-NO21 MPN domain-containing protein (MPND) | CGGGGCGGGGCCGCGTGACGTGCCGGGAAGCCGGAGTCTAGAGCTCC GGGCGCGGGGAGGCGCGGCC (SEQ ID NO: 23) |
| 5UTR-NO24 N-acylsphingosine amidohydrolase 2B (ASAH2B) | GTGGAAGCGGACCTGAGGAGTCGCAATTGTGAACAGGTTCGGTGCTGC GGCTGGGGCTCAGCGAT (SEQ ID NO: 24) |
| 5UTR-NO27 F-box protein 16 (FBX016) | GGGAGTCGGAGAAAAAGCTGACCTAATGAAACTGTGGCAACGTCAGC GCTTGAGGCTTGAAGAGGGAGACAAGCTAAAAGAGGATATCCAGTTGT TTC (SEQ ID NO: 25) |
| 5UTR-NO28 F-box DNA helicase 1 (FBH1) | CAGAGGAGGAGCTCGCTGCCGGGGGACGCTGGGCTGAGCGGCCGGCG GCGCGGCCCCGGCGGCGGCGGCAGCGGGGTCCGGGTCCGGAGCGCCA C (SEQ ID NO: 26) |
| 5UTR-NO30 spermidine synthase (SRM) | GCGGGCGGCGGGCGGAGCTGGTCCCGTTGTGCTGCGGCCCCGCGCGG CCTGCAGTCCCGGGCTCGCGCCCCGCGCCGCCCGCCCGCCCGCC (SEQ ID NO: 27) |
| 5UTR-NO31 N-acylethanolamine acid amidase (NAAA) | ACGGCTTACCCGGGCTCCGACAGCGGCTGCAGGAGCCCAAGTTGCGGC CGCTGGAGCCCGAGCC (SEQ ID NO: 28) |
| 5UTR-NO32 actin beta (ACTB) | ACCGCCGAGACAGCGTCCGCCCCGCGAGCACAGAGCCTCGCCTTTGCC GATCCGCCGCCCGTCCACACCCGCCGCCAGCTCACC (SEQ ID NO: 29) |
| 5UTR-NO33 MAPK interacting serine/threonine kinase 2 (MKNK2) | CGGCTCCTCTCAGCGGCGGTGGCCCAGGTAGAGGGGTCCGCGCTGGCG GCGGCGGCGGCGCTGTTCCCCGCGCGGTCCGCGGAGCGGGGTCCGGG CTGCGCGACGTGGGGCGGCGGCGGCACTGCGGCCCCGGCCCAAGCCC GACCCCGGGTCCCCTCCTCGGCCGCCCCCCGCCCGGCCGCCCGCCCTC GGGCCTCCCCCCGAACCCTCGGTCCCCTCCCCCGCTGGCGGGGAACGG ACAGAAG (SEQ ID NO: 30) |
| 5UTR-NO35 orosomucoid 1 (ORM1) | ACAGAGTAAACTTTTGCTGGGCTCCAAGTGACCGCCCATAGTTTATTAT AAAGGTGACTGCACCCTGCAGCCACCAGCACTGCCTGGCTCCACGTGC CTCCTGGTCTCAGTG (SEQ ID NO: 31) |
| 5UTR-NO36 glyceraldehyde-3-phosphate dehydrogenase (GADPH) | AAATTGAGCCCGCAGCCTCCCGCTTCGCTCTCTGCTCCTCCTGTTCGAC AGTCAGCCGCATCTTCTTTTGCGTCGCCAGCCGAGCCACATCGCTCAGA CACC (SEQ ID NO: 32) |
| 5UTR-NO38 ubiquinone oxidoreductase complex assembly factor 2 (NDUFAF2) | GTTACTAGAAGGGCTCCAGGATGGAGGCCGACCTGACTTCTCCGCCTC GGTGGGCTGGGTCGGCGGCTGGAGCATTACCCCTACTGCGGGTCCCGC TGCTGGCAGCGCTGGAAACTGGGTGGACGGC (SEQ ID NO: 33) |
| 5UTR-NO39 fibroblast growth factor receptor 2 (FGFR2) | TGACTGCAGCAGCAGCGGCAGCGCCTCGGTTCCTGAGCCCACCGCAGG CTGAAGGCATTGCGCGTAGTCCATGCCCGTAGAGGAAGTGTGCAGATG GGATTAACGTCCACATGGAGATATGGAAGAGGACCGGGGATTGGAACC GTAACC (SEQ ID NO: 34) |
| 5UTR-NO40 MYCBP associated protein (MYCBPAP) | TGGTGACGTCACGGCGCTCGTCGGCGCCGCCTGTGGCGTCACAGGCCG GGAACGCAGGGCTTTCTAGGGGTCCGTCGCTCTTGAAGCCGCCGGCGG CGGGCGCGTGCGCGGCCCGATGAAGAAGGAGGTTTCCAAGCCGTCTCC GCCCAAGTTGATCGGTGG (SEQ ID NO: 35) |
| 5UTR-NO41 charged multivesicular body protein 2A (CHMP2A) | GGTGAGTGGCCCCTGCTGGACCGTGAATCCCGGGCGGGCGTGCGGGCG GCAGCGTACGAGGCCTTCTACTCGCCCGGGTGTCCGGTGCCTCGCTTCT CCCTGGGACCCTGTCGTCAACATCGAGGCCTGCCAGTGAGCTTCTACC ATC (SEQ ID NO: 36) |
| 5UTR-NO43 tumor susceptibility 101 (TSG101) | GTAGTGGTGCCGACTTCCTGTTGTTTGAGGCCGGGTTGGGGGTGTGCG ATTGTGTGGGACGGTCTGGGGCAGCCCAGCAGCGGCTGACCCTCTGCC TGCGGGGAAGGGAGTCGCCAGGCGGCCGTC (SEQ ID NO: 37) |
| 5UTR-NO44 pre-mRNA processing factor 8 (PRPF8) | GAACTCGGGTTGCGCACTTCCCGGCGCTGGGAACGCGGAGCGGACGC AGTCTGGCCGCCATTGCGCTGCGGGGAAAGCGGCCTCTTGTGTGAGGG CCTGTGGGATTCTCCGGAT (SEQ ID NO: 38) |
| 5UTR-NO45 nuclear factor kappa B subunit 2 (NFKB2) | GGTATTTTCGGGACTTTCCTAAGCTGCTCTAACTTTCCTGCCCCTTCCCC GGCCAAGCCCAACTCCGGATCTCGCTCTCCACCGGATCTCACCCGCCA CACCCGGACAGGCGGCTGGAGGAGGCGGGCGTCTAAAATTCTGGGAA GCAGAACCTGGCCGGAGCCACTAGACAGAGCCGGGCCTAGCCCAGAG AC (SEQ ID NO: 39) |
| 5UTR-NO46 NEDD8 activating enzyme E1 subunit 1 (NAE1) | AGTGCGCCTGCGCGCTTGTGGAGCTGGTGGCGGCGCTCCGCAGGGGCT CGGCTGTTTTCCGCGCGGCAGGCGCGGCCATGGCGCAGCTGGGAAAGC TGCTCAAGGAGCAGAAGTACGACCGGCAGCTGAGGCGATTCCTCCATA CATTTGACAGCTGTCTGAACCCTTATGGAGCATACAGTGGAGTGGAAA G (SEQ ID NO: 40) |
| 5UTR-NO47 HDGF like 1 (HDGFL1) | TTAGGTGGCGCCGGTCCCCTCGAACAGGTCGCTCAACAACTCCCGCCC AGCAGCCGCCCTTACTGCGCGCGCGCAGACTTCGGCGTCTACTTCCGG TGTGGCCCAGGCGGGGTCCGCAGAACCAGCT (SEQ ID NO: 41) |
| 5UTR-NO48 gasdermin D (GSDMD) | CCTGGGCAAGCCCTGCGTCAGGTTGCAGTTTCACTTTTAGCTCTGGGCA CCTCCAGCTCCTGCTCGCCGGACGGCTCCCAGGGAGAGCAGACGCGCC AGACGCGCCACCCTCGGGGCGCCGACGGTCACGGAGC (SEQ ID NO: 42) |
| 5UTR-NO49 F-box and WD repeat domain containing 12 (FBXW12) | TTCTTTCTCCTCCACTGCAAAGTTAAATGCGAGAAGGTAGAAACCCAG AGGCCATGCTGGCGCTGAGAGATGAGCCCCACTCACCAGATTCAAGAT CCCAAGTGCTGCAGACTTTGGCAAAGCCTATAAATTCAGAGCAGCCCG GAGAGGAGAAAGGAAAGTGGATGTGGGTTCAGGCCGCATGAA (SEQ ID NO: 43) |
| 5UTR-NO50 F-box and WD repeat domain containing 10 (FBXW10) | ACGGTAAAGCCATGTTTCCAAGGAGGTCTGTACAAAAAGCCAGACTTC TGCTGGCAGTTACTGAGAGAGATAGGCTTTCCATCCATGGCAGCCATTT ACTTTTGCTCTGGGAGACGTTTGTAATAGAAAAGGCACAACTGGGGTAT TTATTCATTCCCCCCGTTCCTCTAGTGTTTGGTGGCGTTGCCGTTGCAAG TGCGCAGGGCTAAAATGGACTGGTTATCTTAGGATC (SEQ ID NO: 44) |
| 5UTR-NO51 F-box and leucine rich repeat protein 8 (FBXL8) | GCTTCCCGTCCGCTGTCCTCTGCTGCCAGTCCCCTGCCCCGGGCAAAGC CCATCTGGTCCGCCGAGCAGGCCGGAGCTATTGGGAGTGGCGcATCCTC CCACCCCAGCCGGATCTGGGCC (SEQ ID NO: 45) |
| 5UTR-NO52 autophagy related 4D cysteine peptidase (ATG4D) | CTGGGGACGGGGGCCGAGTAGCGCCTTCCCCGGGCCCCGTGAACCGGC TGCGGGTCGCCCTTGGGGGGCAGCGGCCGCAGCCCCCCACCTGGGCCC TCGGTCCGCCCTCCCGGCGCGTCC (SEQ ID NO: 46) |
| 5UTR-NO54 | TAATACGACTCACTATAGGACAGATCGCCTGGAGACGCCATCCACGCTG TTTTGACCTCCATAGAAGACACCGGGACCGATCCAGCCTCCGCGGCCG GGAACGGTGCATTGGAACGCGGATTCCCCGTGCCAAGAGTGACGTAAG TACCGCAGTCACCGTCCTTGACACG (SEQ ID NO: 9) |

The above 5'-UTR sequences were synthesized by Sangon Biotechnology (Shanghai) Co., Ltd. According to the quality analysis report of Sangon, the 5'-UTR sequences were consistent with the theoretically designed sequences.

### Example 4 Synthesis of 3'-UTR

The following 3'-UTR sequences were designed.

**Table 2: 3'-UTR sequence listing**

| 3'-UTR designation and source gene | Nucleotide sequence of 3'-UTR |
|---|---|
| 3UTR-NO1 apolipoprotein A1 (APOA1) | GGCGCCCGCCGCCGCCCCCCTTCCCGGTGCTCAGAATAAACGTTTCC AAAGTGGG (SEQ ID NO: 47) |
| 3UTR-NO2 UL122 | ACTATTGTATATATATCAGTTACTGTTATGGACCCCACGTCACTATTGTA TACTCTATATTATACTCTATGTTATACTCTGTAATCCTACTCA (SEQ ID NO: 48) |
| 3UTR-NO3 CARD only protein (COP1) | TCTTCACCCAGTCTGTCTCTTAGAATTTATTTCCCACTGACACAAACA AGCCATTTTGTCTGCTTGTATAACCC (SEQ ID NO: 49) |
| 3UTR-NO7 Myb like, SWIRM and MPN domains 1 (MYSM1) | TTATTTTAAAGTTAAGACATTTTAATCTTGACACAGTAGATCTTACTTT CAAAGTTATAAACTTGAAGTGATTGTAGTT (SEQ ID NO: 50) |
| 3UTR-NO9 N-acylsphingosine amidohydrolase 2B (ASAH2B) | TGAAAAGTTGATAGATCATTTAAAGAACAGCTTTACTCTCTACACATT ATATAAGTGATTTCAAATG (SEQ ID NO: 51) |
| 3UTR-NO12 NADH:ubiquinone oxidoreductase complex assembly factor 2 (NDUFAF2) | ATGCATTATGGTCAAATCTTTTCATGTATATGGATGTGACTATTTTAACA AATAAAAGAAGTGAAAAGTTATTTACCTTGT (SEQ ID NO: 52) |
| 3UTR-NO18 apolipoprotein D (APOD) | CCAGGTTCTACAGGGAGGCTGCACCCACTCCATGTTACTTCTGCTTCG CTTTCCCCTACCCCCCCCCCATAAAGACAAACCAATCAACCACGACA AAGGAAGTTGACCTGAACATGTAACCATGCCCTACCCTGTTACCTTGC TAGCTGCAAAATAAACTTGTTGCTGACCTGCTGTGCTCGCAGTAGATT CCAAGTT (SEQ ID NO: 53) |
| 3UTR-NO21 hemoglobin subunit beta (HBB) | GCTCGCTTTCTTGCTGTCCAATTTCTATTAAAGGTTCCTTTGTTCCCTA AGTCCAACTACTAAACTGGGGGATATTATGAAGGGCCTTGAGCATCTG GATTCTGCCTAATAAAAAACATTTATTTTCATTGC (SEQ ID NO: 54) |
| 3UTR-NO26 transferrin (TF) | AATCTCAGAGGTAGGGCTGCCACCAAGGTGAAGATGGGAACGCAGAT GATCCATGAGTTTGCCCTGGTTTCACTGGCCCAAGTGGTTTGTGCTAA CCACGTCTGTCTTCACAGCTCTGTGTTGCCATGTGTGCTGAACAAAA AATAAAAATTATTATTGATTTTATATTTCAAAAACTCCATTCTTTCCTAA ATATTTTCAACAAAGGATTTCTTTATGCATTCTGCCTAAATACCTATGC AACTGAGCCCTTCCTTCTCAGCTCAAGATTCGTCTGGTCTTTCCCTAC AGCTTTGTGTGTGCCATGGCCACATCTCCTGGGTACAGTTCAAGGAG ACATCTTTTCTAAAAGGGTCTGCGTGATCATTAAAATATAATCAAATGT A (SEQ ID NO: 55) |
| 3UTR-NO27 thymosin beta 4 X-linked (TMSB4X) | TGAGGCGTGCGCCGCCAATATGCACTGTACATTCCACAAGCATTGCCT TCTTATTTTACTTCTTTTAGCTGTTTAACTTTGTAAGATGCAAAGAGGT TGGATCAAGTTTAAATGACTGTGCTGCCCCTTTCACATCAAAGAACTA CTGACAACGAAGGCCGCGCCTGCCTTTCCCATCTGTCTATCTATCTGG CTGGCAGGGAAGGAAAGAACTTGCATGTTGGTGAAGGAAGAAGTGG GGTGGAAGAAGTGGGGTGGGACGACAGTGAAATCTAGAGTAAAACC AAGCTGGCCCAAGGTGTCCTGCAGGCTGTAATGCAGTTTAATCAGAG TGCCATTTTTTTTTTTGTTCAAATGATTTTAATTATTGGAATGCACAATT TTTTTAATATGCAAATAAAAAGTTTAAAAACTTA (SEQ ID NO: 56) |
| 3UTR-NO28 C3 and PZP like alpha-2-macroglobulin domain containing 8 (CPAMD8) | TCCTACAACCACATGCAGTTGTGGGACCGCAGTTTGGTCCTGGGGAC CATTCATACCCACACACCCAGCTTGTGCCTGTGGTTAACATCTCAGAA AACTCTGGTAAATGATCACTCCAGGATATTGACAAGAATACACGTTAC TGATCTTACTCAC (SEQ ID NO: 57) |
| 3UTR-NO29 vimentin (VIM) | AAATTGCACACACTCAGTGCAGCAATATATTACCAGCAAGAATAAAAA AGAAATCCATATCTTAAAGAAACAGCTTTCAAGTGCCTTTCTGCAGTT TTTCAGGAGCGCAAGATAGATTTGGAATAGGAATAAGCTCTAGTTCTT AACAACCGACACTCCTACAAGATTTAGAAAAAAGTTTACAACATAAT CTAGTTTACAGAAAAATCTTGTGCTAGAATACTTTTTAAAAGGTATTTT |
| | GAATACCATTAAAACTGCTTTTTTTTTTCCAGCAAGTATCCAACCAACT TGGTTCTGCTTCAATAAATCTTTGGAAAAACTC (SEQ ID NO: 58) |
| 3UTR-NO30 HDGF like 1 (HDGFL1) | TTACCAGCGTTTCCAGAAGAGCCCCTGCCCCGTTCCTGCTGCGGCCT GGCCGTTCTTGGGGAATCTGACCACGGCGTGCAAACTGGGACTGCCT TTCCCTCTCCTCAGCCCGTCCTCCTCCAACCCGCGCTCCTTTGCCCTG CCGGGCAACAGGATGGCAGGCCACCTGACTCTCACCTCTGTGCCCCC (SEQ ID NO: 59) |
| 3UTR-NO31 transthyretin (TTR) | GGGACTTCTCCTCCAGTGGACCTGAAGGACGAGGGATGGGATTTCAT GTAACCAAGAGTATTCCATTTTTACTAAAGCAGTGTTTTCACCTCATAT GCTATGTTAGAAGTCCAGGCAGAGACAATAAAACATTCCTGTGAAAG GCACTTTTCATTCCACTTTAACTTGATTTTTTAAATTCCCTTATTGTCCC TTCCAAAAAAAAGAGAATCAAAATTTTACAAAGAATCAAAGGATTCT AGAAAGTATCTGGGCAGAACGCTAGGAGAGATCCAAATTTCCATTGT CTTGCAAGCAAAGCACGTATTAAATATGATCTGCAGCCATTAAAAAGA CACATTCTGTAAA (SEQ ID NO: 60) |
| 3UTR-NO34 spermidine synthase (SRM) | GCCCAGGCGCCACCACTGATGCCACCCAGGACCTCGGACCTTGGAGC CTGCGGGGTGCCTCGGCCCCTCCAGCCCCGGGCCGGACCTCCTGCTG GCTCTCGCCCACCAACCAAGTGTTACAAGCCCCAGAATGCTGCCCGG CCTGCCCTGCTGGGCGGACTGTCTGTGTGTCTGTCTCTCTGGCGTTCC ACCTCCAAGCCTATACCAGCTGTGTACAGCGCCATCTCTCTGCCTTCT GTTGCCCCTCACTCACCAAACACGTGTATTTATAGC (SEQ ID NO: 61) |
| 3UTR-NO35 hemoglobin subunit alpha 1 (HBA1) | GCTGGAGCCTCGGTGGCCATGCTTCTTGCCCCTTGGGCCTCCCCCCAG CCCCTCCTCCCCTTCCTGCACCCGTACCCCCGTGGTCTTTGAATAAAG TCTGAGTGGGCGGC (SEQ ID NO: 62) |
| 3UTR-NO40 pre-mRNA processing factor 8 (PRPF8) | CCGTTTCCCTGCCTCCTGCTTCAGCCTCCCGAGGCCGAAGCCTCAGC CCCTCCAGACAGGCCGCTGACATTCAGCAGTTTGGCCTCTTTCCCTCT GTCTGTGCTTGTGTTGTTGACCTCCTGATGGCTTGTCATCCTGAATAA AATATAATAATAAATTTTGTATAAATAGG (SEQ ID NO: 63) |
| 3UTR-NO42 LMBR1 domain containing 1 (LMBRD1) | CAGCCTTCTGTCTTAAAGGTTTTATAATGCTGACTGAATATCTGTTATG CATTTTTAAAGTATTAAACTAACATTAGGATTTGCTAACTAGCTTTCAT CAAAAATGGGAGCATGGCTATAAGACAACTATATTTTATTATATGTTTT CTGAAGTAACATTGTATCATAGATTAACATTTTAAATTACCATAATCATG CTATGTAAATATAAGACTACTGGCTTTGTGAGGGAATGTTTGTGCAAA ATTTTTTCCTCTAATGTATAATAGTGTTAAATTGATTAAAAATCTTCCAG AATT (SEQ ID NO: 64) |
| 3UTR-NO43 interferon alpha 1 (IFNA1) | CATCTGGTCCAACATGAAAACAATTCTTATTGACTCATACACCAGGTC ACGCTTTCATGAGTTCTGTCATTTCAAAGACTCTCACCCCTGCTATAA CTATGACCATGCTGATAAACTGATTTATCTATTTAAATATTTATTTAACT |
| | ATTCATAAGATTTAAATTATTTTTGTTCATATAACGTCATGTGCACCTTT ACACTGTGGTTAGTGTAATAAAACATGTTCCTTATATTTACTC (SEQ ID NO: 65) |
| 3UTR-NO44 coiled-coil domain containing 146 (CCDC146) | ATATGTGAACAAATCCAGGCCTCTCAAGGAAAAGACTTCAACCAGGC TTCCTTGTACCCACAGGTGAAAAATGTGAGCATAATACTTCTAATATTA TTGATAAGTAAGGTAACCACAATTAGTCAGCAACAGAGTACAACAGG GTTTCTATTTACCCACCAACTACTATACCTTTCATGACGTTGAATGGGA CATAGAACTGTCCTACATTTATGTCAAAGTATATATTTGAATCGCTTATA TTTTCTTTTTCACTCTTTATATTGAGTACATTCCAGAAATTTGTAGTAGG CAAGGTGCTATAAAAATGCACTAAAAATAAATCTGTTCTCAATG (SEQ ID NO: 66) |
| 3UTR-NO45 ghrelin and obestatin prepropeptide (GHRL) | TCGCCCACAAGCCTTACTCACCTCTCTCTAAGTTTAGAAGCGCTCATC TGGCTTTTCGCTTGCTTCTGCAGCAACTCCCACGACTGTTGTACAAGC TCAGGAGGCGAATAAATGTTCAAACTGTA (SEQ ID NO: 67) |
| 3UTR-NO50 growth hormone 1 (GH1) | CGGGTGGCATCCCTGTGACCCCTCCCCAGTGCCTCTCCTGGCCCTGG AAGTTGCCACTCCAGTGCCCACCAGCCTTGTCCTAATAAAATTAAGTT GCATC (SEQ ID NO: 10) |

The above 3'-UTR sequences were synthesized by Sangon Biotechnology (Shanghai) Co., Ltd. According to the quality analysis report of Sangon, the 3'-UTR sequences were consistent with the theoretically designed sequences.

### Example 5 Synthesis of polyA

The following polyA sequences were designed.

**Table 3: poly A sequence listing**

| **poly(A) designation** | **Nucleotide sequence** |
|---|---|
| polyA-V1 | AAAAAAAAAAAAAAAAAAAAAAAAAAAAAGCATATGACTAAAAAA AAAAAAAAAAAAAAAAAAAAAAAAAAAAAAAAAAAAAAAAAAAA AAAAAAAAAAAAAAAAA (SEQ ID NO: 11) |
| polyA-V2 | AAAAAAAAAAAAAAAAAAAAAAAAAAAAGCATATGTCAAAAAAAAAA AAAAAAAAAAAAAAAAAAAAAAAAAAAAAAAAAAAAAAAAAAAAAAA AAAAAAAAAAAAAAAAA (SEQ ID NO: 68) |
| polyA-V3 | AAAAAAAAAAAAAAAAAAAAAAAAAAAAAAGCATATCAGTAAAAAA AAAAAAAAAAAAAAAAAAAAAAAAAAAAAAAAAAAAAAAAAAAAAA AAAAAAAAAAAAAAAAAA (SEQ ID NO: 69) |
| polyA-V4 | AAAAAAAAAAAAAAAAAAAAAAAAAAAAACGATATGACTAAAAAA AAAAAAAAAAAAAAAAAAAAAAAAAAAAAAAAAAAAAAAAAAAA |
| | AAAAAAAAAAAAAAAAAA (SEQ ID NO: 70) |
| polyA-V5 | AAAAAAAAAAAAAAAAAAAAAAAAAAAAAAAAAACGATGATACTAAAAAA AAAAAAAAAAAAAAAAAAAAAAAAAAAAAAAAAAAAAAAAAAAAAAAAAA AAAAAAAAAAAAAAAAAAA (SEQ ID NO: 71) |
| polyA-V6 | AAAAAAAAAAAAAAAAAAAAAAAAAAAAAAAAAAACGGTTGGAAAAAAA AAAAAAAAAAAAAAAAAAAAAAAAAAAAAAAAAAAAAAAAAAAAAAAAAA AAAAAAAAAAAAAAAAA (SEQ ID NO: 72) |
| polyA-V7 | AAAAAAAAAAAAAAAAAAAAAAAAAAAAAATTTCGGTTGGGAAAA AAAAAAAAAAAAAAAAAAAAAAAAAAAAAAAAAAAAAAAAAAA AAAAAAAAAAAAAAAAAA (SEQ ID NO: 73) |
| polyA-V8 | AAAAAAAAAAAAAAAAAAAAAAAAAAAAAAcagcgtatatAAAAAAAAAA AAAAAAAAAAAAAAAAAAAAAAAAAAAAAAAAAAAAAAAAAAAAA AAAAAAAAAAAAAAA (SEQ ID NO: 74) |
| polyA-V9 | AAAAAAAAAAAAAAAAAAAAAAAAAAAAAAgatatgcactAAAAAAAAAAAAAAAAAA AAAAAAAAAAAAAAAAAAAAAAAAAAAAAAAAAAAAAAAAAAAAAAAAAAAAAA AAAAAAAAAAAAAAAA (SEQ ID NO: 75) |
| polyA-V10 | AAAAAAAAAAAAAAAAAAAAAAAAAAAAAAAAAAAcatgtacgatAAAAAAAAA AAAAAAAAAAAAAAAAAAAAAgatatgcactAAAAAAAAAAAAAAAAAAAAAAAA AAAAAAAAAAAAAAAAAAAAA (SEQ ID NO: 76) |

The above polyA sequences were synthesized by IDT (intergrated DNA technologies) Co., Ltd. According to the quality analysis report of IDT, the sequences were consistent with the theoretically designed sequences.

### Example 6 Construction of engineered plasmid

Homology arm sequences were introduced by means of PCR at both ends of the S protein coding sequence of SARS-COV-2 as set forth in SEQ ID NO: 12, and then the resulting PCR product was homologously recombined with the large molecular fragment, which was recovered from double digestion of plasmid H with BamHI and KpnI. The resulting product was then transformed into competent cells DH5α. Single clones were selected, and sequencing was verified to finally obtain the correct engineered plasmid. The 5'-UTR used in this engineered plasmid was 5UTR-NO54 as set forth in SEQ ID NO: 9, the 3'-UTR was 3UTR-NO50 as set forth in SEQ ID NO: 10, and the sequence of the polyA used was set forth in SEQ ID NO: 11.

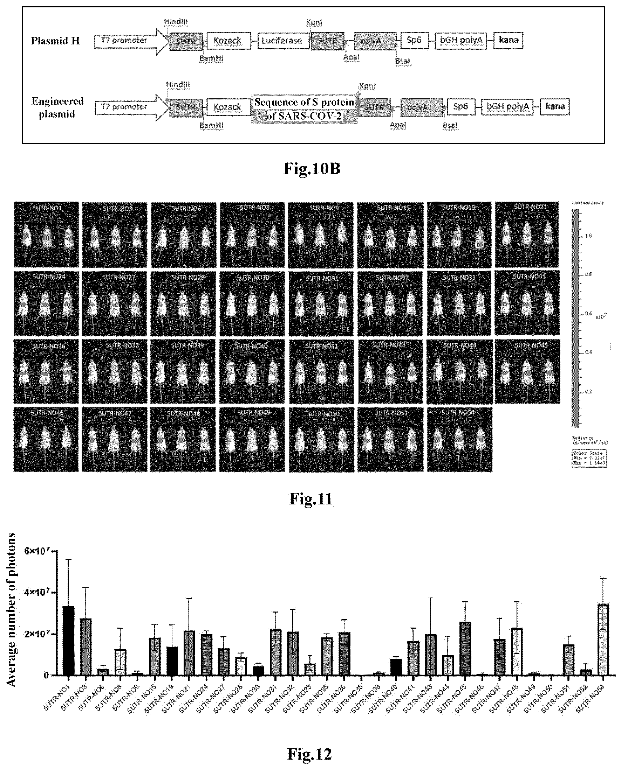

Data characterization: starting from plasmid H, sequencing verification was performed. The results showed that the nucleotide sequence was completely inserted into the correct position and was completely consistent with the theoretical sequence. The map of the engineered plasmid is shown in Figure 9, the flow chart of construction is shown in Figure 1B, and the schematic diagram of construction and modification is shown in Figure 10B.

### Example 7 Preparation of SARS-COV-2 Vaccine mRNA

1. The engineered plasmid template prepared in Example 6 was extracted to ensure that the supercoil rate of the plasmid is above 90%.
2. Plasmid Linearization
   (1) Enzyme linearization: 20 µg of the engineered plasmid was subject to linearization using the respective enzymes for digestion.
      Note: the reaction system was adjusted according to the actual situation. Subject to the purification kit column, the plasmid in each reaction system should not exceed 20µg.
   (2) The system was formulated into a 0.2 mL tube, mixed well, and incubated in a 37°C incubator for overnight or 2 h for enzyme digestion reaction.
   (3) 1 µL of the digested product and the original plasmid were subject to electrophoresis to check whether the digestion was complete.
3.Purification of the linearized plasmid: Recovery using Takara recovery kit
   (1) 3 times the amount of Buffer DC was added to the PCR reaction solution (or other enzymatic reaction solution) (if the amount of Buffer DC to be added is less than 100 µL, then 100 µL should be added) and the reaction was mixed well.
   (2) The Spin Column in the kit was placed on the Collection Tube.
   (3) The solution from step (1) above was transferred to the Spin Column and centrifuged at 12,000 rpm for 1 minute at room temperature. The filtrate was discarded.
   (4) 700 µL of Buffer WB was added to the Spin Column, centrifuged at 12,000 rpm for 30 seconds at room temperature, and the filtrate was discarded.
4. Phenol-chloroform extraction (to remove RNase, proteins, etc.)
   (1) The plasmid to be extracted was diluted to 300 µL or 500 µ. An equal volume of phenol chloroform was added (note the separation of the layers and do not absorb the upper layer), and the tube was centrifuged at 12,000 rpm for 10 min.
   (2) After centrifugation, the upper layer of liquid was aspirated as much as possible. An equal volume of phenol chloroform was added, and the tube was centrifuged at 12,000 rpm for 10 min.
   (3) After centrifugation, the upper layer of liquid was aspirated as much as possible again (be careful not to aspirate the lower layer this time). 0.1×5M NaCl and 0.7×isopropanol were added, and the tube was subject to ice bath for 15 minutes. The tube was centrifuged at 12000 rpm for 10 minutes and the supernatant was discarded.
   (4) The tube was washed once with 200 µL of 70% ethanol (pre-cooled), and centrifuged at 12,000 rpm for 1 min. The supernatant was discarded and the tube was centrifuged again at 12,000 rpm for 1 min, and the pellet was thoroughly aspirated with a small pipette tip.
   (5) After drying at room temperature, an appropriate amount of UItraPure DNase/RNase-Free-Distilled Water was added with thorough mixing.
   (6) The concentration of the purified sample was determinted by OneDrop.
      Identification: 100 ng of the purified plasmid together with the original plasmid were subject to electrophoresis. After confirming that the plasmid was completely linearized and had no impurity bands, it can be used for mRNA synthesis.
*5. In vitro* transcription of mRNA (see Vazyme IVT reaction kit)
   (1) Cleaning the experimental area: First, the biosafety cabinet was sterilized with UV light for 0.5 h, then wiped clean with alcohol cotton balls and sprayed with RNase inhibitor. After 5 min, the inhibitor was wiped clean with alcohol cotton balls and the RNA experiment started.
   (2) System preparation: Before preparing the system, individual component reagents were brought out, vortex mixed on an oscillator, centrifuged, and placed in an ice box for later use.
      Note: During the preparation of the system, the whole process was carried out on ice, and the plasmid template and IVT system were prepared separately.
   (3) Preparation of IVT system: all components except plasmid template were added below the liquid surface of the system, and T7 RNA polymerase was added finally. The system was vortex mixed thoroughly, and uracil (U) was replaced by N1-methylpseudouridine.
   (4) The required amount of plasmid was transferred into a new PCR tube, which was incubated with the IVT system for 5 min. Finally, the two systems were mixed together, vortex mixed, centrifuged, and incubated at 37°C for 2 h.
   (5) The system was prepared into a 0.2 mL flat-top thin-walled tube, mixed well, and placed in a PCR instrument for reaction at 37°C for 2 h. Note: The reaction system can also be simultaneously scaled up for production according to the required production volume.
   (6) The linearized plasmid template was removed using DNase. The reaction system was added with 1 µL (20 µL system) or 5 µL (100 µL system) DNase and incubated at 37°C for 15 min.
6. Purification method: (see Thermo MEGAclear Kit)
   Column purification
   (1) Each reaction sample was transferred to a 1.5 mL EP tube (if the system is less than 100 µL, then Elution Solution is added to make up to 100 µL), which was mixed gently;
   (2) 350 µL Binding Solution Concentrate was added, mixed gently with a pipette;
   (3) 250 µL of anhydrous ethanol was added, mixed gently with a pipette;
   (4) The filter cartridge provided with the kit was inserted into the collection tube. 700 µL of the mixture was added into the filter cartridge, combined at room temperature for 10 min, and centrifuged at 12,000 g for 1 min. The filtrate was discared, the collection tube was resued, and then RNA washing was performed;
   (5) 500 µL Wash Solution was added and filtering through the filter was performed (centrifuged at 12000 g for 1 min);
   (6) Step 5 was repeated;
   (7) The Wash Solution was removed and centrifugation was continued at the highest speed for 1 min to remove the residual Wash Solution;
   (8) An appropriate amount (80-100 µL) of preheated RNase-Free Distilled Water was added into the filter cartridge, with the lid closed. The tube was allowed to stand at 70°C for 10 min, and centrifuged at 12,000 g for 1 min. The number of elutions can be increased as needed.
   (9) Concentration determination: the concentration was determined by onedrop or Qubit (determination was performed after 10-fold dilution).
7. Identification: 5 µL of diluted purified sample was mixed with NorthernMax Formaldehyde Load Dye, incubated at 75°C for 10 minutes, and then kept on ice. Then gel electrophoresis with 1% agarose gel was performed. After confirming that the mRNA size was correct and the band was not diffuse, the next step of capping reaction can be performed.
8. Capping reaction

### mRNACap1 type capping reaction:

The Cap1 type cap structure and reaction principle are as follows:
pppN1(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc
5'-Cap1 type cap structure:
cap G¹G² = m⁷G⁺-5'-ppp-5'-Gm^{2'}-3'-p- [m⁷ = 7-CH₃; m^{2'} = 2'-*O*-CH₃; -ppp- = - PO₂H-O-PO₂H-O-PO₂H)-; -p- = -PO₂H-], 37°C for 5 min or 65°C for 5 min (CELL SCRIPT, the reaction system is shown in Table 4 below).

**Table 4: Cap1 type capping reaction system**

| | |
|---|---|
| H₂O | Make up to 67 µL |
| No capping mRNA | 25 pmol |

| | |
|---|---|
| Note: Due to the influence of the system, the amount of cappping mRNA per time (100µL system) should not exceed 60µg. | |

Capping (100 µL system) (see CELL SCRIPT Capping Reaction Kit) is shown in Table 5 below.

**Table 5: 100 µL capping system**

| | |
|---|---|
| 10×ScriptCap Capping Buffer | 10µL |
| 10 mM GTP | 10µL |
| 20 mM SAM | 2.5µL |
| ScriptGuard RNase Inhibitor | 2.5µL |
| ScriptCap 2'-O-Methyltransferase (100 U/µL) | 4µL |
| ScriptCap Capping Enzyme (10 U/µL) | 4µL |

The pre-heated mRNA was mixed with the above system and incubated at 37°C for 1 h.

### 9. Purification method: the same as the purification method for product obtained after in vitro transcription

Characterization data: the concentration was determined using onedrop or Qubit.

Experimental results: The sequence of the final mRNA product encoding the S protein of SARS-COV-2 is the sequence obtained by replacing all uracil (U) nucleosides in SEQ ID NO: 15 with N1-methyl pseudouridine. The experiment confirmed that the final mRNA product was obtained.

### Example 8 Screening test of 5'-UTR

The effects of 5'-UTRs on the luciferase expression level were compared using *in vivo* imaging (IVIS) data.

The mRNA synthesis process was as follows:
The different 5'-UTRs in Example 3 were inserted between HindIII and BamHI of plasmid G to obtain a series of plasmids having the same sequence in other parts but different 5'-UTR sequences. The engineered plasmid was replaced by these plasmids, and a series of mRNAs having the same sequence in other parts but different 5'-UTR sequences were prepared according to the method described in Example 7.

The mouse *in vivo* imaging experiment (IVIS) was as follows:
The specific process of encapsulating mRNA products containing different 5'-UTRs was as follows:
The batch size was 1.5 mL, the mass ratio of mRNA to lipid was 1:10, and the concentration of HAc-NaOAc buffer (0.2 M, pH 5.0) in the final aqueous phase was 0.025M. Each prescription sample was prepared using microfluidic equipment (MPE-L2) and chip (SN.000035) at a flow rate of 9 mL/min:3 mL/min for aqueous phase: alcohol phase. The two phases were injected into the microfluidic chip for mixing according to the interface requirements. The experimental design information is shown in Table 6 below.

**Table 6: Experimental design**

| Group | Lipid | Lipid:DSPC:C HO-HP:M-DMG2000(Mol %) | Note |
|---|---|---|---|
| 5UTR-NO(1,2...)5 4 | SM-102 | 50:10:38.5:1.5 | The prescription information regarding the formulated mRNA aqueous phase and lipid alcohol phase is shown in Table 7 below. |

**Table 7: Aqueous phase-alcohol phase prescription table**

| Aqueous phase (mRNA) | Mass /mg | mRNA sample concentrat ion | mRNA volume / µL | Acetic acid - sodium acetate buffer (pH 5.0 ) volume / µL | Depc water volume / µL | Total volume / µL |
|---|---|---|---|---|---|---|
| | 0.3 | X ( usually 2 µg / µL ) | Y (300 µg /X) | 141 | 1125-141-Y | 1125 |

| | Components | Mother liquor concentrat ion ( ethanol preparatio n ) | Molar mass % | Mass /mg | Volume required for working fluid /µL | Total volume/ µL |
|---|---|---|---|---|---|---|
| Alcohol phase ( lipid nanoparticle s ) | Lipid SM-102 (MW:710.2) | 20.0 mg/mL | 50% | 3.00 | 150 | 375 |
| | DSPC (MW:790.14) | 10.0 mg/mL | 10% | 0.67 | 67 | |
| | CHO-HP (MW : 386.7) | 20.0 mg/mL | 38.5% | 1.26 | 63 | |
| | M-DMG2000 (MW : 2650) | 25.0 mg/mL | 1.5% | 0.34 | 13 | |
| | EtOH (MW : 46.07) | | NA | NA | 82 | |

Among them, the chemical structure of lipid SM-102 is shown below:

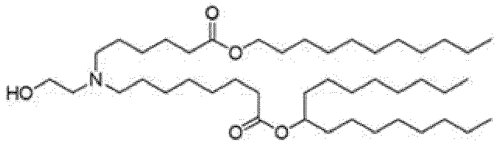

### SM-102

The encapsulated preparation samples were dialyzed for fluid exchange, as follows:
Dialysis solution preparation:
1×PBS + 8% sucrose solution: 2 packets of 1 ×PBS powder injection were brought into a beaker, dissolved and mixed with 2L DEPC water. Then 160g sucrose was further added and mixed to obtain 1×PBS + 8% sucrose solution.

Each group of drug solutions was placed in a 100 kD dialysis bag, and immersed in a beaker containing 1 L of dialysis solution. The beaker was wrapped in aluminum foil and dialyzed at 100 rpm for 1 h at room temperature. Then the dialysis solution was replaced and dialysis continued for 1 hour.

Regarding the obtained final mRNA preparations containing different 5'-UTRs Nanjing Yunqiao Purui Biotech Co., Ltd. was commissioned to do the animal imaging experiments. The specific process was as follows:
Male BALB/C mice, 5-7 weeks old, were raised and tested in an SPF laboratory environment. The encapsulated mRNA preparation product was injected into the tail vein. Three mice were tested for each 5'-UTR, and for each mouse, 20 µg of the mRNA preparation product was injected into the tail vein. After 16h-18h, the mice were anesthetized by means of isoflurane inhalation anesthesia and injected with D-Luciferin luciferase development substrate (150 mg/kg). The animals were placed in a supine position, and the signal distribution and expression intensity of luciferin in the animals *in vivo* were observed under the IVIS *in vivo* imaging system.

Observation of relevant indicators:
(1) Images observed by IVIS, including the original images and the comparison images after unifying the bar values of the photon number;
(2) Analysis data regarding fluorescence signal distribution and expression intensity of luciferin.

The experimental results are shown in Figures 11 and 12: the 5'-UTRs shown in 5UTR-NO1, 5UTR-NO3, 5UTR-NO21, 5UTR-NO24, 5UTR-NO31, 5UTR-NO32, 5UTR-NO36, 5UTR-NO43, 5UTR-NO45, and 5UTR-NO48 demonstrated better effects, and the 5'-UTR shown in 5UTR -NO54 demonstrated the optimum effect.

### Example 9 Screening test of 3'-UTR

The effects of 3'-UTRs on luciferase expression level were screened and compared using *in vivo* imaging (IVIS) data.

### 1. Containing no 5'-UTR

mRNAs which contain no 5'-UTR and contain a 3'-UTR shown in 3UTR-NO3, 3UTR-NO30 or 3UTR-NO50 were prepared by referring to Example 7, and then these mRNAs were prepared into final mRNA preparations according to Example 8, respectively. Animal imaging experiments were conducted by Nanjing Yunqiao Puri Biotech Co., Ltd. The specific process was as follows:
Male BALB/C mice, 5-7 weeks old, were raised and tested in an SPF laboratory environment. The encapsulated mRNA preparation product was injected into the tail vein. Three mice were tested for each mRNA preparation product, and for each mouse, 20 µg of the mRNA preparation product was injected into the tail vein. After 16h-18h, the mice were anesthetized by means of isoflurane inhalation anesthesia and injected with D-Luciferin luciferase development substrate (150 mg/kg). The animals were placed in a supine position, and the signal distribution and expression intensity of luciferin in the animals *in vivo* were observed under the IVIS *in vivo* imaging system.

Observation of relevant indicators:
(1) Images observed by IVIS, including the original images and the comparison images after unifying the bar values of the photon number;
(2) Analysis data regarding fluorescence signal distribution and expression intensity of luciferin.

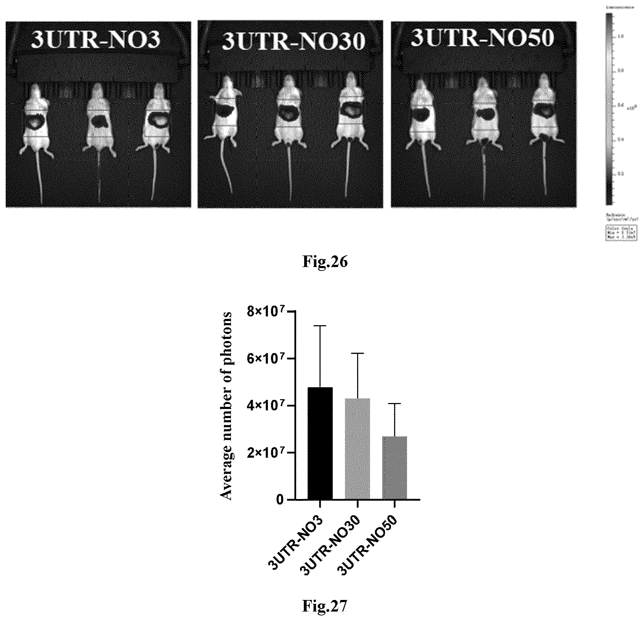

The experimental results are shown in Figures 26 and 27. The mRNAs which contain no 5'-UTR but contain a single 3' -UTR shown in 3UTR-NO3, 3UTR -NO30 or 3UTR-NO50 can increase the expression level of the protein. Among them, the mRNA containing a single 3'-UTR shown in 3UTR-NO3 or 3UTR-NO30 has a better effect than the mRNA containing a single 3'-UTR shown in 3UTR-NO50.

### 2. Containing 5'-UTR

The mRNA synthesis process was as follows:
5UTR-NO54 was inserted between HindIII and BamHI of blank plasmid G, and after the 5'-UTR sequence was fixed, different 3'-UTRs in Example 4 were inserted between ApaI and KpnI of the plasmid to obtain a series of plasmids containing the 5UTR-NO54 sequence, and having the same sequence in other parts but different 3'-UTRs. The engineered plasmid was replaced by these plasmids, and a series of mRNAs containing the 5UTR-NO54 sequence, and having the same sequence in other parts but different 3'-UTRs were prepared according to the method described in Example 7.

The mouse *in vivo* imaging experiment (IVIS) was as follows:
The specific process of encapsulating the mRNA products containing different 3'-UTRs was similar to the process of encapsulating the mRNA products containing different 5'-UTRs in Example 8, except that the mRNA products containing different 5'-UTRs were replaced by the mRNA products containing different 3'-UTRs.

Regarding the obtained mRNA preparations containing different 3'-UTRs, Nanjing Yunqiao Purui Biotech Co., Ltd. was commissioned to do the animal imaging experiments. The specific process was as follows:
Male BALB/C mice, 5-7 weeks old, were raised and tested in an SPF laboratory environment. The encapsulated mRNA preparation product was injected into the tail vein. Three mice were tested for each 3'-UTR, and for each mouse, 20 µg of the mRNA preparation product was injected into the tail vein. After 16h-18h, the mice were anesthetized by means of isoflurane inhalation anesthesia and injected with D-Luciferin luciferase development substrate (150 mg/kg). The animals were placed in a supine position, and the signal distribution and expression intensity of luciferin in the animals *in vivo* were observed under the IVIS *in vivo* imaging system.

Observation of relevant indicators:
(1) Images observed by IVIS, including the original images and the comparison images after unifying the bar values of the photon number;
(2) Analysis data regarding fluorescence signal distribution and expression intensity of luciferin.

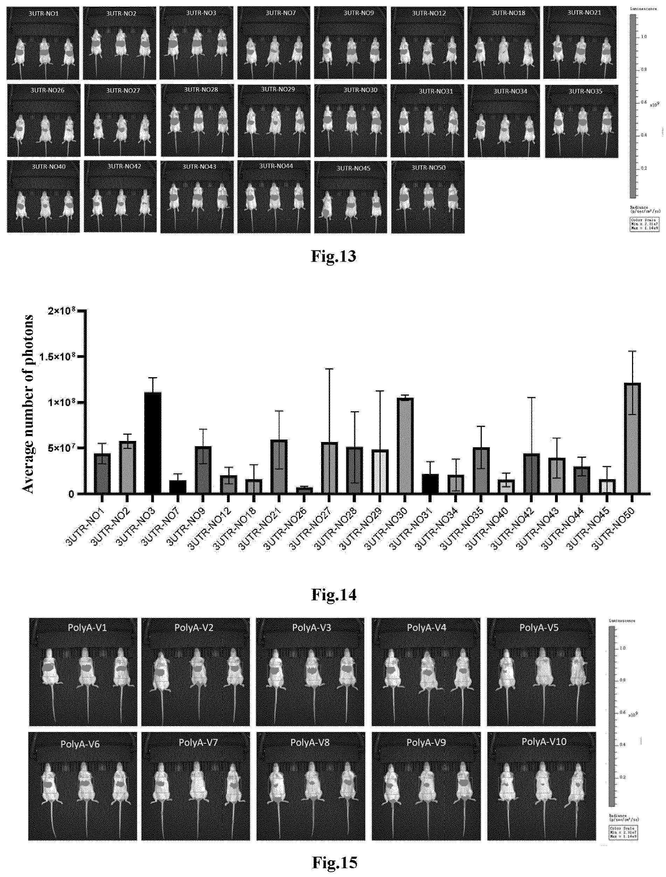

The experimental results are shown in Figures 13 and 14. When 5UTR-NO54 was chosen as the 5'-UTR, different 3'-UTRs were used, and it was found that 3UTR-NO2, 3UTR-NO3, 3UTR-NO9, 3UTR-NO21, 3UTR-NO27, 3UTR-NO28, 3UTR-NO30, 3UTR-NO35, and 3UTR-NO50 showed better effects.

### Example 10 Screening test of polyA

Preparation of mRNA: different polyA sequences in Example 5 were inserted into the ApaI site of plasmid F to obtain a series of plasmids having the same sequence in other parts but different polyA sequences. The engineered plasmid was replaced by these plasmids, and a series of mRNAs having the same sequence in other parts but different polyA sequences were prepared according to the method described in Example 7.

The effects of PolyA on mRNA were compared using *in vivo* imaging IVIS data.

The mouse *in vivo* imaging experiment (IVIS) was as follows:
The specific process of encapsulating the mRNA products containing different PolyA was similar to the specific process of encapsulating the mRNA products containing different 5'-UTR in Example 8, except that the mRNA products containing different 5'-UTR were replaced by the mRNA products containing different Poly A.

Regading the obtained final mRNA preparations with different PolyA, Nanjing Yunqiao Puri Biotechnology Co., Ltd. was commissioned to do the animal imaging experiments. The specific process was as follows:
Male BALB/C mice, 5-7 weeks old, were raised and tested in an SPF laboratory environment. The encapsulated mRNA preparation product was injected into the tail vein. Three mice were tested for each 5'-UTR, and for each mouse, 12 µg of the mRNA preparation product was injected into the tail vein. After 16h-18h, the mice were anesthetized by means of isoflurane inhalation anesthesia and injected with D-Luciferin luciferase development substrate (150 mg/kg). The animals were placed in a supine position, and the signal distribution and expression intensity of luciferin in the animals *in vivo* were observed under the IVIS *in vivo* imaging system.

Observation of relevant indicators:
(1) Images observed by IVIS, including the original images and the comparison images after unifying the bar values of the photon number;
(2) Analysis data regarding fluorescence signal distribution and expression intensity of luciferin.

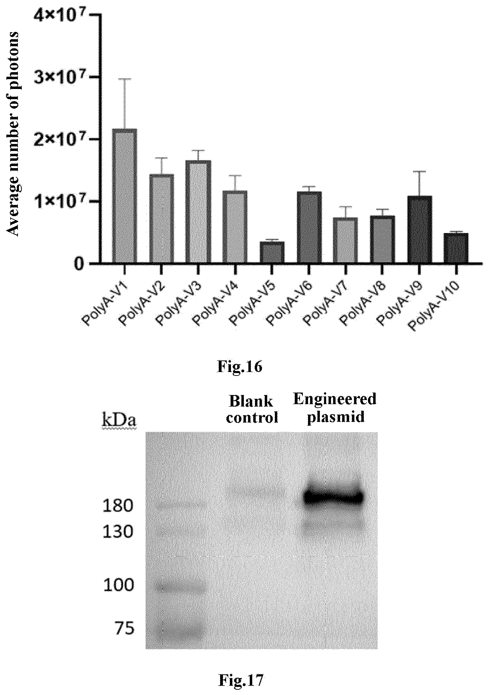

The results from Figures 15 and 16 demonstrated that polyAV1, polyAV2, polyAV3, polyAV4, polyAV6, and polyAV9 can improve the protein expression level *in vivo* compared to other polyA sequences, among which polyAV1 showed the highest protein expression level *in vivo.*

### Example 11 Preparation of SARS-COV-2 mRNA vaccine and in vivo immune serum in mouse

The mRNA used in this example was prepared according to the method described in Example 7, and its sequence was the sequence obtained by replacing all uracil (U) nucleosides in SEQ ID NO: 15 with N1-methylpseudouridine.

The formulation system for encapsulating SARS-COV-2 vaccine mRNA in the in vivo immunization assay is shown in Tables 8 and 9 below.

**Table 8: Encapsulation formulation system**

| | | |
|---|---|---|
| Microfluidic device | Micro syringe | flow rate of encapsulation: 9ml/min (aqueous phase): 3ml/min (alcohol phase) |
| Microfluidic Chip | SN.000058 | |
| Lipid | Compound (I) (see preparation method of Example 17) | |

**Table 9: Aqueous phase-alcohol phase prescription table**

| Aqueous phase (mRNA) | Mass/mg | mRNA sample concentration | mRNA volume/ µL | Acetic acid-sodium acetate buffer (pH 5.0) volume/µ L | Depc water volume/ µL | Total volume/µ L |
|---|---|---|---|---|---|---|
| | 1 | 2.7 µg/µL | 360 | 469 | 2921 | 3750 |

| | Components | Mother liquor concentration (ethanol preparation) | Molar mass% | Mass/mg | Volume required for workin g fluid /µL | Total volume/µ L |
|---|---|---|---|---|---|---|
| Alcohol Phase | Compound (I) (MW:764.26) | 20.0 mg/mL | 50% | 10 | 500 | 1250 |
| | DSPC(MW:790.14) | 10.0 mg/mL | 10% | 2.07 | 207 | |
| | CHO-HP(MW:386.7) | 20.0 mg/mL | 38.5% | 3.9 | 195 | |
| | M-DMG2000(MW:2650) | 25.0 mg/mL | 1.5% | 1.04 | 42 | |
| | EtOH(MW:46.07) | | NA | NA | 306 | |

| | | | | | | |
|---|---|---|---|---|---|---|
| M-DMG2000 is PEG 2000- DMG | | | | | | |

Dialysis solution preparation:
1×PBS + 8% sucrose solution: 2 packets of 1 ×PBS powder injection were brought into a beaker, dissolved and mixed with 2L DEPC water. Then 160g sucrose was further added and mixed to obtain 1×PBS + 8% sucrose solution.

Each group of drug solutions was placed in a 100 kD dialysis bag, and immersed in a beaker containing 1 L of dialysis solution. The beaker was wrapped in aluminum foil and dialyzed at 100 rpm for 1 h at room temperature. Then the dialysis solution was replaced and dialysis continued for 1 hour.

The dialyzed preparation samples were sterilized and filtered, specifically as follows: the samples were sterilized and filtered through a 0.22 µm disposable filter membrane; and the encapsulation efficiency of the final preparation products was tested.

Grouping for *in vivo* immunization assay of SARS-COV-2 mRNA vaccine: female mice of 16 g ~ 18g were adapted and raised in SPF environment. The mice were divided into three groups for experiment, namely: saline group (blank control, 6), mRNA vaccine-5µg test group (6), mRNA vaccine-10µg test group (6). The test sample was injected intramuscularly, and the time of the first injection of the vaccine was recorded as the first day; on the 26^{th} day, the second immunization was performed (consistent with the dosage and method of the first immunization). On the 7^{th}, 14^{th}, 33^{rd} and 44^{th} days post vaccine immunization, orbital blood was collected to test IgG antibodies specific to delta (for testing in Example 12). On the 14^{th}, 37^{th} and 44^{th} days, orbital blood was collected from the mouse for neutralization assay of wild-type SARS-COV-2 and mutant delta pseudoviruses (for testing in Example 13).

Serum acquisition steps: the blood was collected into a non-anticoagulant tube, placed on ice for 30 minutes, and centrifuged at 4°C, 3500rpm for 10 minutes. After stratification, the light yellow liquid was carefully aspirated from the upper layer with a pipette.

### Example 12 IgG antibody titration assay against S protein in serum from the mouse immunized with SARS-COV-2 mRNA vaccine

The immune serum required for the specific S protein IgG antibody titration assay was provided by the experiment in Example 11.

Materials and reagents: SARS-CoV-2 Spike Trimer (T19R, G142D, EF156-157del, R158G, L452R, T478K, D614G, P681R, D950N, purchased from acrobiosystems, catalog number SPN-C52He), Goat anti-Mouse IgG(H+L) HRP Conjugated, Ms mAb to SARS spike glycoprotein [IA9], 96-well plate, PBS, bovine serum albumin, one-component TMB colorimetric solution, and stop solution.

The first step was antigen coating: the antigen SARS-CoV-2 Spike Trimer was diluted to 1µg/mL with 1×PBS, 100µL of antigen was added to each well, the plate was sealed with a sealing film, incubated at 37°C for 4h, or 4°C for 16h, the fluid in the well was discarded, the plate was washed three times with the washing solution.

The second step was blocking: blocking solution (3% BSA, formulated with PBST) was added to the plate at 230 µL/well, the plate was sealed with a sealing film, incubated at 37°C for 40-60 minutes, the plate was washed three times after completion of the incubation.

Sample preparation: The mouse immune serum sample to be tested (provided in Example 11) was diluted to a suitable concentration gradient (1:10-1:31250, with a starting dilution of 1:10 and 5-fold dilution). A larger dilution volume was used, and it was generally required that the sample aspiration volume was >20 µL. The positive control Ms mAb to SARS spike glycoprotein [IA9] was diluted 1:50000. 100 µL/well, incubated at 37°C for 60 min, and washed three times after completion of the incubation.

Addition of enzyme-labeled secondary antibody Goat anti-Mouse IgG (H+L) HRP Conjugated: enzyme-labeled secondary antibody was diluted at 1:10000. Incubated at 37°C for 40 min, and washed three times after completion of the incubation.

Substrate development (TMB development substrate): 100 µL per well, protected from light at room temperature for 5 minutes.

Stop reaction: 50 µL of stop solution was added to each well to terminate the reaction and the experimental results were meastured within 20 minutes.

Indicators for detection: ELISA results were expressed in OD at wavelength nm (450nm-630nm).

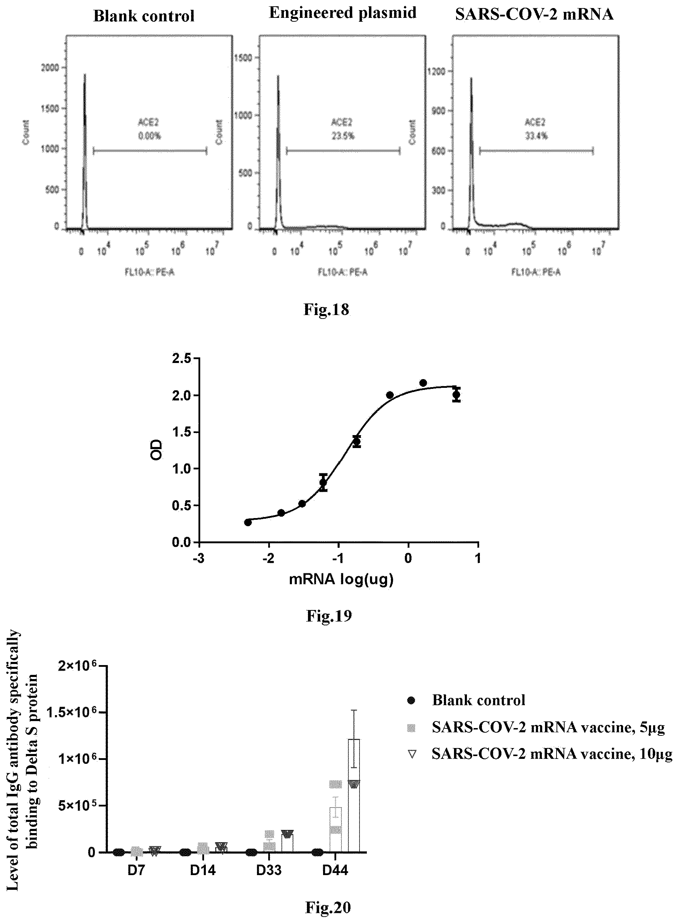

The test results are shown in Figure 20: SARS-COV-2 mRNA vaccine produced higher IgG antibodies against the Delta S protein (10µg mRNA vaccine produced an average IgG antibody titer of 1.3+e6 on day 44).

### Example 13 Pseudovirus neutralization assay for serum from the mouse immunized with SARS-COV-2 mRNA vaccine

The immune serum required for the pseudovirus neutralization assay was provided by the experiment in Example 11.

Reagents and materials: Pseudovirus SARS-CoV-2 Delta strain-Fluc: 1~2×10 ⁴ TCID50/mL

Inactivated mouse serum: 56°C, 30 min, Vero cells, DMEM, FBS, luciferase reporter gene detection reagent.

The starting dilution of serum samples (diluted with serum-free DMEM): 1:20 (serum samples were required to be inactivated in advance at 56°C for 30 min).

100 µL of DMEM medium containing 10% FBS was added to wells B2~G2 of the 96-well white plate as a cell control (CC), and 100 µL of DMEM medium containing 10% FBS was added to wells B3~G3 as a virus control (VC).

150 µL of diluted serum sample (provided in Example 11) was added to wells B4 to B12 of the 96-well white plate as the initially dilutedsample, and 100 µL of DMEM serum-free medium was added to wells C4 to G12.

A volume of 50 µL was transferred from B4 to B12 to the next row, mixed well, followed by 3-fold gradient dilution for a total of 7 gradients. The excess 50 µL of fluid in the last row was discarded.

50µL of diluted virus was added to each well except for B2~G2, and the virus titer was diluted to 13000TCID50/mL, incubated at 37°C for 1 h.100µL of Vero cells with a density of 0.5×10⁶ cells/well was added to each well. After incubation for 24 h, the 96-well white plate was removed and equilibrated to room temperature. 100µL of culture medium was aspirated from the plate, and 100µL of Bio-Lite reporter gene detection reagent equilibrated at room temperature was added. The plate was shaken for 2 min, and the chemiluminescence value (RLU) was detected by the plate reader after standing at room temperature for 5 min.

Nanjing Novozymes Biotechnology Co., Ltd. was commissioned to implement the pseudovirus neutralization assay, and the specific experimental steps were carried out as described above.

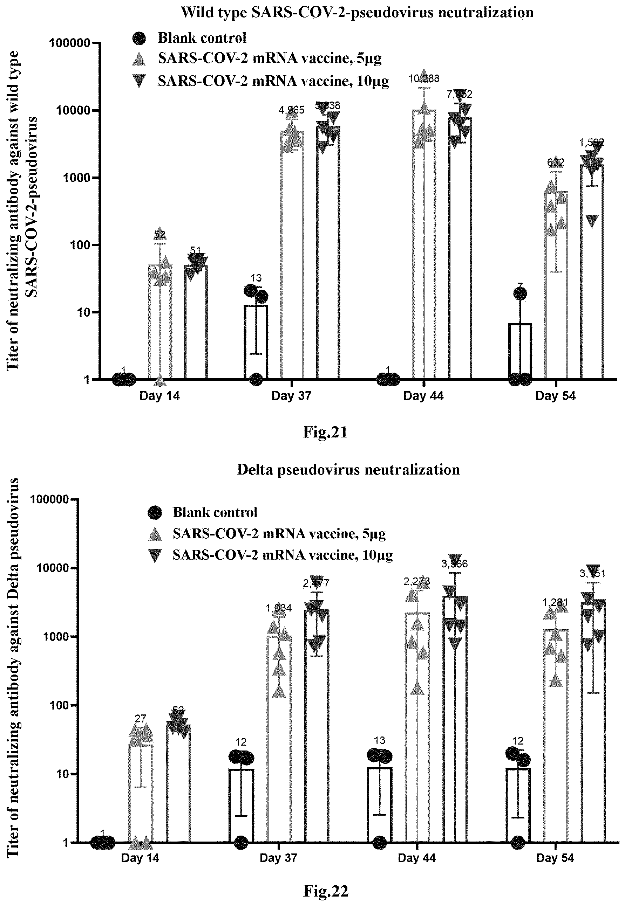

The results are shown in Figures 21 and 22: SARS-COV-2 mRNA vaccine can produce a superior immune protection effect against wild-type SARS-COV-2 (the neutralizing antibody IC50 by 10µg mRNA vaccine on the 44th day is 7952) and Delta (the neutralizing antibody IC50 by 10µg mRNA vaccine on the 44th day is 3966) pseudoviruses.

### Example 14 Western blot activity assay on cells with engineered plasmid

Reagents and materials: HEK293T, DMEM basic (1×), Opti-MEM^{™} I reduced serum medium, Lipofectamine^{®} 2000 Reagent, 6-well plate, sample buffer&leammli 2× concentrate, 8% SDS-PAGE Color Preparation Kit, Ms mAb to SARS spike glycoprotein [IA9], Goat anti-Mouse IgG (H+L) HRP Conjugated, PageRuler prestained Protein Ladder, bovine serum albumin, ultrasensitive ECL luminescence reagent, methanol.

One day in advance, 293T cells were plated in a 60 mm culture dish at 2×10⁶/well, and cell transfection was performed when the cell confluence reached 70%-90% the next day.

The transfection agent was prepared according to the transfection ratio required by the assay, and each well of cells was transfected with 5 µg of the engineered plasmid, incubated in a CO₂ incubator at 37°C for 24 hours. Western blot assay was then performed.

Cells were lysed and heated at 95-100°C for 10 minutes for complete denaturation. When the samples resumed to room temperature, they were loaded onto the gel for electrophoresis. After completion, the membrane transfer was performed for half an hour. The membrane was blocked with 5% skim milk powder at room temperature for 1 hour. The primary antibody (Ms mAb to SARS spike glycoprotein [IA9] 1:2000) was diluted with 1% BSA, incubated at room temperature for 1 hour, and then washed three times with TBST. The secondary antibody (Goat anti-Mouse IgG(H+L) HRP Conjugated, 1:5000) was diluted with 1% BSA, shaken at room temperature for 1 hour, and washed three times with TBST. Chemiluminescence was detected on ECL colorimeter.

The results are shown in Figure 17: The engineered plasmid vector can successfully express the corresponding antigen protein at a high level in the cell.

### Example 15 FACS activity assay on cells with engineered plasmid and corresponding mRNA

mRNA preparation: The preparation process for SARS-COV-2 mRNA is detailed in Example 7.

Reagents and materials: HEK293T, Lipofectamine^{®} 2000 Reagent, DMEM basic (1×), 24-well plate, TrypLE Express, flow cytometry tube (BD), primary antibody for flow cytometry: ACE2-Fc (acrobiosystems), secondary antibody for flow cytometry: PE anti-human IgG Fc Recombinant Antibody (Biolegend).

Transfection of HEK293T cells with the engineered plasmid or the corresponding mRNA: the HEK293 T cells were plated in a 24-well plate at 1×10⁵ /well one day in advance, and the cells were transfected when the cell confluence reached 70%-90% the next day.

The transfection agent was prepared according to the transfection ratio required by the assay. 100 µL of transfection complex was added per well, 3 µg DNA or 3 µg mRNA was transfected per well, incubated in a CO₂ incubator at 37°C for 24 h, and then FACS was performed.

FACS detection: the cell supernatant was removed, and cell detachment solution was added to each well for detachment at room temperature for 1 min. 1 mL 1×PBS was added for resuspension, centrifuged at 2000 rpm for 2 min, and the supernatant was removed. The blank group was the non-transfection group (if there is an isotype control antibody, then an isotype control group is introduced).

Primary antibody incubation: the primary antibody ACE2-Fc (acrobiosystems) was diluted to 1 µg/mL in 1×PBS, and 100 µL was added to each sample. The sample was incubated at 4°C for 30 min, centrifuged at 2000 rpm for 2 min and the supernatant was removed. The sample was washed three times with 1 mL 1×PBS.

Secondary antibody incubation: the secondary antibody PE anti-human IgG Fc Recombinant Antibody (Biolegend) was diluted to 1 µg/mL in 1×PBS, and 100 µL was added to each sample. The sample was incubated at 4°C for 30 min, centrifuged at 2000 rpm for 2 min and the supernatant was removed. The sample was washed three times with 1 mL 1×PBS, and then 100 µL 1× PBS was added for resuspension, followed by detection.

The test results are shown in FIG18: both the engineered plasmid and the mRNA product can express the correct S protein in the transfected cells at a high level.

### Example 16 ELISA activity assay of SARS-COV-2 mRNA

mRNA preparation: The mRNA preparation process is detailed in Example 7.

Reagents and materials: DMEM basic (1×), fetal bovine serum FBS, Lipofectamine^{™} MessengerMAX^{™}, Opti-MEM^{™} I reduced serum medium, 0.25% Trypsin-EDTA (1×), lysate, Cocktail, Anti-nCoV-RBD Neutralizing Antibody, HRP conjugated Anti-nCoV-RBD-1 Neutralizing antibody, PBS buffer, ELISA colorimetric solution, stop solution, 96-well plate.

A 96-well plate was plated at a density of 6×10⁵ cells /mL, and the number of cells was 6×10⁴. Three replicates were set for each sample. 24 hours after plating, mRNA was transfected, and the transfection complex was prepared. Transfection was performed according to the mRNA transfection gradient (µg/well). Three replicates were set, and the transfection gradient was 0.05µg, 0.015µg, 0.03µg, 0.06µg, 0.18µg, 0.54µg, 1.62µg, and 4.86µg. 24 hours after transfection, the culture medium was discarded. The cells were washed once with PBS, lysed with 120µL NP40 lysis buffer (plus 100× cocktail) on ice for 10 minutes, centrifuged at 4000rpm for 10 minutes, and 100µL was aspirated for subsequent ELISA assay. Anti-nCoV-RBD Neutralizing Antibody was diluted to 1µg/mL with coating solution (PBS buffer), 100µL/well, and was allowed to stand overnight at 4°C. After washing, blocking solution (3% W/V BSA/PBS buffer) was added at 240 µL/well to the plate, and the plate was sealed with a sealing film, incubated at 37°C for 1 hour. After washing, 100 µL of the harvested cell supernatant was pipetted into a 96-well plate and incubated at room temperature for 2.5 hours. After washing, the HRP conjugated Anti-nCoV-RBD-1 Neutralizing antibody was diluted 8000 times with sample diluent (0.5% W/V BSA/washing solution (washing solution is PBS buffer plus 0.05% Tween20)), 100 µL/well, incubated at room temperature for 1 hour. After washing, ELISA colorimetric solution was added for color development, 100 µL/well, incubated at room temperature for 10 minutes. Stop solution was added to terminate the reaction, and the results were read with a plate reader.

The results are shown in Figure 19: SARS-COV-2 mRNA product can express S protein in cells in a dose-dependent manner.

### Example 17 Synthesis of Compound (I)

### Step 1): Synthesis of nonyl 8-((3-hydroxycyclohexyl)amino)octanoate

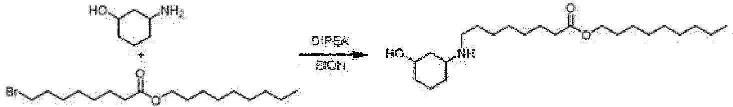

1-octylnonyl 8-bromooctanoate (3.50 g, 10 mmol), 3-aminocyclohexanol (11.5 g, 100 mmol), and 30 mL of ethanol were added in sequence into a 100 mL reaction bottle. After stirring and dissolving, N,N-diisopropylethylamine (2.58 g, 20 mmol) was further added. The mixture was reacted at room temperature for 24 h, and 100 mL of dichloromethane was added. After washing with water three times, the mixture was dried over anhydrous sodium sulfate, concentrated, and purified using a flash column chromatography system (dichloromethane: methanol = 20:1 to 5:1) to yield nonyl 8-((3-hydroxycyclohexyl)amino)octanoate (2.34 g, 61%).

¹HNMR (600 MHz, CDCl₃) δ 4.20-4.13 (m, 0.5H), 4.05 (t, 2H), 3.83 (m, 0.5H), 3.09 (m, 0.5H), 2.87 (m, 0.5H), 2.76-2.59 (m, 2H), 2.29 (t, 2H), 2.00 (m, 0.5H), 1.93-1.78 (m, 1.5H), 1.78-1.65 (m, 2H), 1.65-1.46 (m, 8H), 1.40-1.18 (m, 20H), 0.88 (t, 3H).

LCMS:384.3[M+H]⁺ .

### Step 2): Synthesis of compound (I)

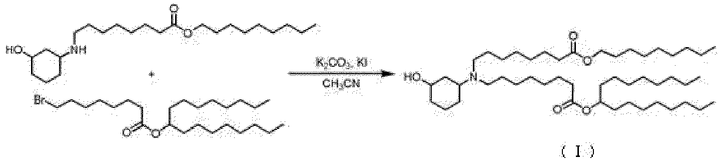

Nonyl 8-((3-hydroxycyclohexyl)amino)octanoate (383 mg, 1 mmol), 1-octylnonyl 8-bromooctanoate (554 mg, 1.2 mmol), and acetonitrile 20 mL were added in sequence into a 100 mL reaction bottle. After stirring and dissolving, potassium carbonate (276 mg, 2 mmol) and potassium iodide (166 mg, 1 mmol) were further added. The mixture was reacted at room temperature for 24 h and 100 mL of dichloromethane was added. After washing with water three times, the mixture was dried over anhydrous sodium sulfate, concentrated, and purified using a flash column chromatography system (dichloromethane: methanol = 20:1 to 5:1) to yield compound (I) (596 mg, 78%).

¹H NMR (600 MHz, CDCl₃) δ 4.95-4.81 (m, 1H), 4.30-4.12 (m, 0.5H), 4.07 (t, 2H), 3.72-3.61 (m, 0.5H), 2.97 (m, 0.5H), 2.64-2.52 (m, 0.5H), 2.48-2.37 (m, 4H), 2.30 (q, 4H), 1.93-1.81 (m, 2H), 1.72-1.57 (m, 8H), 1.51 (t, 4H), 1.43-1.18 (m, 56H), 0.89 (m, 9H).

LCMS:765.3[M+H]⁺*.*

### Example 18 Assay on the protective activity of the SARS-COV-2 mRNA vaccine against the Omicron variant

Grouping for *in vivo* immunization assay of SARS-COV-2 mRNA vaccine: female mice of 16 g ~ 18g were adapted and raised in SPF environment. The mice were divided into three groups for experiment, namely: saline group (blank control, 5), mRNA vaccine-5µg test group (5), mRNA vaccine-10µg test group (5). The test sample was injected intramuscularly, and the time of the first injection of the vaccine was recorded as the first day; on the 21^{th} day, the second immunization was performed (consistent with the dosage and method of the first immunization). On the 7^{th}, 14^{th}, 28^{th} and 35^{th} days post vaccine immunization, orbital blood was collected to test IgG antibodies specific to Omicron. On the 35^{th} day, blood was collected from the mouse, and omicron (BA.2) pseudovirus neutralization assay was performed. At the same time, spleen lymphocytes were obtained for IFNγ-Elispot assay to test the cellular immune response of the vaccine.

**Serum acquisition steps:** the blood was collected into a non-anticoagulant tube, placed on ice for 30 minutes, and centrifuged at 4°C, 3500rpm for 10 minutes. After stratification, the light yellow liquid was carefully aspirated from the upper layer with a pipette.

### 1. Determination on the titer of the IgG antibody specifically binding to Omicron S protein after immunization of the mouse with SARS-COV-2 mRNA vaccine

Materials and reagents: Omicron Spike Trimer (purchased from acrobiosystems, catalog number SPN-C52Hz), Goat anti-Mouse IgG (H+L) HRP Conjugated, Ms mAb to SARS spike glycoprotein [IA9], 96-well plate, PBS, bovine serum albumin, one-component TMB colorimetric solution, and stop solution.

The experimental procedures were performed by referring to Example 12.

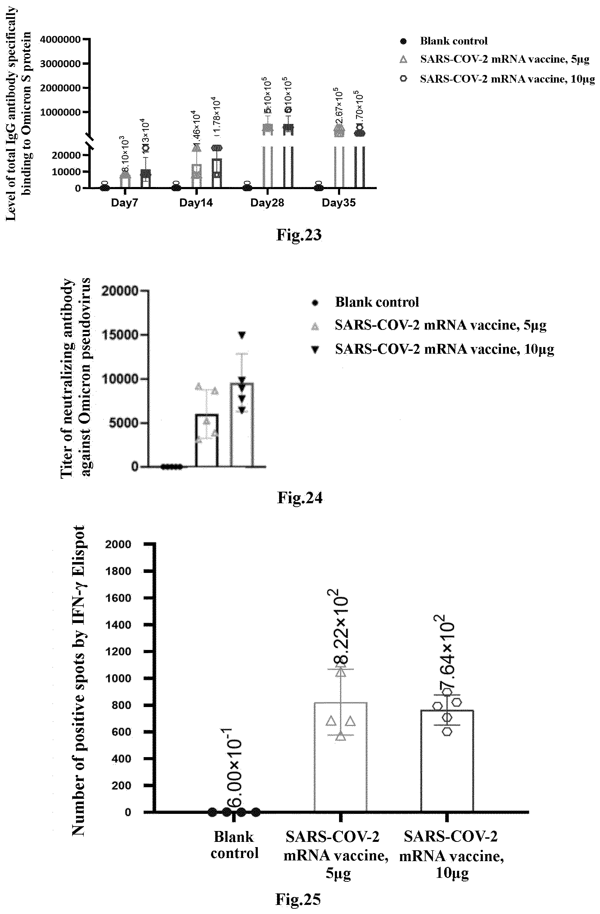

The results are shown in Figure 23: SARS-COV-2 mRNA vaccine produced IgG antibody against the Omicron S protein at a higher level (5µg mRNA vaccine produced an average IgG antibody titer of 2.67+e5 on day 35).

### 2. Determination on the titer of the neutralizing antibody against Omicron pseudovirus after immunization of the mouse with SARS-COV-2 mRNA vaccine

Reagents and materials: Pseudovirus SARS-CoV-2 Omicron (BA.2) strain-Fluc: 1~2× 10⁴ TCID50/mL

Inactivated mouse serum: 56°C, 30 min, Vero cells, DMEM, FBS, luciferase reporter gene detection reagent.

The experimental procedures were performed by referring to Example 13.

The results are shown in Figure 24: SARS-COV-2 mRNA vaccine can produce a superior immune protection effect against the Omicron (IC50 of the neutralizing antibody by 10µg mRNA vaccine on the 35^{th} day is 7952) pseudovirus.

### 3. IFN-y Elispot assay of spleen lymphocytes

Reagents and materials: SARS-CoV-2 Spike S1 Peptide Pool (PP003-A), SARS-CoV-2 Spike S2 Peptide Pool (PP003-B), mouse lymphocyte separation medium (Dakewe Biotech, 7211011), Mouse IFN-γ ELISPOT kit (Dakewe Biotech, CT317-PR2)
(1) Lymphocyte separation: 5 mL of mouse lymphocyte separation medium which was warmed to room temperature was placed into a 60 mm culture dish and the spleen was ground. The separation medium in which spleen cells were suspended was transferred immediately to a 15 mL centrifuge tube and covered with 1 mL of RPMI 1640 culture medium (the liquid surface boundary should be kept clear). The tube was centrifuged at room temperature at 800 g for 30 min in a horizontal rotor; and the acceleration and deceleration should be set at a lower value. After centrifugation, the cells were layered, and the lymphocyte layer was aspirated. 10 mL of RPMI 1640 culture medium was further added, and the tube was inverted for washing. The tube was centrifuged at room temperature at 250 g for 10 min and the cells were collected. The supernatant was discarded, and the cells were resuspend in culture medium and counted.
(2) Peptide library stimulation Elispot assay:
   Positive control wells: positive stimulator working solution was added (the PMA stock solution was diluted 10-fold with serum-free medium or RPMI 1640 to a concentration of 10 µg/mL, then 10 µL/well was added to each well).

Negative control wells: the culture medium for cell resuspension was added.

Experimental wells: the experimenter's own stimuli was added (the S1+S2 peptide library was diluted with serum-free culture medium or RPMI 1640. The final concentration of the working solution is 1 µg/mL).

The specific process was carried out according to the kit manual.

The results are shown in Figure 25: After the mouse was immunized with SARS-COV-2 mRNA vaccine, lymphocytes can be activated to produce a stronger cellular immune response.

The above Examples have demonstrated that, the inventors of the present application have successfully designed a novel RNA coding sequence of SARS-COV-2 S protein and a novel mRNA vaccine obtained therefrom. The mRNA vaccine shows cross-protective reactions against different strains, including SARS-COV-2 wild-type, Delta and Omicron variants, and has superior immune effects against Delta and Omicron variants, and can be developed and mass-produced more rapidly.

The above Examples have also demonstrated that, the inventors of the present application have also successfully constructed a universal polynucleotide molecule (e.g., 3'-UTR, 5'-UTR, vector, etc.) for preparing a novel RNA vaccine (e.g., mRNA vaccine), which improves the level of protein expressed by the mRNA. The polynucleotide molecule can not only be used to prepare an mRNA vaccine with superior immune effects against SARS-COV-2 wild-type, Delta and Omicron variants, but can also be used to transfect cells or bacteria to produce corresponding antibodies or antigenic proteins, and is therefore a universal core element combination.

The above Examples have also demonstrated that, the inventors of the present application have also successfully constructed a universal polynucleotide molecule (such as 3'-UTR, 5'-UTR, vector, etc.), which improves the level of protein expressed by the mRNA. The polynucleotide molecule can be used not only for preparing proteins and/or polypeptides *in vitro,* but also for producing corresponding proteins and/or polypeptides *in vivo,* and is a universal core element.

## Claims

1. An RNA encoding the S protein of SARS-COV-2, wherein the S protein comprises the amino acid sequence as set forth in SEQ ID NO: 13; preferably, the RNA comprises the nucleic acid sequence as set forth in SEQ ID NO: 14, and more preferably, the nucleic acid sequence of the RNA is set forth in SEQ ID NO: 14.

2. The RNA of claim 1, wherein one or more uridine nucleosides, such as 1, 2, 3, 4, 5, 6, 7, 8, 9, 10 uridine nucleosides or at least 50%, at least 60%, at least 70%, at least 80%, at least 90% or 100% of the uridine nucleosides in the RNA are replaced by at least one nucleoside selected from the group consisting of pseudouridine, N1-methyl-pseudouridine, N1-ethyl pseudouridine, 2-thiouridine, 4'-thiouridine, 5-methylcytosine, 5-methyluridine, 2-thio-1-methyl-1-deaza-pseudouridine, 2-thio T-methyl-pseudouridine, 2-thio-5-aza-uridine, 2-thio-dihydro-pseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine or 5-methoxyuridine and 2'-O-methyluridine, preferably pseudouridine or N1-methylpseudouridine or N1-ethylpseudouridine, more preferably N1-methylpseudouridine; and/or
one or more cytosine nucleosides in the RNA, such as 1, 2, 3, 4, 5, 6, 7, 8, 9, 10 cytosine nucleosides or at least 50%, at least 60%, at least 70%, at least 80%, at least 90% or 100% of the cytosine nucleosides are replaced by 5-methylcytosine nucleoside.

3. The RNA of claim 1 or 2, wherein all or part of the uridine nucleosides in the RNA are replaced by pseudouridine, preferably N1-methylpseudouridine; preferably, all or part of the uridine nucleosides in the nucleic acid sequence as set forth in SEQ ID NO: 14 are replaced by pseudouridine, preferably N1-methylpseudouridine; more preferably, all uridine nucleosides in the nucleic acid sequence as set forth in SEQ ID NO: 14 are replaced by N1-methylpseudouridine.

4. The RNA of any one of claims 1 to 3, further comprising at least one of a 5'-cap structure, a 5'-UTR, a 3'-UTR and a polyA.

5. The RNA of claim 4, wherein the 5'-cap structure is selected from at least one of m⁷GpppG, m₂^{7,3'-O}GpppG, m⁷Gppp(5')N1 or m⁷Gppp(m^{2'-O})N1, preferably m⁷Gppp(5')N1 or m⁷Gppp(m^{2'-O})N1, wherein "m7G" represents a 7-methylguanosine cap nucleoside, "ppp" represents a triphosphate bond between the 5' carbon of the cap nucleoside and the first nucleotide of the primary RNA transcript, N1 is the 5'-most nucleotide, "G" represents a guanosine nucleoside, "7" represents a methyl group at the 7-position of guanine, and "m^{2'-O}" represents a methyl group at the 2' O-position of the nucleotide;
and/or the 5'-UTR is selected from the following (1)-(5):
(1) a 5'-UTR comprising an RNA sequence corresponding to the nucleic acid sequence as set forth in SEQ ID NOs: 9, 16, 17, 23, 24, 28, 29, 32, 37, 39 or 42, a homologue, a fragment or a variant thereof, wherein the homologue, the fragment or the variant has the same or better function of improving translation efficiency as the 5'-UTR shown in the RNA sequence corresponding to SEQ ID NOs: 9, 16, 17, 23, 24, 28, 29, 32, 37, 39 or 42; preferably, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 9, 16, 17, 23, 24, 28, 29, 32, 37, 39 or 42;
(2) a 5'-UTR consisting of an RNA sequence corresponding to the nucleic acid sequence as set forth in SEQ ID NOs: 9, 16, 17, 23, 24, 28, 29, 32, 37, 39 or 42;
(3) a 5'-UTR consisting of an RNA sequence corresponding to the nucleic acid sequence as set forth in SEQ ID NO: 9;
(4) a 5'-UTR obtained by concatenating two or more identical 5'-UTRs in (1) to (3) above; or
(5) a 5'-UTR obtained by concatenating two or more different 5'-UTRs in (1) to (3) above;
and/or the 3'-UTR is selected from the following (1)-(5):
(1) a 3'-UTR comprising an RNA sequence corresponding to the nucleic acid sequence as set forth in SEQ ID NOs: 47-67 or 10, a homologue, a fragment or a variant thereof; the homologue, the fragment or the variant has the same or better function of improving translation efficiency and/or stability as the 3'-UTR shown in the RNA sequence corresponding to SEQ ID NOs: 47-67 or 10; preferably, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 47-67 or 10;
(2) a 3'-UTR consisting of an RNA sequence corresponding to the nucleic acid sequence as set forth in SEQ ID NOs: 47-67 or 10;
(3) a 3'-UTR consisting of an RNA sequence corresponding to the nucleic acid sequence as set forth in SEQ ID NO: 10;
(4) a 3'-UTR obtained by concatenating two or more identical 3'-UTRs in (1) to (3) above; or
(5) a 3'-UTR obtained by concatenating two or more different 3'-UTRs in (1) to (3) above;
and/or the polyA is a truncated polyA, i.e., a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA is selected from the following (1)-(3):
(1) a polyA comprising an RNA sequence corresponding to the nucleic acid sequence as set forth in SEQ ID NOs: 68-70, 72, 75 or 11, a homologue, a fragment or a variant thereof, wherein the homologue, the fragment or the variant has the same or better function of improving translation efficiency and/or stability as the polyA shown in the RNA sequence corresponding to SEQ ID NOs: 68-70, 72, 75 or 11; preferably, the homologue comprises a nucleic acid sequence having at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 68-70, 72, 75 or 11;
(2) a polyA consisting of an RNA sequence corresponding to the nucleic acid sequence as set forth in SEQ ID NOs: 68-70, 72, 75 or 11; or
(3) a polyA consisting of an RNA sequence corresponding to the nucleic acid sequence as set forth in SEQ ID NO: 11.

6. The RNA of any one of claims 1-5, comprising the nucleic acid sequence as set forth in SEQ ID NO: 15, more preferably, the nucleic acid sequence of the RNA is set forth in SEQ ID NO: 15.

7. The RNA of claim 6, wherein all or part of the uridine nucleosides in the RNA are replaced by pseudouridine, preferably N1-methyl pseudouridine; preferably, all or part of the uridine nucleosides in the nucleic acid sequence as set forth in SEQ ID NO: 15 are replaced by pseudouridine, preferably N1-methyl pseudouridine; more preferably, all uridine nucleosides in the nucleic acid sequence as set forth in SEQ ID NO: 15 are replaced by N1-methyl pseudouridine.

8. A protein expressed by the RNA of any one of claims 1 to 7, preferably, the protein comprises the amino acid sequence as set forth in SEQ ID NO: 13, more preferably, the amino acid sequence of the protein is set forth in SEQ ID NO: 13.

9. A DNA encoding the RNA of any one of claims 1 to 7; preferably, the DNA comprises the nucleic acid sequence as set forth in SEQ ID NO: 12; preferably, the DNA comprises the nucleic acid sequence as set forth in SEQ ID NO: 8.

10. A vector comprising the DNA of claim 9.

11. A cell comprising the vector of claim 10.

12. A lipid nanoparticle comprising the RNA of any one of claims 1-7.

13. The lipid nanoparticle of claim 12, further comprising one or more of an ionizable cationic lipid, a helper lipid, a structural lipid and a PEG-lipid.

14. The lipid nanoparticle of claim 13, wherein the ionizable cationic lipid is selected from one or more of Dlin-MC3-DMA, Dlin-KC2-DMA, DODMA, c12-200 or DlinDMA; and/or the helper lipid is selected from one or more of DSPC, DOPE, DOPC or DOPS; and/or the structural lipid is selected from at least one of cholesterol, cholesterol ester, steroid hormone, steroid vitamin and bile acid; and/or the PEG-lipid is selected from PEG-DMG or PEG-DSPE, preferably PEG-DMG, more preferably, PEG-DMG is a polyethylene glycol (PEG) derivative of 1,2-dimyristyl glyceride, and further preferably, the average molecular weight of PEG is about 2000 or 5000 Daltons, preferably 2000 Daltons.

15. A pharmaceutical composition comprising the RNA of any one of claims 1-7, the protein of claim 8, the DNA of claim 9, the vector of claim 10, the host cell of claim 11 or the lipid nanoparticle of any one of claims 12-14, and a pharmaceutically acceptable carrier and/or excipient.

16. Use of the RNA of any one of claims 1 to 7, the protein of claim 8, the DNA of claim 9, the vector of claim 10, the host cell of claim 11, or the lipid nanoparticle of any one of claims 12 to 14, or the pharmaceutical composition of claim 15 in the preparation of a vaccine for preventing SARS-COV-2 infection.

17. An artificial RNA molecule comprising a 5'-UTR and a nucleic acid sequence encoding a protein and/or polypeptide of interest, wherein the 5'-UTR is selected from the following (1)-(5):
(1) a 5'-UTR comprising an RNA sequence corresponding to the nucleic acid sequence as set forth in SEQ ID NOs: 9, 16, 17, 23, 24, 28, 29, 32, 37, 39 or 42, a homologue, a fragment or a variant thereof, wherein the homologue, the fragment or the variant has the same or better function of improving translation efficiency as the 5'-UTR shown in the RNA sequence corresponding to SEQ ID NOs: 9, 16, 17, 23, 24, 28, 29, 32, 37, 39 or 42; preferably, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 9, 16, 17, 23, 24, 28, 29, 32, 37, 39 or 42;
(2) a 5'-UTR consisting of an RNA sequence corresponding to the nucleic acid sequence as set forth in SEQ ID NOs: 9, 16, 17, 23, 24, 28, 29, 32, 37, 39 or 42;
(3) a 5'-UTR consisting of an RNA sequence corresponding to the nucleic acid sequence as set forth in SEQ ID NO: 9;
(4) a 5'-UTR obtained by concatenating two or more identical 5'-UTRs in (1) to (3) above; or
(5) a 5'-UTR obtained by concatenating two or more different 5'-UTRs in (1) to (3) above.

18. The artificial RNA molecule of claim 17, further comprising a 3'-UTR and a polyA; Preferably, the 3'-UTR is selected from the following (1)-(5):
(1) a 3'-UTR comprising an RNA sequence corresponding to the nucleic acid sequence as set forth in SEQ ID NOs: 47-67 or 10, a homologue, a fragment or a variant thereof; the homologue, the fragment or the variant has the same or better function of improving translation efficiency and/or stability as the 3'-UTR shown in the RNA sequence corresponding to SEQ ID NOs: 47-67 or 10; preferably, the nucleic acid sequence of the homologue has at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 47-67 or 10;
(2) a 3'-UTR consisting of an RNA sequence corresponding to the nucleic acid sequence as set forth in SEQ ID NOs: 47-67 or 10;
(3) a 3'-UTR consisting of an RNA sequence corresponding to the nucleic acid sequence as set forth in SEQ ID NO: 10;
(4) a 3'-UTR obtained by concatenating two or more identical 3'-UTRs in (1) to (3) above; or
(5) a 3'-UTR obtained by concatenating two or more different 3'-UTRs in (1) to (3) above;
and/or the polyA is a truncated polyA, i.e., a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA is selected from the following (1)-(3):
(1) a polyA comprising an RNA sequence corresponding to the nucleic acid sequence as set forth in SEQ ID NOs: 68-70, 72, 75 or 11, a homologue, a fragment or a variant thereof, wherein the homologue, the fragment or the variant has the same or better function of improving translation efficiency and/or stability as the polyA shown in the RNA sequence corresponding to SEQ ID NOs: 68-70, 72, 75 or 11; preferably, the homologue comprises a nucleic acid sequence having at least 40%, 50%, 60%, 70%, 80%, 85%, 90%, 91%, 92%, 93%, 94%, 95%, 96%, 97%, 98% or 99% homology with the RNA sequence corresponding to the nucleic acid sequence as set forth in SEQ ID NOs: 68-70, 72, 75 or 11;
(2) a polyA consisting of an RNA sequence corresponding to the nucleic acid sequence as set forth in SEQ ID NOs: 68-70, 72, 75 or 11; or
(3) a polyA consisting of an RNA sequence corresponding to the nucleic acid sequence as set forth in SEQ ID NO: 11.

19. An RNA molecule comprising:
(1) a first nucleotide sequence encoding a polypeptide and/or protein of interest; and
(2) a second nucleotide sequence containing a 3'-untranslated region (3'-UTR);
the 3'-UTR comprises at least one of the following polynucleotides:
(a): 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146 and GHRL;
(b): a fragment of the 3'-UTR described in (a);
(c): a variant of the 3'-UTR described in (a); and
(d): a variant of the fragment described in (b);
the first nucleotide sequence and the second nucleotide sequence do not naturally occur in the same RNA molecule.

20. The RNA molecule of claim 19, wherein the gene is a human gene.

21. The RNA molecule of claim 19 or 20, wherein the second nucleotide sequence comprises at least one of the following polynucleotides:
(e): 3'-UTR derived from at least one of the genes COP1 and HDGFL1;
(f): a fragment of the 3'-UTR described in (e);
(g): a variant of the 3'-UTR described in (e); and
(h): a variant of the fragment described in (f).

22. The RNA molecule of any one of claims 19 to 21, wherein the second nucleotide sequence comprises at least one of the following polynucleotides :
an RNA encoded by a polynucleotide having a sequence as set forth in at least one of SEQ ID NOs: 47 to 67, a fragment of an RNA encoded by a polynucleotide having a sequence as set forth in at least one of SEQ ID NOs: 47 to 67, a variant of an RNA encoded by a polynucleotide having a sequence as set forth in at least one of SEQ ID NOs: 47 to 67, and a variant of a fragment of an RNA encoded by a polynucleotide having a sequence as set forth in at least one of SEQ ID NOs: 47 to 67;
preferably, the variant of an RNA encoded by a polynucleotide having a sequence as set forth in at least one of SEQ ID NOs: 47 to 67, the fragment of an RNA encoded by a polynucleotide having a sequence as set forth in at least one of SEQ ID NOs: 47 to 67, and the variant of a fragment of an RNA encoded by a polynucleotide having a sequence as set forth in at least one of SEQ ID NOs: 47 to 67 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67.

23. The RNA molecule of any one of claims 19 to 22, wherein the second nucleotide sequence comprises at least one of : 3'-UTR of at least two genes among the genes described in (a); a fragment of 3'-UTR of at least two genes among the genes described in (a); a variant of 3'-UTR of at least two genes among the genes described in (a); and a variant of a fragment of 3'-UTR of at least two genes among the genes described in (a).

24. The RNA molecule of any one of claims 19 to 23, wherein the second nucleotide sequence comprises at least one of: at least two copies of the 3'-UTR in (a), at least two copies of a fragment of the 3'-UTR in (a), at least two copies of a variant of the 3'-UTR in (a), and at least two copies of a variant of a fragment of the 3'-UTR in (a).

25. The RNA molecule of any one of claims 19 to 24, further comprising at least one of a promoter, a 5'- cap structure, a 5'-UTR and a polyA.

26. The RNA molecule of claim 25,
(1) the 5'-cap structure comprises at least one of m⁷GpppG, m₂^{7,3'-O}GpppG, m⁷Gppp(5')N1 and m⁷Gppp(m^{2'-O})N1;
(2) the 5' -UTR comprises:
i) at least one of: a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D, a fragment, a variant and a variant of a fragment thereof; preferably, an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46;
preferably, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and the variant of a fragment of an RNA encoded by a polynucleotide having a sequence as set forth in at least one of SEQ ID NOs: 16 to 46 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46;
ii) at least one of the following polynucleotides: an RNA encoded by a polynucleotide having a sequence as set forth in SEQ ID NO: 9, a fragment of an RNA encoded by a polynucleotide having a sequence as set forth in SEQ ID NO: 9, a variant of an RNA encoded by a polynucleotide having a sequence as set forth in SEQ ID NO: 9, and a variant of a fragment of an RNA encoded by a polynucleotide having a sequence as set forth in SEQ ID NO: 9;
preferably, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9;
iii) at least two copies of one of the polynucleotides in i) or ii); or
iv) at least two polynucleotides in i);
(3) the nucleotides constituting the polyA include at least 20, at least 40, at least 80, at least 100 or at least 120 A nucleotides; preferably, the nucleotides constituting the polyA include consecutively at least 20, at least 40, at least 80, at least 100 or at least 120 A nucleotides;
preferably, the nucleotides constituting the polyA include one or more nucleotides other than the A nucleotide; preferably, the polyA is a truncated polyA, preferably, a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11; preferably, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11;
more preferably, the polyA is an RNA encoded by a polynucleotide having a sequence as set forth in one of SEQ ID NOs: 68 to 70, 72, 75 and 11; preferably, the polyA is an RNA encoded by a polynucleotide having a sequence as set forth in SEQ ID NO: 11.

27. An RNA molecule comprising:
(1) a first nucleotide sequence encoding a polypeptide and/or protein of interest; and
(2) a third nucleotide sequence containing a 5'-untranslated region (5'-UTR);
the 5'-UTR comprises at least one of the following polynucleotides:
(a): a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D;
(b): a fragment of the 5'-UTR described in (a);
(c): a variant of the 5'-UTR described in (a); and
(d): a variant of the fragment described in (b);
the first nucleotide sequence and the third nucleotide sequence do not naturally occur in the same RNA molecule.

28. The RNA molecule of claim 27, wherein the gene is a human gene.

29. The RNA molecule of claim 27 or 28, wherein the third nucleotide sequence comprises at least one of the following polynucleotides: an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46;
preferably, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and a variant of a fragment of an RNA encoded by a polynucleotide having a sequence as set forth in at least one of SEQ ID NOs: 16 to 46 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46.

30. The RNA molecule of any one of claims 27 to 29, wherein the third nucleotide sequence comprises at least one of: 5'-UTR of at least two genes among the genes in (a), a fragment of 5'-UTR of at least two genes among the genes in (a), a variant of 5'-UTR of at least two genes among the genes in (a), and a variant of the fragment of 5'-UTR of at least two genes among the genes in (a);
and/or, the third nucleotide sequence comprises: at least two copies of the 5'-UTR in (a), at least two copies of a fragment of the 5'-UTR in (a), at least two copies of a variant of the 5'-UTR in (a), and at least two copies of a variant of a fragment of the 5'-UTR in (a).

31. An RNA molecule comprising:
(1) a first nucleotide sequence encoding a polypeptide and/or protein of interest; and
(2) a 5'-untranslated region (5'-UTR); wherein the 5'-UTR comprises at least one of the following polynucleotides: an RNA encoded by a polynucleotide having a sequence as set forth in SEQ ID NO: 9, a fragment of an RNA encoded by a polynucleotide having a sequence as set forth in SEQ ID NO: 9, a variant of an RNA encoded by a polynucleotide having a sequence as set forth in SEQ ID NO: 9, and a variant of a fragment of an RNA encoded by a polynucleotide having a sequence as set forth in SEQ ID NO: 9;
preferably, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9.

32. The RNA molecule of any one of claims 27 to 31, further comprising at least one of a promoter, a 5'- cap structure, a 3'-UTR and a polyA;
preferably, (1) the 5'- cap structure comprises at least one of m⁷GpppG, m₂^{7,3'-O}GpppG, m⁷Gppp(5')N1 and m⁷Gppp(m^{2'-O)}N1;
(2) the 3'-UTR comprises:
i): at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof;
preferably, an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10;
preferably, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10;
ii) at least two copies of one of the polynucleotides described in i); or
iii) at least two polynucleotides described in i);
(3) the nucleotides constituting the polyA include at least 20, at least 40, at least 80, at least 100 or at least 120 A nucleotides; preferably, the nucleotides constituting the polyA include consecutively at least 20, at least 40, at least 80, at least 100 or at least 120 A nucleotides;
preferably, the nucleotides constituting the polyA include one or more nucleotides other than the A nucleotide; preferably, the polyA is a truncated polyA; preferably, a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11; preferably, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11;
more preferably, the polyA is an RNA encoded by a polynucleotide having a sequence as set forth in one of SEQ ID NOs: 68 to 70, 72, 75 and 11; preferably, the polyA is an RNA encoded by a polynucleotide having a sequence as set forth in SEQ ID NO: 11.

33. The RNA molecule of any one of claims 19 to 32, wherein all or part of the uridines in the RNA molecule are replaced by at least one nucleoside selected from the group consisting of pseudouridine, N1-methyl-pseudouridine, N1-ethyl pseudouridine, 2-thiouridine, 4'-thiouridine, 5-methylcytosine, 5-methyluridine, 2-thio-1-methyl-1-deaza-pseudouridine, 2-thio T-methyl-pseudouridine, 2-thio-5-aza-uridine, 2-thio-dihydro-pseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine or 5-methoxyuridine and 2'-O-methyluridine, preferably, all or part of the uridine nucleosides in the RNA molecule are replaced by pseudouridine, N1-methylpseudouridine or N1-ethylpseudouridine;
preferably, one or more cytosine nucleosides or at least 50%, at least 60%, at least 70%, at least 80%, at least 90% or 100% of the cytosine nucleosides in the RNA molecule are replaced by 5-methylcytosine.

34. A DNA encoding the artificial RNA molecule of any one of claims 17 to 18 or a DNA encoding the RNA molecule of any one of claims 19 to 33.

35. A vector comprising the DNA of claim 34.

36. A vector comprising a fourth nucleotide sequence encoding a 3'-UTR, wherein the fourth nucleotide sequence comprises at least one of the following polynucleotides:
(a) a polynucleotide encoding a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146 and GHRL;
(b): a polynucleotide encoding a fragment of the 3'-UTR described in (a);
(c): a polynucleotide encoding a variant of the 3'-UTR described in (a); and
(d): a polynucleotide encoding a variant of the fragment described in (b).

37. The vector of claim 36, wherein the gene is a human gene; and/or the vector does not contain a polynucleotide encoding a protein and/or polypeptide of interest.

38. The vector of claim 36 or 37, wherein the fourth nucleotide sequence comprises at least one of the following polynucleotides :
(e): a polynucleotide encoding a 3'-UTR derived from at least one of the genes COP1 and HDGFL1;
(f): a polynucleotide encoding a fragment of the 3'-UTR described in (e);
(g): a polynucleotide encoding a variant of the 3'-UTR described in (e); and
(h): a polynucleotide encoding a variant of the fragment described in (f).

39. The vector of any one of claims 33 to 38, wherein the fourth nucleotide sequence comprises at least one of the following polynucleotides :
(1) at least one of: a polynucleotide having a sequence as set forth in at least one of SEQ ID NOs: 47 to 67, a fragment of a polynucleotide having a sequence as set forth in at least one of SEQ ID NOs: 47 to 67, a variant of a polynucleotide having a sequence as set forth in at least one of SEQ ID NOs: 47 to 67, and a variant of a fragment of a polynucleotide having a sequence as set forth in at least one of SEQ ID NOs: 47 to 67; preferably, the variant of a polynucleotide having a sequence as set forth in at least one of SEQ ID NOs: 47 to 67, the fragment of a polynucleotide having a sequence as set forth in at least one of SEQ ID NOs: 47 to 67, and the variant of a fragment of a polynucleotide having a sequence as set forth in at least one of SEQ ID NOs: 47 to 67 have at least 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67;
(2) a polynucleotide encoding a 3'-UTR of at least two genes among the genes described in (a); a polynucleotide encoding a fragment of the 3'-UTR of at least two genes among the genes described in (a); a polynucleotide encoding a variant of the 3'-UTR of at least two genes among the genes described in (a); and a polynucleotide encoding a variant of a fragment of the 3'-UTR of at least two genes among the genes described in (a); and
(3) at least one of: at least two copies of the 3'-UTR in (a), at least two copies of a fragment of the 3'-UTR in (a), at least two copies of a variant of the 3'-UTR in (a), and at least two copies of a variant of a fragment of the 3'-UTR in (a).

40. The vector of any one of claims 36 to 39, further comprising at least one of a promoter, a polynucleotide encoding 5'-UTR, and a polynucleotide encoding a polyA.

41. The vector of claim 40, wherein the vector satisfies at least one of the following (1) to (2):
(1) the 5'-UTR comprises:
i) at least one of a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D, a fragment, a variant and a variant of a fragment thereof; preferably, an RNA encoded by a polynucleotide having a sequence as set forth in at least one of SEQ ID NOs: 16 to 46, a fragment of an RNA encoded by a polynucleotide having a sequence as set forth in at least one of SEQ ID NOs: 16 to 46, a variant of an RNA encoded by a polynucleotide having a sequence as set forth in at least one of SEQ ID NOs: 16 to 46, and a variant of a fragment of an RNA encoded by a polynucleotide as set forth in at least one of SEQ ID NOs: 16 to 46;
preferably, the variant of an RNA encoded by a polynucleotide having a sequence as set forth in at least one of SEQ ID NOs: 16 to 46, the fragment of an RNA encoded by a polynucleotide having a sequence as set forth in at least one of SEQ ID NOs: 16 to 46, and the variant of a fragment of an RNA encoded by a polynucleotide having a sequence as set forth in at least one of SEQ ID NOs: 16 to 46 have at least 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 16 to 46;
ii) at least one of the following polynucleotides: an RNA encoded by a polynucleotide having a sequence as set forth in SEQ ID NO: 9, a fragment of an RNA encoded by a polynucleotide having a sequence as set forth in SEQ ID NO: 9, a variant of an RNA encoded by a polynucleotide having a sequence as set forth in SEQ ID NO: 9, and a variant of a fragment of an RNA encoded by a polynucleotide having a sequence as set forth in SEQ ID NO: 9;
preferably, the variant of an RNA encoded by a polynucleotide having a sequence as set forth in SEQ ID NO: 9, the fragment of an RNA encoded by a polynucleotide having a sequence as set forth in SEQ ID NO: 9, and the variant of a fragment of an RNA encoded by a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in SEQ ID NO: 9;
iii) at least two copies of one of the polynucleotides in i) or ii); or
iv) at least two polynucleotides in i);
(2) the nucleotides constituting the polyA include at least 20, at least 40, at least 80, at least 100 or at least 120 A nucleotides; preferably, the nucleotides constituting the polyA include consecutively at least 20, at least 40, at least 80, at least 100 or at least 120 A nucleotides;
preferably, the nucleotides constituting the polyA include one or more nucleotides other than the A nucleotide; preferably, the polyA is a truncated polyA, preferably, a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11; preferably, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11;
more preferably, the polyA is an RNA encoded by a polynucleotide having a sequence as set forth in one of SEQ ID NOs: 68 to 70, 72, 75 and 11; preferably, the polyA is an RNA encoded by a polynucleotide having a sequence as set forth in SEQ ID NO: 11.

42. A vector comprising a fifth nucleotide sequence encoding a 5'- UTR, wherein the fifth nucleotide sequence comprises at least one of the following polynucleotides:
(a): a polynucleotide encoding a 5'-UTR derived from at least one of the genes APOA1, CARD16, ALB, APOC1, EEF1A1, RBP4, GHRL, MPND, ASAH2B, FBX016, FBH1, SRM, NAAA, ACTB, MKNK2, ORM1, GADPH, NDUFAF2, FGFR2, MYCBPAP, CHMP2A, TSG101, PRPF8, NFKB2, NAE1, HDGFL1, GSDMD, FBXW12, FBXW10, FBXL8 and ATG4D;
(b): a polynucleotide encoding a fragment of the 5'-UTR described in (a);
(c): a polynucleotide encoding a variant of the 5'-UTR described in (a); and
(d): a polynucleotide encoding a variant of the fragment described in (b).

43. The vector of claim 42, wherein the gene is a human gene; and/or the vector does not contain a polynucleotide encoding a protein or polypeptide of interest.

44. The vector of claim 42 or 43, wherein the fifth nucleotide sequence comprises at least one of the following polynucleotides: a polynucleotide having a sequence as set forth in at least one of SEQ ID NOs: 16 to 46, a fragment of a polynucleotide having a sequence as set forth in at least one of SEQ ID NOs: 16 to 46, a variant of a polynucleotide having a sequence as set forth in at least one of SEQ ID NOs: 16 to 46, and a variant of a fragment of a polynucleotide having a sequence as set forth in at least one of SEQ ID NOs: 16 to 46;
preferably, the fifth nucleotide sequence comprises at least one of the following polynucleotides: a variant of a polynucleotide having a sequence as set forth in at least one of SEQ ID NOs: 16 to 46, a fragment of a polynucleotide having a sequence as set forth in at least one of SEQ ID NOs: 16 to 46, and a variant of a fragment of a polynucleotide having a sequence as set forth in at least one of SEQ ID NOs: 16 to 46, which has at least 40%, 50%, 60%, 70%, 80%, 85%, 90%, 91%, 92%, 93%, 94%, 95%, 96%, 97%, 98% or 99% identity to a polynucleotide having a sequence as set forth in at least one of SEQ ID NOs: 16 to 46.

45. The vector of any one of claims 42 to 44, wherein the fifth nucleotide sequence comprises at least one of: a 5'-UTR of at least two genes among the genes in (a), a fragment of the 5'-UTR of at least two genes among the genes in (a), a variant of the 5'-UTR of at least two genes among the genes in (a), and a variant of a fragment of the 5'-UTR of at least two genes among the genes in (a);
and/or, the fifth nucleotide sequence comprises: at least two copies of the 5'-UTR in (a), at least two copies of a fragment of the 5'-UTR in (a), at least two copies of a variant of the 5'-UTR in (a), and at least two copies of a variant of a fragment of the 5'-UTR in (a).

46. A vector comprising a polynucleotide sequence encoding a 5'-UTR; wherein the polynucleotide sequence encoding the 5'-UTR comprises at least one of the following polynucleotides: a polynucleotide having a sequence as set forth in SEQ ID NO: 9, a fragment of a polynucleotide having a sequence as set forth in SEQ ID NO: 9, a variant of a polynucleotide having a sequence as set forth in SEQ ID NO: 9, and a variant of a fragment of a polynucleotide having a sequence as set forth in SEQ ID NO: 9;
preferably, the variant of a polynucleotide having a sequence as set forth in SEQ ID NO: 9, the fragment of a polynucleotide having a sequence as set forth in SEQ ID NO: 9, and the variant of a fragment of a polynucleotide having a sequence as set forth in SEQ ID NO: 9 have at least 40%, 50%, 60%, 70%, 80%, 85%, 90%, 91%, 92%, 93%, 94%, 95%, 96%, 97%, 98% or 99% identity to the polynucleotide having a sequence as set forth in SEQ ID NO: 9.

47. The vector of any one of claims 42 to 46, further comprising at least one of a promoter, a polynucleotide encoding 3'-UTR, and a polynucleotide encoding a polyA; preferably, (1) the 3'-UTR comprises:
i): at least one of a 3'-UTR derived from at least one of the genes APOA1, IE1, COP1, MYSM1, ASAH2B, NDUFAF2, APOD, HBB, TF, TMSB4X, CPAMD8, VIM, HDGFL1, TTR, SRM, HBA1, PRPF8, LMBRD1, IFNA1, CCDC146, GHRL and GH1, a fragment, a variant and a variant of a fragment thereof;
preferably, an RNA encoded by a polynucleotide having a sequence as set forth in at least one of SEQ ID NOs: 47 to 67 and 10, a fragment of an RNA encoded by a polynucleotide having a sequence as set forth in at least one of SEQ ID NOs: 47 to 67 and 10, a variant of an RNA encoded by a polynucleotide having a sequence as set forth in at least one of SEQ ID NOs: 47 to 67 and 10, and a variant of a fragment of an RNA encoded by a polynucleotide having a sequence as set forth in at least one of SEQ ID NOs: 47 to 67 and 10;
preferably, the variant of an RNA encoded by a polynucleotide having a sequence as set forth in at least one of SEQ ID NOs: 47 to 67 and 10, the fragment of an RNA encoded by a polynucleotide having a sequence as set forth in at least one of SEQ ID NOs: 47 to 67 and 10, and the variant of a fragment of an RNA encoded by a polynucleotide having a sequence as set forth in at least one of SEQ ID NOs: 47 to 67 and 10 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in at least one of SEQ ID NOs: 47 to 67 and 10;
ii) at least two copies of one of the polynucleotides described in i); or
iii) at least two polynucleotides described in i);
(2) the nucleotides constituting the polyA include at least 20, at least 40, at least 80, at least 100 or at least 120 A nucleotides; preferably, the nucleotides constituting the polyA include consecutively at least 20, at least 40, at least 80, at least 100 or at least 120 A nucleotides;
preferably, the nucleotides constituting the polyA include one or more nucleotides other than the A nucleotide; preferably, the polyA is a truncated polyA; preferably, a 10 bp non-A linker sequence is added after a plurality of consecutive nucleotides A and then a plurality of consecutive nucleotides A is added; preferably, the polyA is 30 consecutive nucleotides A, then a 10 bp non-A linker sequence and then 70 consecutive nucleotides A;
preferably, the polyA comprises at least one of: an RNA encoded by a polynucleotide having a sequence as set forth in at least one of SEQ ID NOs: 68 to 76 and 11, a fragment of an RNA encoded by a polynucleotide having a sequence as set forth in at least one of SEQ ID NOs: 68 to 76 and 11, a variant of an RNA encoded by a polynucleotide having a sequence as set forth in at least one of SEQ ID NOs: 68 to 76 and 11, and a variant of a fragment of an RNA encoded by a polynucleotide having a sequence as set forth in at least one of SEQ ID NOs: 68 to 76 and 11; preferably, the fragment, the variant and the variant of a fragment of an RNA encoded by a polynucleotide having a sequence as set forth in at least one of SEQ ID NOs: 68 to 76 and 11 have at least 40%, 50%, 60%, 70%, 80%, 85%, 90%, 91%, 92%, 93%, 94%, 95%, 96%, 97%, 98% or 99% identity to the RNA encoded by a polynucleotide having a sequence as set forth in one of SEQ ID NOs: 68 to 76 and 11;
more preferably, the polyA is an RNA encoded by a polynucleotide having a sequence as set forth in one of SEQ ID NOs: 68 to 70, 72, 75 and 11; preferably, the polyA is an RNA encoded by a polynucleotide having a sequence as set forth in SEQ ID NO: 11.

48. A host cell comprising the vector of any one of claims 35 to 47.

49. A lipid nanoparticle comprising the artificial RNA molecule of claim 17 or 18, or the RNA molecule of any one of claims 19 to 33;
preferably, the lipid nanoparticle further comprises one or more of an ionizable cationic lipid, a helper lipid, a structural lipid and a PEG-lipid;
the ionizable cationic lipid is selected from one or more of Dlin-MC3-DMA, Dlin-KC2-DMA, DODMA, c12-200 and DlinDMA; and/or the helper lipid is selected from one or more of DSPC, DOPE, DOPC and DOPS; and/or the structural lipid is selected from at least one of cholesterol, cholesterol ester, steroid hormone, steroid vitamin and bile acid; and/or the PEG-lipid is selected from PEG-DMG or PEG-DSPE; preferably, the average molecular weight of PEG is about 2000 Daltons to 5000 Daltons; further preferably, the average molecular weight of PEG is about 2000 Daltons or 5000 Daltons.

50. A pharmaceutical composition comprising the artificial RNA molecule of claim 17 or 18, the RNA molecule of any one of claims 19 to 33, the DNA of claim 34, the vector of claims 35 to 47, the host cell of claim 48 or the lipid nanoparticle of claim 49, and a pharmaceutically acceptable carrier.

51. Use of the artificial RNA molecule of claim 17 or 18, the RNA molecule of any one of claims 19 to 33, the DNA of claim 34, the vector of claims 35 to 47, the host cell of claim 48, the lipid nanoparticle of claim 49, or the pharmaceutical composition of claim 50 in the preparation of a medicament;
preferably, the medicament is used for gene therapy, gene vaccination, protein replacement therapy, antisense therapy or therapy by interfering RNA.

52. Use of the artificial RNA molecule of claim 17 or 18, the RNA molecule of any one of claims 19 to 33, the DNA of claim 34, the vector of claims 35 to 47, the host cell of claim 48, the lipid nanoparticle of claim 49, or the pharmaceutical composition of claim 50 in the preparation of a medicament for nucleic acid transfer;
preferably, the nucleic acid is RNA, messenger RNA (mRNA), antisense oligonucleotide, DNA, plasmid, ribosomal RNA (rRNA), micro RNA (miRNA), transfer RNA (tRNA), small interfering RNA (siRNA) and small nuclear RNA (snRNA).
preferably, the medicament is for use in gene therapy, gene vaccination or protein replacement therapy;
preferably, the medicament is for use in the treatment and/or prevention of a disease; preferably, the medicament is a vaccine; more preferably, the medicament is a vaccine for preventing SARS-COV-2 infection;
preferably, the disease is selected from the group consisting of rare diseases, infectious diseases, cancer, genetic diseases, autoimmune diseases, diabetes, neurodegenerative diseases, cardiovascular diseases, renal vascular diseases, and metabolic diseases;
preferably, the cancer comprises one or more of lung cancer, stomach cancer, liver cancer, esophageal cancer, colon cancer, pancreatic cancer, brain cancer, lymphoma, blood cancer or prostate cancer; the genetic disease comprises one or more of hemophilia, thalassemia and Gaucher's disease;
the medicament is a nucleic acid drug, wherein the nucleic acid comprises at least one of the following: RNA, messenger RNA (mRNA), DNA, plasmid, ribosomal RNA (rRNA), single guide RNA (sgRNA) and cas9 mRNA.
